(19) Europäisches Patentamt
European Patent Office
Office européen des brevets

(11) EP 4 458 836 A1

(12) EUROPEAN PATENT APPLICATION
published in accordance with Art. 153(4) EPC

(43) Date of publication:
**06.11.2024 Bulletin 2024/45**

(21) Application number: **22916080.9**

(22) Date of filing: **27.12.2022**

(51) International Patent Classification (IPC):
***C07H 19/10*** (2006.01) ***A61K 31/7088*** (2006.01)
***A61K 48/00*** (2006.01) ***A61P 31/12*** (2006.01)
***A61P 35/00*** (2006.01) ***C07H 19/067*** (2006.01)
***C07H 21/04*** (2006.01) ***C12M 1/00*** (2006.01)
***C12M 1/34*** (2006.01) ***C12N 15/11*** (2006.01)
***C12Q 1/6844*** (2018.01)

(52) Cooperative Patent Classification (CPC):
**A61K 31/7088; A61K 48/00; A61P 31/12; A61P 35/00; C07H 19/067; C07H 19/10; C07H 21/04; C12M 1/00; C12M 1/34; C12N 15/11; C12Q 1/6844;** Y02P 20/55

(86) International application number:
**PCT/JP2022/048121**

(87) International publication number:
**WO 2023/127857 (06.07.2023 Gazette 2023/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.12.2021 JP 2021213197**
**30.08.2022 JP 2022136985**

(71) Applicant: **Riken Genesis Co., Ltd.**
**Tokyo 141-0032 (JP)**

(72) Inventors:
- **YOSHIKAWA, Haruhisa**
**Tokyo 141-0032 (JP)**
- **BAN, Ikuya**
**Tokyo 141-0032 (JP)**
- **YASUNAGA, Maki**
**Tokyo 141-0032 (JP)**
- **IMANISHI, Takeshi**
**Tokyo 141-0032 (JP)**
- **KITAMURA, Masato**
**Tokyo 141-0032 (JP)**

(74) Representative: **De Clercq & Partners**
**Edgard Gevaertdreef 10a**
**9830 Sint-Martens-Latem (BE)**

Remarks:
The complete document including Reference Table(s) and the Sequence Listing(s) can be downloaded from the EPO website

# (54) NOVEL ARTIFICIAL NUCLEIC ACID, METHOD FOR PRODUCING SAME, AND USE OF SAME

(57) The present invention provides an artificial nucleic acid having units derived from compound represented by formula (1) or a salt thereof

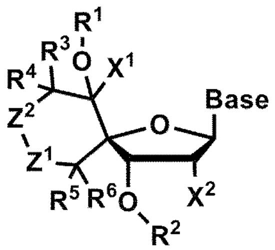

(1)

(wherein "Base" represents an aromatic heterocyclic group which may have a substituent, or an aromatic hydrocarbon ring group which may have a substituent; $X^1$ represents a hydrogen atom, an alkyl group which may have a substituent, an alkenyl group which may have a substituent, an alkynyl group which may have a substituent, a cycloalkyl group which may have a substituent, or an aryl group which may have a substituent; $X^2$ represents a hydrogen atom or $-OR^7$; $R^1$, $R^2$, and $R^7$ are the same as or different from each other and independently represent a hydrogen atom, an alkyl group which may have a substituent, an alkenyl group which may have a substituent, an alkynyl group which may have a substituent, a cycloalkyl group which may have a substituent, a cycloalkenyl group which may have a substituent, an aryl group which may have a substituent, a protecting group for a hydroxy group, a phosphino group which has a substituent, a dihydroxyphosphinyl group which may have a substituent, or a hydroxymercaptophosphinyl group which may have a substituent; $R^3$, $R^4$, $R^5$, and $R^6$ are the same as or different from each other and independently represent a hydrogen atom, an alkyl group which may have a substituent, an alkenyl group which may have a substituent, an alkynyl group which may have a substituent, a cycloalkyl group which may have a substituent, or an aryl group which may have a substituent; $Z^1$ represents O or $NZ^{11}$; $Z^{11}$ represents a hydrogen atom, an alkyl group, an alkenyl group, an alkynyl group, a cycloalkyl group, an aryl group, an aralkyl group, or a protecting group for an amino group; $Z^2$ represents a single bond or $CZ^{21}Z^{22}$; and $Z^{21}$ and $Z^{22}$ are the same as or different from each other and independently represent a hydrogen atom, an alkyl group which may have a substituent, an alkenyl group which may have a substituent, an alkynyl group which may have a substituent, a cycloalkyl group which may have a substituent, or an aryl group which may have a substituent).

## Description

TECHNICAL FIELD

The present invention relates to a novel artificial nucleic acid, a method for producing the artificial nucleic acid, a use of the artificial nucleic acid, and others.

BACKGROUND ART

An oligonucleotide is a short sequence of naturally occurring DNA, naturally occurring RNA or an artificial nucleic acid. It has been shown that an oligonucleotide can regulate the expression of a gene at various gene transcription or translation levels and can examine the state of the sequence for a gene and is therefore very useful for the treatment or diagnosis of a specific disease.

The technique for the regulation of the expression of a gene and the examination/diagnosis of genetic information is roughly classified into two types depending on the types of targets. The first case is a case where a target is single-stranded RNA or single-stranded DNA such as messenger RNA (mRNA) or microRNA (miRNA), and the second case is a case where the target is double-stranded genomic DNA.

When the target is single-stranded RNA or single-stranded DNA, the process of the translation of a gene can be inhibited (or the genetic diagnosis can be performed) by an antisense method in which an oligonucleotide binds to the single-stranded RNA or the single-stranded DNA complementary to form a double strand. When the oligonucleotide is a double-stranded RNA molecule, the complementary binding of the oligonucleotide to target mRNA can cause the decomposition of the target mRNA with a "slicer" enzyme in an RISC complex (RNA interference method). In the case of RNA interference method, the oligonucleotide may be an oligonucleotide which is equivalent to endogenous microRNA that can bind to a 3'-UTR (a 3'-untranslated region) in target mRNA and can inhibit the translation of the target mRNA as the result of the incomplete complementarity (microRNA mimics).

An oligonucleotide can induce the activation of a gene or the increase in transcription of the gene through, for example, the complementary binding of the oligonucleotide to long antisense non-coding RNA or the inhibition of complementary microRNA by the oligonucleotide, and, as a result, can increase the translation of target mRNA in the microRNA (anti-microRNA).

An oligonucleotide which can be used as a functional material for use in a technique for regulating gene expression or examining/diagnosing genetic information has been required to have properties such as excellent sequence-specific binding affinity for a target nucleic acid, high resistance to degradation enzymes and safety in living bodies. DNA and RNA which are naturally occurring materials have poor resistance to degradation enzymes and insufficient binding affinity, and are therefore unsuitable as functional materials. For these reasons, numerous artificial nucleic acids have been developed so far for the purpose of improving the functionalities of oligonucleotides.

Typical examples of the artificial nucleic acid include a peptide nucleic acid (PNA), a crosslinked nucleic acid, a morpholino nucleic acid (PMO), and a phosphorothioate-type nucleic acid (S-oligonucleotide) which has such a structure that one of non-binding oxygen atoms in a phosphoric acid diester moiety in a nucleic acid is substituted by a sulfur atom.

Among the artificial nucleic acids, for example, three types of artificial nucleic acids described below are known as an artificial nucleic acid having a spiro ring (Non-Patent Documents 1 and 2).

[Formula 1]

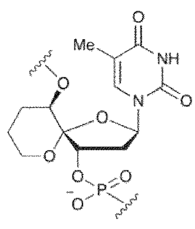

Structural formula 1

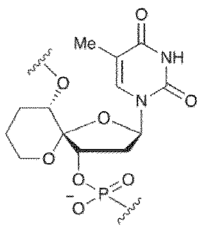

Structural formula 2

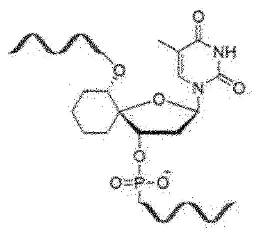

Structural formula 3

PRIOR ART DOCUMENTS

NON-PATENT DOCUMENTS

[Non-Patent Document 1] Bioorg. Med. Chem. 31 (2021) 115966
[Non-Patent Document 2] Summary of lectures at the 71st Kansai Branch Meeting of the Pharmaceutical Sciences of Japan

## DISCLOSURE OF THE INVENTION

## PROBLEM TO BE SOLVED BY THE INVENTION

However, amid the diversification in use applications of oligonucleotides, there is still room for improving the functionalities of the already developed artificial nucleic acids and it has been demanded to develop a novel artificial nucleic acid aiming at the further improvement in the functionality of the artificial nucleic acid.
The object of the present invention is to provide a novel artificial nucleic acid which is useful in various genome technologies, a method for producing the artificial nucleic acid, a use of the artificial nucleic acid, and others.

## SOLUTIONS TO THE PROBLEMS

As a result of intensive studies to achieve the above-mentioned object, the present inventors have found that a novel artificial nucleic acid having a 1,7-dioxaspiro[4.4]nonane skeleton, a 1,7-dioxaspiro[4.5]decane skeleton, a 1-oxa-7-azaspiro[4.4]nonane skeleton, or a 1-oxa-7-azaspiro[4.5]decane skeleton is useful for various genomic technologies. Based on such findings, the present inventors have further studied and accomplished the present invention.
The present invention includes the following aspects.

Item 1.

A compound represented by formula (1) or a salt thereof:

[Formula 2]

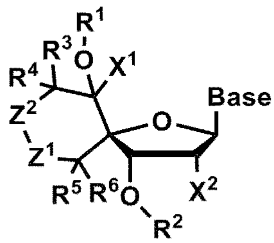

(1)

(wherein "Base" represents an aromatic heterocyclic group which may have a substituent, or an aromatic hydrocarbon ring group which may have a substituent;
$X^1$ represents a hydrogen atom, an alkyl group which may have a substituent, an alkenyl group which may have a substituent, an alkynyl group which may have a substituent, a cycloalkyl group which may have a substituent, or an aryl group which may have a substituent;
$X^2$ represents a hydrogen atom or $-OR^7$;
$R^1$, $R^2$, and $R^7$ are the same as or different from each other and independently represent a hydrogen atom, an alkyl group which may have a substituent, an alkenyl group which may have a substituent, an alkynyl group which may have a substituent, a cycloalkyl group which may have a substituent, a cycloalkenyl group which may have a substituent, an aryl group which may have a substituent, a protecting group for a hydroxy group, a phosphino group which has a substituent, a dihydroxyphosphinyl group which may have a substituent, or a hydroxymercaptophosphinyl group which may have a substituent;
$R^3$, $R^4$, $R^5$, and $R^6$ are the same as or different from each other and independently represent a hydrogen atom, an alkyl group which may have a substituent, an alkenyl group which may have a substituent, an alkynyl group which may have a substituent, a cycloalkyl group which may have a substituent, or an aryl group which may have a substituent;
$Z^1$ represents O or $NZ^{11}$;
$Z^{11}$ represents a hydrogen atom, an alkyl group, an alkenyl group, an alkynyl group, a cycloalkyl group, an aryl group, an aralkyl group, or a protecting group for an amino group;
$Z^2$ represents a single bond or $CZ^{21}Z^{22}$; and
$Z^{21}$ and $Z^{22}$ are the same as or different from each other and independently represent a hydrogen atom, an alkyl

group which may have a substituent, an alkenyl group which may have a substituent, an alkynyl group which may have a substituent, a cycloalkyl group which may have a substituent, or an aryl group which may have a substituent).

Item 2.

The compound or the salt thereof according to item 1, wherein $Z^1$ represents O. Item 3.
The compound or the salt thereof according to item 1, wherein $Z^1$ represents $NZ^{11}$ and $Z^2$ represents $CZ^{21}Z^{22}$.

Item 4.

The compound or the salt thereof according to item 3, wherein $Z^{11}$ represents a hydrogen atom or an alkyl group.

Item 5.

The compound or the salt thereof according to any one of items 1 to 4, wherein $Z^{21}$ and $Z^{22}$ are the same as or different from each other and independently represent a hydrogen atom or an alkyl group.

Item 6.

The compound or the salt thereof according to any one of items 1 to 5, wherein $X^1$ represents a hydrogen atom or an alkyl group.

Item 7.

The compound or the salt thereof according to any one of items 1 to 6, wherein $X^1$ represents a hydrogen atom.

Item 8.

The compound or the salt thereof according to any one of items 1 to 7, wherein $R^3$, $R^4$, $R^5$, and $R^6$ are the same as or different from each other and independently represent a hydrogen atom or an alkyl group.

Item 9.

The compound or the salt thereof according to any one of items 1 to 8, wherein $R^3$, $R^4$, $R^5$, and $R^6$ independently represent a hydrogen atom.

Item 10.

The compound or the salt thereof according to any one of items 1 to 9, wherein $R^7$ represents a hydrogen atom or an alkyl group.

Item 11.

The compound or the salt thereof according to any one of items 1 to 10, wherein $R^1$ and $R^2$ are the same as or different from each other and independently represent a hydrogen atom, an alkyl group which may have a substituent, an aryl group which may have a substituent, an alkylcarbonyl group, an arylcarbonyl group, an alkylsulfonyl group, an arylsulfonyl group, a group represented by a formula: $-Si(R^8)_3$ (wherein $R^8$'s are the same as or different from each other and independently represent an alkyl group or an aryl group), a group represented by a formula: $-P(R^{9a})(R^{9b})$ (wherein $R^{9a}$ and $R^{9b}$ are the same as or different from each other and independently represents a hydroxy group, a mercapto group, an amino group, an alkoxy group, a haloalkoxy group, a cyanoalkoxy group, an alkylthio group, a haloalkylthio group, a cyanoalkylthio group, or an alkylamino group), a dihydroxyphosphinyl group, or a hydroxymercaptophosphinyl group.

Item 12.

The compound or the salt thereof according to any one of items 1 to 11, wherein "Base" represents a 2,4-dioxo-1,2,3,4-tetrahydropyrimidin-1-yl group which may have a substituent, a 2-oxo-1,2-dihydropyrimidin-1-yl group which may have a substituent, a purin-9-yl group which may have a substituent, or a 6-oxo-1,6-dihydro-9H-purin-9-yl group which

may have a substituent.

Item 13.

A method for producing the compound or the salt thereof according to any one of items 1 to 12, the method comprising:

a step of cyclization of a compound represented by formula (2) or a salt thereof in the presence or absence of an amine represented by formula (2'): $Z^{11}$-$NH_2$ ($Z^{11}$ is as defined above),

[Formula 3]

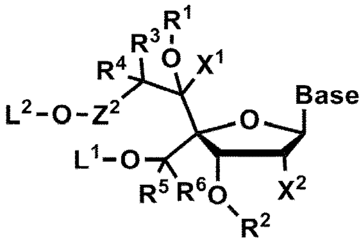

(2)

(wherein $L^1$ represents a hydroxy activating group, $L^2$ represents a hydrogen atom or a hydroxy activating group, and "Base", $X^1$, $X^2$, $R^1$, $R^2$, $R^3$, $R^4$, $R^5$, $R^6$, and $Z^2$ are as defined above).

Item 14.

An oligonucleotide comprising a unit represented by formula (6) or a salt thereof

[Formula 4]

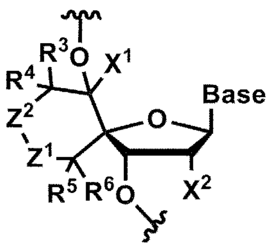

(6)

(wherein "Base" represents an aromatic heterocyclic group which may have a substituent, or an aromatic hydrocarbon ring group which may have a substituent;

$X^1$ represents a hydrogen atom, an alkyl group which may have a substituent, an alkenyl group which may have a substituent, an alkynyl group which may have a substituent, a cycloalkyl group which may have a substituent, or an aryl group which may have a substituent;

$X^2$ represents a hydrogen atom or $-OR^7$;

$R^7$ represents a hydrogen atom, an alkyl group which may have a substituent, an alkenyl group which may have a substituent, an alkynyl group which may have a substituent, a cycloalkyl group which may have a substituent, a cycloalkenyl group which may have a substituent, an aryl group which may have a substituent, a protecting group for a hydroxy group, a phosphino group which has a substituent, a dihydroxyphosphinyl group which may have a substituent, or a hydroxymercaptophosphinyl group which may have a substituent;

$R^3$, $R^4$, $R^5$, and $R^6$ are the same as or different from each other and independently represent a hydrogen atom, an alkyl group which may have a substituent, an alkenyl group which may have a substituent, an alkynyl group which may have a substituent, a cycloalkyl group which may have a substituent, or an aryl group which may have a substituent;

$Z^1$ represents O or $NZ^{11}$;

$Z^{11}$ represents a hydrogen atom, an alkyl group, an alkenyl group, an alkynyl group, a cycloalkyl group, an aryl group, an aralkyl group, or a protecting group for an amino group;

$Z^2$ represents a single bond or $CZ^{21}Z^{22}$; and
$Z^{21}$ and $Z^{22}$ are the same as or different from each other and independently represent a hydrogen atom, an alkyl group which may have a substituent, an alkenyl group which may have a substituent, an alkynyl group which may have a substituent, a cycloalkyl group which may have a substituent, or an aryl group which may have a substituent).

Item 15.

A method of detecting a target nucleic acid, comprising steps of:

(I) amplifying the target nucleic acid selectively by a nucleic acid amplification method; and
(II) detecting the target nucleic acid that has been amplified in the step (I),

in which an oligonucleotide that is used for the amplification or the detection comprises the oligonucleotide or the salt thereof according to item 14.

Item 16.

A kit for detecting or amplifying a target nucleic acid, comprising:

(a) a primer and a probe, in which at least one of the primer and the probe comprises the oligonucleotide or the salt thereof according to item 14; or
(b) a clamp nucleic acid and a primer, in which at least one of the clamp nucleic acid and the primer comprises the oligonucleotide or the salt thereof according to item 14.

Item 17.

A composition comprising the compound or the salt thereof according to any one of items 1 to 12, or the oligonucleotide or the salt thereof according to item 14.

Item 18.

A compound represented by formula (2) or a salt thereof:

[Formula 5]

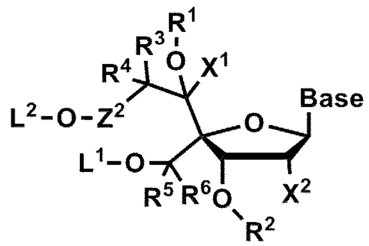

(2)

(wherein "Base" represents an aromatic heterocyclic group which may have a substituent, or an aromatic hydrocarbon ring group which may have a substituent;
$X^1$ represents a hydrogen atom, an alkyl group which may have a substituent, an alkenyl group which may have a substituent, an alkynyl group which may have a substituent, a cycloalkyl group which may have a substituent, or an aryl group which may have a substituent;
$X^2$ represents a hydrogen atom or $-OR^7$;
$R^1$, $R^2$, and $R^7$ are the same as or different from each other and independently represent a hydrogen atom, an alkyl group which may have a substituent, an alkenyl group which may have a substituent, an alkynyl group which may have a substituent, a cycloalkyl group which may have a substituent, a cycloalkenyl group which may have a substituent, an aryl group which may have a substituent, a protecting group for a hydroxy group, a phosphino group which has a substituent, a dihydroxyphosphinyl group which may have a substituent, or a hydroxymercaptophosphinyl group which may have a substituent;
$R^3$, $R^4$, $R^5$, and $R^6$ are the same as or different from each other and independently represent a hydrogen atom, an

alkyl group which may have a substituent, an alkenyl group which may have a substituent, an alkynyl group which may have a substituent, a cycloalkyl group which may have a substituent, or an aryl group which may have a substituent;

$L^1$ represents a hydroxy activating group;

$L^2$ represents a hydrogen atom or a hydroxy activating group;

$Z^2$ represents a single bond or $CZ^{21}Z^{22}$; and

$Z^{21}$ and $Z^{22}$ are the same as or different from each other and independently represent a hydrogen atom, an alkyl group which may have a substituent, an alkenyl group which may have a substituent, an alkynyl group which may have a substituent, a cycloalkyl group which may have a substituent, or an aryl group which may have a substituent).

Item 19.

A compound represented by formula (4) or a salt thereof:

[Formula 6]

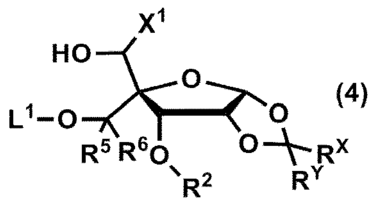

(4)

(wherein $R^2$ represents a hydrogen atom, an alkyl group which may have a substituent, an alkenyl group which may have a substituent, an alkynyl group which may have a substituent, a cycloalkyl group which may have a substituent, a cycloalkenyl group which may have a substituent, an aryl group which may have a substituent, a protecting group for a hydroxy group, a phosphino group which has a substituent, a dihydroxyphosphinyl group which may have a substituent, or a hydroxymercaptophosphinyl group which may have a substituent;

$R^5$, $R^6$, $R^X$, $R^Y$, and $X^1$ are the same as or different from each other and independently represent a hydrogen atom, an alkyl group which may have a substituent, an alkenyl group which may have a substituent, an alkynyl group which may have a substituent, a cycloalkyl group which may have a substituent, or an aryl group which may have a substituent; and

$L^1$ represents a hydroxy activating group).

EFFECTS OF THE INVENTION

According to the present invention, a novel artificial nucleic acid useful in various genomic technologies can be provided.

BRIEF DESCRIPTION OF THE DRAWINGS

Fig. 1A shows a schematic diagram of one example of a kit according to the present invention including a container in which a composition containing a primer and a probe is included.

Fig. 1B shows a schematic diagram of one example of a kit according to the present invention including both of a container in which a composition containing a primer is included and a container in which a composition containing a probe is included.

Fig. 1C shows a schematic diagram of one example of a kit according to the present invention including a container in which a composition containing a forward primer is included, a container in which a composition containing a reverse primer is included, and a container in which a composition containing a probe is included.

Fig. 2 is a graph showing the relationship between a reaction time with CAVP (Crotalus Adamanteus Venom Phosphodiesterase I) at a final concentration of 2.0 μg/mL and a residual ratio of an undigested oligonucleotide.

DETAILED DESCRIPTION OF THE PREFERRED EMBODIMENTS

1. Definition of terms

In the present description, the term "alkyl group" refers to a monovalent group having such a structure that one hydrogen atom is removed from a linear or branched saturated hydrocarbon.

The number of carbon atoms in the alkyl group is not particularly limited, and is for example 1 to 20, preferably 1 to 10, more preferably 1 to 6, particularly preferably 1 to 4.

Examples of the alkyl group include a methyl group, an ethyl group, a propyl group (e.g., a n-propyl group, an i-propyl group), a butyl group (e.g., a n-butyl group, an i-butyl group, an s-butyl group, a t-butyl group), a pentyl group (e.g., a n-pentyl group, an i-pentyl group, a neopentyl group), a hexyl group, a heptyl group, an octyl group (e.g., a n-octyl group, a 2-ethylhexyl group), a nonyl group, and a decyl group.

In the present description, the term "alkylene group" refers to a bivalent group having such a structure that two hydrogen atoms are removed from a linear or branched saturated hydrocarbon.

The number of carbon atoms in the alkylene group is not particularly limited, and is for example 1 to 10, preferably 1 to 8, more preferably 1 to 6.

Examples of the alkylene group include a $C_1$-alkylene group (e.g., a methylene group), a $C_2$-alkylene group (e.g., a methylmethylene group, a dimethylene group), a $C_3$-alkylene group (e.g., a trimethylene group, a dimethylmethylene group), a $C_4$-alkylene group (e.g., a tetramethylene group), a $C_5$-alkylene group (e.g., a pentamethylene group), and a $C_6$-alkylene group (e.g., a hexamethylene group).

In the present description, the term "alkenyl group" refers to a monovalent group having such a structure that one hydrogen atom is removed from a linear or branched unsaturated hydrocarbon containing a carbon-carbon double bond.

The number of carbon atoms in the alkenyl group is not particularly limited, and is for example 2 to 20, preferably 2 to 10, more preferably 2 to 6.

Examples of the alkenyl group include an ethenyl group (i.e., a vinyl group), a propenyl group (e.g., a 1-propenyl group, an allyl group), a butenyl group, a pentenyl group, a hexenyl group, a geranyl group, and a farnesyl group.

In the present description, the term "alkynyl group" refers to a monovalent group having such a structure that one hydrogen atom is removed from a linear or branched unsaturated hydrocarbon containing a carbon-carbon triple bond.

The number of carbon atoms in the alkynyl group is not particularly limited, and is for example 2 to 20, preferably 2 to 10, more preferably 2 to 6.

Examples of the alkynyl group include an ethynyl group, a propargyl group, and a 1-butynyl group.

In the present description, the term "cycloalkyl group" refers to a monovalent group derived from a saturated aliphatic hydrocarbon ring.

The number of carbon atoms in the cycloalkyl group is not particularly limited, and is for example 3 to 20, preferably 5 to 12, more preferably 5 to 10.

Examples of the cycloalkyl group include a cyclopropyl group, a cyclobutyl group, a cyclopentyl group, a cyclohexyl group, a cycloheptyl group, a cyclooctyl group, a norbornyl group, and an adamantyl group.

In the present description, the term "cycloalkenyl group" refers to a monovalent group derived from an unsaturated aliphatic hydrocarbon ring containing a carbon-carbon double bond.

The number of carbon atoms in the cycloalkenyl group is not particularly limited, and is for example 3 to 20, preferably 5 to 12, more preferably 5 to 10.

Examples of the cycloalkenyl group include a cyclopropenyl group, a cyclobutenyl group, a cyclopentenyl group, a cyclohexenyl group, a norbornenyl group, and an adamantenyl group.

In the present description, the term "aromatic hydrocarbon ring group" refers to a monovalent group derived from an aromatic hydrocarbon ring, and is also referred to as an "aryl group".

The number of constituent atoms in the aromatic hydrocarbon ring is not particularly limited, and is for example 6 to 20, preferably 6 to 14, more preferably 6 to 12, particularly preferably 6 to 10.

The aromatic hydrocarbon ring may be a monocyclic ring or a condensed ring (e.g., a di- to tri-cyclic condensed ring).

Examples of the aromatic hydrocarbon ring group include a phenyl group, an indenyl group, a naphthyl group, a fluorenyl group, a phenanthrenyl group, and an anthracenyl group.

In the present description, the term "heterocyclic ring" is used in the meaning that an "aliphatic heterocyclic ring" and an "aromatic heterocyclic ring" are included.

In the present description, the term "aliphatic heterocyclic ring" refers to an aliphatic ring which contains, as ring constituent atoms, a carbon atom and at least one hetero atom selected from the group consisting of a nitrogen atom, an oxygen atom, a sulfur atom, a silicon atom, and the like.

The number of constituent atoms in the aliphatic heterocyclic ring is not particularly limited, and is for example

5 to 20, preferably 5 to 12, more preferably 6 to 10.

The number of hetero atom(s) among the constituent atoms of the aliphatic heterocyclic ring is not particularly limited, and is for example 1 to 4.

Examples of the aliphatic heterocyclic ring include an oxygenated aliphatic heterocyclic ring (e.g., tetrahydrofuran, dioxolane, pyran, tetrahydropyran, dioxane), a sulfur-containing aliphatic heterocyclic ring (e.g., tetrahydrothiophene, thiopyran, tetrahydrothiopyran), a nitrogenated aliphatic heterocyclic ring (e.g., pyrrolidine, piperidine, azepane), a nitrogenated and oxygenated aliphatic heterocyclic ring (e.g., morpholine), a nitrogenated and sulfur-containing aliphatic heterocyclic ring (e.g., thiomorpholine), and an aliphatic heterocyclic ring containing a siloxane bond.

In the present description, the term "aromatic heterocyclic ring" refers to an aromatic ring containing, as ring constituent atoms, a carbon atom and at least one hetero atom selected from the group consisting of a nitrogen atom, an oxygen atom, a sulfur atom, and the like.

The number of constituent atoms in the aromatic heterocyclic ring is not particularly limited, and is for example 5 to 20, preferably 5 to 12, more preferably 5 to 10.

The number of hetero atom(s) among the constituent atoms of the aromatic heterocyclic ring is not particularly limited, and is, for example, 1 to 4. Among the constituent atoms of the aromatic heterocyclic ring, the number of carbon atoms is not particularly limited, but is, for example, 1 to 10.

The aromatic heterocyclic ring may be a monocyclic ring or a condensed ring (e.g., a di- or tri-cyclic condensed ring).

Examples of the aromatic heterocyclic ring include an oxygenated aromatic heterocyclic ring (e.g., furan, benzofuran, isobenzofuran, chromene, benzopyran, xanthene), a sulfur-containing aromatic heterocyclic ring (e.g., thiophene, thianthrene), a nitrogenated aromatic heterocyclic ring (e.g., pyrrole, imidazole, pyrazole, triazole, pyridine, pyrazine, pyrimidine, pyridazine, indole, isoindole, indolizine, purine, quinoline, isoquinoline, 1,8-naphthyridine, quinoxaline, quinazoline, cinnoline, phthalazine, pteridine, carbazole, phenanthridine, acridine, perimidine, phenazine), an oxygenated and sulfur-containing aromatic heterocyclic ring (e.g., phenoxathiin), a nitrogenated and oxygenated aromatic heterocyclic ring (e.g., oxazole, isoxazole, furazan, phenoxazine), and a nitrogenated and sulfur-containing aromatic heterocyclic ring (e.g., thiazole, isothiazole, phenothiazine).

In the present description, the term "heterocyclic group" refers to a monovalent group having such a structure that one hydrogen atom is removed from the heterocyclic ring. The term "aromatic heterocyclic group" refers to a monovalent group obtained by removing one hydrogen atom from the aromatic heterocyclic ring, and is also referred to as a "heteroaryl group".

In the present description, the wording "may have a substituent" or "may be substituted by a substituent" is used in the meaning including both of a case where no substituent is contained and a case where one substituent or two or more same-type or different-type substituents are contained in place of an arbitrary hydrogen atom. In the case of having a substituent or the substituents, the number of the substituent(s) may be selected from the range of 1 to the maximum number of possible substitutes as not being particularly limited, and is, for example, 1 to 3, preferably 1 or 2.

In the present description, the term "substituent" refers to an atom or an atomic group which is replaced for a hydrogen atom. In the present description, the term "substituent" refers to an atom or an atomic group which is replaced for a hydrogen atom. Examples of the substituent include a halogen atom (e.g., a fluorine atom, a chlorine atom, a bromine atom, an iodine atom), an oxo group (=O), a thioxo group (=S), a hydroxy group, a mercapto group, an amino group, a carboxy group, an alkyl group, an alkenyl group, an alkynyl group, a cycloalkyl group, a cycloalkenyl group, an aryl group, an acyl group, an alkylsulfonyl group, an arylsulfonyl group, a cyano group, a heterocyclic group, and a combination of any two or more of them (e.g., a haloalkyl group, a cyanoalkyl group, an aralkyl group, an alkoxy group, an alkylamino group).

The term "a combination of two or more of them" includes an arbitrary combination of the groups which are exemplified as the substituents.

In the present description, the term "$C_{x-y}$" means that the number of carbon atom(s) in a group following this term is x to y inclusive. Each of x and y represents a positive integer, in which x and y satisfy the requirement represented by the formula: $x < y$.

In the present description, the term "haloalkyl group" refers to an alkyl group substituted by one halogen atom or two or more same or different halogen atoms.

A preferred example of the haloalkyl group is a $C_{1-6}$ haloalkyl group, a more preferred example is a $C_{1-4}$ haloalkyl group, and a still more preferred example is a trifluoromethyl group, a trichloromethyl group, or a 2,2,2-trifluoroethyl group.

In the present description, the term "cyanoalkyl group" refers to an alkyl group substituted by one cyano group or two or more cyano groups.

A preferred example of the cyanoalkyl group is a $C_{1-6}$ cyanoalkyl group, a more preferred example is a $C_{1-4}$ cyanoalkyl group, and a still more preferred example is a cyanomethyl group or a 2-cyanoethyl group.

In the present description, the term "aralkyl group" refers to an alkyl group substituted by one aryl group or two or more same or different aryl groups.

A preferred example of the aralkyl group is a $C_{6-14}$-aryl-$C_{1-4}$-alkyl group, and a more preferred example is a phenylmethyl group (i.e., a benzyl group), a phenylethyl group (i.e., a phenethyl group), a naphthylmethyl group, a naphthylethyl group, a triphenylmethyl group (i.e., a trityl group), or a fluorenylmethyl group.

In the present description, the term "alkoxy group" refers to a group represented by the formula: -O-alkyl. A preferred example of the alkoxy group is a $C_{1-6}$ alkoxy group, and a more preferred example is a methoxy group, an ethoxy group, a propoxy group (e.g., a n-propoxy group, an i-propoxy group), or a butoxy group (e.g., a t-butoxy group).

The haloalkoxy group and the cyanoalkoxy group refer to a group represented by the formula: -O-haloalkyl and a group represented by the formula: -O-cyanoalkyl, respectively.

In the present description, the term "alkylthio group" refers to a group represented by the formula: -S-alkyl. A preferred example of the alkylthio group is a $C_{1-6}$ alkylthio group, and a more preferred example is a methylthio group, an ethylthio group, a propylthio group (e.g., a n-propylthio group, an i-propylthio group), or a butylthio group.

A haloalkylthio group and a cyanoalkylthio group refer to a group represented by the formula: -S-haloalkyl and a group represented by the formula: -S-cyanoalkyl, respectively.

In the present description, the term "alkylamino group" refers to an amino group which is substituted by one or two same or different alkyl groups.

The alkylamino group includes a monoalkylamino group and a dialkylamino group.

A preferred example of the monoalkylamino group is a mono-$C_{1-6}$ alkylamino group, and a more preferred example is a monomethylamino group, a monoethylamino group, a monopropylamino group (e.g., a mono(n-propyl)amino group, a mono(i-propyl)amino group), or a monobutylamino group.

A preferred example of the dialkylamino group is a di-$C_{1-6}$ alkylamino group, and a more preferred example is a dimethylamino group, a diethylamino group, a dipropylamino group (e.g., a di(n-propyl)amino group, a di(i-propyl)amino group), or a dibutylamino group.

In the present description, the term "phosphino group which has a substituent" refers to a group having such a structure that at least one hydrogen atom in a phosphino group ($-PH_2$) is substituted by another atom or atomic group.

An example of the phosphino group which has a substituent is a group represented by the formula: $-P(R^{9a})(R^{9b})$ (wherein $R^{9a}$ and $R^{9b}$ are the same as or different from each other and independently represent a hydroxy group, a mercapto group, an amino group, an alkoxy group, a haloalkoxy group, a cyanoalkoxy group, an alkylthio group, a haloalkylthio group, a cyanoalkylthio group, or an alkylamino group).

In the present description, the term "dihydroxyphosphinyl group which may have a substituent" refers to a dihydroxyphosphinyl group (i.e., a phosphono group) ($-P(=O)(OH)_2$) or a dihydroxyphosphinyl group in which at least one hydrogen atom is substituted by another atom or an atomic group (e.g., a protecting group for a hydroxy group).

The latter group includes a group represented by the following formula:

[Formula 7]

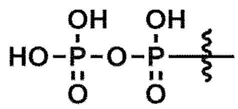

which may have a substituent (hereinafter, also referred to as a "diphosphate group"), and a group represented by the following formula:

[Formula 8]

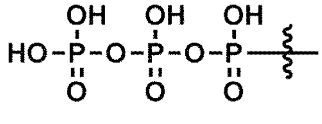

which may have a substituent (hereinafter, also referred to as a "triphosphate group").

In the present description, the term "hydroxymercaptophosphinyl group which may have a substituent" refers to a hydroxymercaptophosphinyl group (-P(=O)(OH)(SH)) or a hydroxymercaptophosphinyl group in which at least one hydrogen atom is substituted by another atom or an atomic group (e.g., a protecting group for a hydroxy group).

In the present description, the term "protecting group for a hydroxy group" refers to a monovalent group which can prevent a hydroxy group from being involved in a reaction for the synthesis of a compound or a salt thereof or a reaction for the synthesis of an oligonucleotide or a salt thereof.

An example of the protecting group for a hydroxy group is, but is not limited to, a group which is stable under

an acidic or neutral condition and can be cleaved by a method such as a hydrogenolysis, a hydrolysis, an electrolysis and a photodissociation.

Specific examples of the protecting group for a hydroxy group include an acyl group which may have a substituent, a thiocarbonyl group which has a substituent, a sulfonyl group which has a substituent, and a silyl group which has a substituent.

In the present description, the term "acyl group" refers to a group represented by the formula: -C(=O)-R (wherein R represents a hydrocarbon group). The hydrocarbon group represented by R may be a linear or branched hydrocarbon group (e.g., an alkyl group), or may be a saturated or unsaturated hydrocarbon ring group (e.g., a cycloalkyl group, an aryl group), or may be a combination thereof (e.g., an aralkyl group).

Examples of the acyl group include an alkylcarbonyl group, an arylcarbonyl group, and an aralkylcarbonyl group.

A preferred example of the alkylcarbonyl group is a ($C_{1-10}$-alkyl)carbonyl group, and a more preferred example is an acetyl group, a propionyl group, a butyryl group, an isobutyryl group, a pentanoyl group, a pivaloyl group, a valeryl group, an isovaleryl group, an octanoyl group, a nonanoyl group, or a decanoyl group.

A preferred example of the arylcarbonyl group is a ($C_{6-14}$-aryl)carbonyl group, and a more preferred example is a benzoyl group, or a naphthoyl group (i.e., an $\alpha$-naphthoyl group, a $\beta$-naphthoyl group).

A preferred example of the aralkylcarbonyl group is a ($C_{6-14}$-aryl-$C_{1-4}$-alkyl)carbonyl group, and a more preferred example is a benzylcarbonyl group.

With respect to the acyl group in the "acyloxy group", the "acylthio group" and the "acylamino group", the same groups as mentioned above can be exemplified.

In the present description, the term "thiocarbonyl group which has a substituent" refers to a group represented by the formula: -C(=S)-A (wherein A represents a heterocyclic group which may have a substituent, -OR, -SR, $-NH_2$, -NHR, or $-NR_2$, and R represents the same as defined above).

Preferable examples of the "substituted thiocarbonyl group" include an aryloxythiocarbonyl group and a heteroarylthiocarbonyl group.

Examples of the aryloxythiocarbonyl group include a ($C_{6-14}$ aryl) oxythiocarbonyl group such as a $-C(=S)-OC_6H_4-CH_3$ group.

Examples of the heteroarylthiocarbonyl group include a $C_{1-10}$ heteroarylthiocarbonyl group such as a -C(=S)-imidazolyl group (e.g., a -C(=S)-(1,3-imidazolyl) group).

In the present description, the term "sulfonyl group which has (having) a substituent" refers to a group represented by the formula: $-S(=O)_2R$ (wherein R is as defined above).

The sulfonyl group which has a substituent includes a sulfonyl group which has an alkyl group which may have a substituent, and a sulfonyl group which has an aryl group which may have a substituent. A preferred example of the sulfonyl group which has an alkyl group is a $C_{1-6}$-alkylsulfonyl group, and a more preferred example is a methanesulfonyl group or an ethanesulfonyl group.

A preferred example of the sulfonyl group which has an aryl group is a $C_{6-14}$-arylsulfonyl group, and a more preferred example is a benzenesulfonyl group or a p-toluenesulfonyl group.

In the present description, the term "silyl group which has (having) a substituent" refers to a silyl group having such a structure that at least one hydrogen atom in a silyl group ($-SiH_3$) is substituted by another atom or an atomic group.

A typical example of the "silyl group which has a substituent" is a group represented by the formula: $-Si(R^8)_3$ (wherein $R^8$'s are the same as or different from each other and independently represent an alkyl group or an aryl group). Examples of the group include a trialkylsilyl group (e.g., a tri-$C_{1-6}$-alkylsilyl group such as a trimethylsilyl group, a triethylsilyl group, a triisopropylsilyl group and a t-butyldimethylsilyl group), a dialkylarylsilyl group (e.g., a di-$C_{1-6}$-alkyl-$C_{6-14}$-arylsilyl group such as a dimethyl(phenyl)silyl group), an alkyldiarylsilyl group (e.g., a $C_{1-6}$-alkyldi-$C_{6-14}$-arylsilyl group such as a t-butyldiphenylsilyl group), and a triarylsilyl group (e.g., a tri-$C_{6-14}$-arylsilyl group such as a triphenylsilyl group).

In the present description, the term "protecting group for an amino group" refers to a monovalent group which can prevent an amino group from being involved in a reaction in the synthesis of a compound or a salt thereof or the synthesis of an oligonucleotide or a salt thereof.

The protecting group for an amino group is, for example, but is not limited to, a group which is stable under an acidic or neutral condition and can be cleaved by a method such as a hydrogenolysis, a hydrolysis, an electrolysis and a photodissociation.

Examples of the protecting group for an amino group include an acyl group which may have a substituent (e.g., an alkylcarbonyl group which may have a substituent, an arylcarbonyl group which may have a substituent, an aralkylcarbonyl group which may have a substituent), an N,N-dialkylformamidyl group which may have a substituent, an alkoxycarbonyl group which may have a substituent, an alkenyloxycarbonyl group which may have a substituent, an aryloxycarbonyl group which may have a substituent, an aralkyloxycarbonyl group which may have a substituent, and a sulfonyl group which has a substituent.

In the present description, the term "formamidyl group" refers to a group represented by the formula: $=CH-NH_2$. A preferred example of an N,N-dialkylformamidyl group is an N,N-di($C_{1-6}$-alkyl)formamidyl group, a more preferred

example is an N,N-di($C_{1-4}$-alkyl)formamidyl group, and a still more preferred example is an N,N-dimethylformamidyl group or an N,N-diethylformamidyl group.

In the present description, the term "alkoxycarbonyl group" refers to a group represented by the formula: -C(=O)-O-alkyl.

A preferred example of the alkoxycarbonyl group is a ($C_{1-6}$-alkoxy)carbonyl group, and a more preferred example is a methoxycarbonyl group, an ethoxycarbonyl group, a propoxycarbonyl group, or a butoxycarbonyl group (e.g., a t-butoxycarbonyl group).

In the present description, the term "alkenyloxycarbonyl group" refers to a group represented by the formula: -C(=O)-O-alkenyl.

A preferred example of the alkenyloxycarbonyl group is a ($C_{2-9}$-alkenyloxy)carbonyl group, and a more preferred example is an allyloxycarbonyl group.

In the present description, the term "aryloxycarbonyl group" refers to a group represented by the formula: -C(=O)-O-aryl.

A preferred example of the aryloxycarbonyl group is a ($C_{6-14}$-aryloxy)carbonyl group, and a more preferred example is a phenoxycarbonyl group or a naphthoxycarbonyl group.

In the present description, the term "aralkyloxycarbonyl group" refers to a group represented by the formula: -C(=O)-O-aralkyl.

A preferred example of the aralkyloxycarbonyl group is a ($C_{6-14}$-aryl-$C_{1-4}$-alkoxy)carbonyl group, and a more preferred example is a benzyloxycarbonyl group or a fluorenylmethyloxycarbonyl group.

The term "functional molecule unit substituent" as used herein refers to a concept including a labeling functional group (e.g., a fluorescently labeling functional group, a chemoluminescently labeling functional group, a group containing a radiation-emitting nuclear species), a group having intercalating capability, a group having capability of binding to a nucleic acid, a functional group having capability of cleaving a nucleic acid, a group having cellular or nuclear translocation capability, a group having metal chelating capability, and the like.

Examples of the fluorescently labeling functional group include residues of fluorescently labeling reagents such as carboxyfluorescein (FAM), fluorescein isothiocyanate (FITC), carboxytetramethylrhodamine (TAMRA) and thiazole orange (1-methyl-4-[(3-methyl-2(3H)-benzothiazolylidene)methyl]quinolinium p-tosylate).

Examples of the chemoluminescently labeling functional group include residues of chemoluminescently labeling reagents such as tris(bipyridine)ruthenium (II) chloride. Examples of the group containing a radiation-emitting nuclear species include groups each containing $^{11}CH_3$-, $^{14}CH_3$-, $^{18}F$- or $^{32}P$-.

Examples of the group having intercalating capability include a group which has an anthracene skeleton, a group having a pyrene skeleton, a group having an anthraquinone skeleton, a group having an acridine skeleton and a group having a naphthalimide skeleton. Other example is a residue of an intercalating agent such as tamoxifen.

Examples of the group having capability of binding to a nucleic acid include residues of nucleic acid binding agents such as netropsin, distamine and PI (Pyrrole Imidazole) polyamide.

Examples of the functional group having capability of cleaving a nucleic acid include residues of nucleic acid cleaving agents such as endonucleases and a bis(bipyridine)chrysenequinone diimine rhodium (II) complex.

Examples of the group having cellular or nuclear translocation capability include residues of cellular or nuclear translocation signal peptides such as TAT (Twin-Arginine Translocation) signal peptide, polyarginine, GalNac (N-acetylgalactosamine) and a signal peptide derived from SV40T antigen.

Examples of the group having meal chelating capability include residues of metal chelating agents such as EDTA, crown ether and a cryptand.

In the present description, the wording "hybridize" refers to a matter that the full length or a part of a given polynucleotide or oligonucleotide and the full length or a part of another polynucleotide or oligonucleotide together form a double strand through hydrogen bonds under a stringent condition. The "stringent condition" may be one which has been commonly employed by a person skilled in the art in the hybridization of a polynucleotide or an oligonucleotide. For example, when there is at least 50%, preferably at least 75%, more preferably at least 90% sequence identity between two polynucleotide or oligonucleotide molecules, the "stringent condition" may be a condition where one of the polynucleotide or oligonucleotide molecules can hybridize with the other specifically. It is known that the stringency in the hybridization is a function of a temperature, a salt concentration, a nucleotide length and a GC content in a polynucleotide or oligonucleotide, and the concentration of a chaotropic agent in a hybridization buffer solution. For example, as the stringent condition, a condition described in Sambrook, J. et. al., 1998, Molecular Cloning: A Laboratory Manual (second edition), Cold Spring Harbor Laboratory Press, New York can be employed.

In the present description, the term "test" refers to the matter that an analyte, e.g., a nucleic acid, in a specimen is examined for the purpose of diagnosis, research or the like. The term "test specimen" refers to a specimen to be subjected to a test.

In the present description, the upper limit and the lower limit of a numerical range may be combined arbitrarily.

2. Compound represented by formula (1) or salt thereof

The compound or the salt thereof according to the present invention is a compound represented by formula (1) or a salt thereof:

[Formula 9]

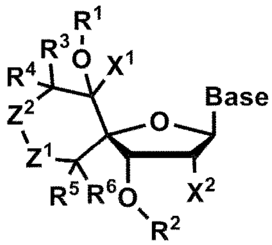

(1)

(wherein "Base", $X^1$, $X^2$, $R^1$, $R^2$, $R^3$, $R^4$, $R^5$, $R^6$, $Z^1$, and $Z^2$ are as defined above).

As shown in formula (1), the compound or the salt thereof according to the present invention has a 1,7-dioxaspiro[4.4]nonane skeleton, a 1,7-dioxaspiro[4.5]decane skeleton, a 1-oxa-7-azaspiro[4.4]nonane skeleton, or a 1-oxa-7-azaspiro[4.5]decane skeleton.

Hereinbelow, a compound represented by formula (N) or a salt thereof refers to a "compound (N)". A compound (1) is referred to as a "nucleotide" when $R^1$ or $R^2$ represents a dihydroxyphosphinyl group which may have a substituent or a hydroxymercaptophosphinyl group which may have a substituent, and is referred to as a "nucleoside" when $R^1$ and $R^2$ each represent another group.

"Base" is preferably an aromatic heterocyclic group which may have a substituent.

The aromatic heterocyclic group is preferably a nitrogenated aromatic heterocyclic group.

The nitrogenated aromatic heterocyclic group is preferably a 6- to 10-membered nitrogenated aromatic heterocyclic group. The 6- to 10-membered nitrogenated aromatic heterocyclic group is preferably a 2,4-dioxo-1,2,3,4-tetrahydropyrimidin-1-yl group, a 2-oxo-1,2-dihydropyrimidin-1-yl group, a purin-9-yl group, or a 6-oxo-1,6-dihydro-9H-purin-9-yl group.

The substituent which replaces in the aromatic heterocyclic group is preferably at least one selected from the group consisting of an alkyl group, an acyl group, and an amino group which may be substituted by a protecting group for an amino group. The number of the substituent(s) is not particularly limited, and is for example 1 to 3.

"Base" is more preferably a thyminyl group which may have a substituent, a cytosinyl group which may have a substituent, an adenyl group which may have a substituent, or a guanyl group which may have a substituent. The substituent is preferably at least one selected from the group consisting of an alkyl group, an acyl group, and an N,N-dialkylformamidyl group, more preferably a $C_{1-4}$-alkyl group, a ($C_{1-4}$-alkyl)carbonyl group, a ($C_{6-14}$-aryl)carbonyl group, or an N,N-di($C_{1-4}$-alkyl)formamidyl group. The number of the substituent(s) is preferably 1 to 3.

"Base" is more preferably a group selected from the group consisting of:

a 2,4-dioxo-5-methyl-1,2,3,4-tetrahydropyrimidin-1-yl group (e.g., a thymin-1-yl group),
a 2-oxo-4-amino-1,2-dihydropyrimidin-1-yl group (i.e., a cytosin-1-yl group),
a 2-oxo-4-acylamino-1,2-dihydropyrimidin-1-yl group (i.e., an N-acyl-cytosin-1-yl group),
a 2-oxo-4-amino-5-methyl-1,2-dihydropyrimidin-1-yl group (i.e., a 5-methylcytosin-1-yl group),
a 2-oxo-4-acylamino-5-methyl-1,2-dihydropyrimidin-1-yl group (i.e., an N-acyl-5-methylcytosin-1-yl group),
a 6-amino-9H-purin-9-yl group (i.e., an adenin-9-yl group),
a 6-acylamino-9H-purin-9-yl group (i.e., an N-acyl-adenin-9-yl group),
a 2-amino-6-oxo-1,6-dihydro-9H-purin-9-yl group (e.g., a guanin-9-yl group),
a 2-acylamino-6-oxo-1,6-dihydro-9H-purin-9-yl group (e.g., an N-acyl-guanin-9-yl group), and
a 2-(N,N-dialkylformamidyl)amino-6-oxo-1,6-dihydro-9H-purin-9-yl group (e.g., an N-(N,N-dialkylformamidyl)-guanin-9-yl group).

"Base" is even more preferably a group selected from the group consisting of:

a thymin-1-yl group,
a 5-methylcytosin-1-yl group,
an N-acetyl-5-methylcytosin-1-yl group,
an N-isobutyryl-5-methylcytosin-1-yl group,

an N-benzoyl-5-methylcytosin-1-yl group,
an adenin-9-yl group,
an N-acetyl-adenin-9-yl group,
an N-isobutyryl-adenin-9-yl group,
an N-benzoyl-adenin-9-yl group,
a guanin-9-yl group,
an N-acetyl-guanin-9-yl group,
an N-isobutyrylguanin-9-yl group,
an N-benzoyl-guanin-9-yl group, and
an N-(N,N-dimethylformamidyl)-guanin-9-yl group.

$X^1$ is preferably a hydrogen atom, an alkyl group, an alkenyl group, an alkynyl group, a cycloalkyl group, or an aryl group, more preferably a hydrogen atom, an alkyl group, or an aryl group, still more preferably a hydrogen atom, or an alkyl group, even more preferably a hydrogen atom or a $C_{1-4}$ alkyl group, and particularly preferably a hydrogen atom, a methyl group, or an ethyl group. In one embodiment, $X^1$ is preferably a hydrogen atom.

In "-$OR^7$" indicated by $X^2$, $R^7$ is preferably a hydrogen atom, an alkyl group, an alkenyl group, an alkynyl group, a cycloalkyl group, a cycloalkenyl group, an aryl group, or a protecting group of a hydroxy group, more preferably a hydrogen atom, an alkyl group, an aryl group, an alkylcarbonyl group, or an arylcarbonyl group, still more preferably a hydrogen atom, an alkyl group, or an alkylcarbonyl group, even more preferably a hydrogen atom, a $C_{1-4}$ alkyl group, or a $C_{1-4}$ alkylcarbonyl group, and particularly preferably a hydrogen atom, or a $C_{1-4}$ alkyl group.

$R^1$ and $R^2$ are each preferably selected from the group consisting of:

a hydrogen atom,
an alkyl group which may have a substituent,
an aryl group which may have a substituent,
a protecting group for a hydroxy group,
a phosphino group which has a substituent,
a dihydroxyphosphinyl group which may have a substituent, and
a hydroxymercaptophosphinyl group which may have a substituent.

The "alkyl group which may have a substituent" in the above group is preferably an alkyl group which may have at least one substituent selected from the group consisting of a halogen atom, an alkoxy group and an aryl group, more preferably an alkyl group which may be substituted by an alkoxy group or an aralkyl group which may be substituted by an alkoxy group. The number of the substituent(s) is preferably 1 to 3.

The "aryl group which may have a substituent" in the above group is preferably an aryl group which may have at least one substituent selected from the group consisting of a halogen atom, an alkyl group and an alkoxy group, more preferably an aryl group which may be substituted by an alkoxy group. The number of the substituent(s) is preferably 1 to 3.

The "protecting group for a hydroxy group" in the above group is preferably an alkylcarbonyl group, an arylcarbonyl group, an alkylsulfonyl group, an arylsulfonyl group, or a group represented by the formula: $-Si(R^8)_3$ (wherein $R^8$'s are the same as or different from each other and independently represent an alkyl group or an aryl group).

The "phosphino group which has a substituent" in the above group is preferably a group represented by the formula: $-P(R^{9a})(R^{9b})$ (wherein $R^{9a}$ and $R^{9b}$ are the same as or different from each other and independently represent a hydroxy group, a mercapto group, an amino group, an alkoxy group, a haloalkoxy group, a cyanoalkoxy group, an alkylthio group, a haloalkylthio group, a cyanoalkylthio group, or an alkylamino group), and more preferably a phosphino group represented by the following formula:

[Formula 10]

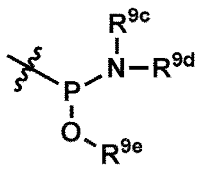

(wherein $R^{9c}$ and $R^{9d}$ are the same as or different from each other and independently represent a hydrogen atom or an alkyl group; and $R^{9e}$ represents a hydrogen atom, an alkyl group, a haloalkyl group, or a cyanoalkyl group).

The "dihydroxyphosphinyl group which may have a substituent" in the above group is preferably a dihydroxyphosphinyl group, a diphosphate group, or a triphosphate group, more preferably a dihydroxyphosphinyl group. These groups may have a protecting group for a hydroxy group as a substituent, in which each of all or some of hydroxy groups may be substituted by a protecting group for a hydroxy group.

The "hydroxymercaptophosphinyl group which may have a substituent" in the above group is preferably a hydroxymercaptophosphinyl group.

$R^1$ and $R^2$ are each more preferably a hydrogen atom, a methyl group, an ethyl group, a propyl group, a butyl group, an allyl group, a benzyl group, a trityl group, a methoxymethyl group, a p-methoxybenzyl group, a monomethoxytrityl group, a dimethoxytrityl group, an acetyl group, an isobutyryl group, a benzoyl group, a methanesulfonyl group, a p-toluenesulfonyl group, a trimethylsilyl group, a triethylsilyl group, a triisopropylsilyl group, a t-butyldimethylsilyl group, a t-butyldiphenylsilyl group, a phosphino group represented by either one of the following formulae:

[Formula 11]

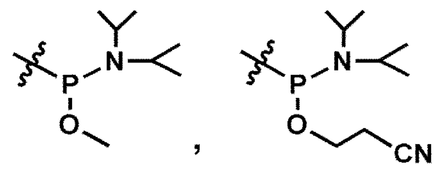

, a dihydroxyphosphinyl group, or a hydroxymercaptophosphinyl group.

The combination of $R^1$ and $R^2$ is preferably a combination in which R1 is a hydrogen atom or an aralkyl group (e.g., a $C_{7-19}$ aralkyl group such as a trityl group) which may be substituted by an alkoxy group (e.g., a $C_{1-4}$ alkoxy group such as a methoxy group and an ethoxy group) and $R^2$ is a hydrogen atom, an aralkyl group (e.g., a $C_{7-19}$ aralkyl group such as a benzyl group), or a phosphino group represented by the following formula:

[Formula 12]

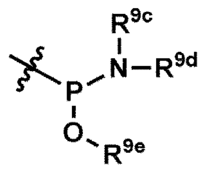

(wherein $R^{9c}$ and $R^{9d}$ are the same as or different from each other and independently represent a hydrogen atom or an alkyl group; and $R^{9e}$ represents a hydrogen atom, an alkyl group, a haloalkyl group, or a cyanoalkyl group).

The combination of $R^1$ and $R^2$ is more preferably a combination in which $R^1$ is a dimethoxytrityl group such as a 4,4'-dimethoxytrityl group and $R^2$ is a phosphino group represented by either one of the following formulae.

[Formula 13]

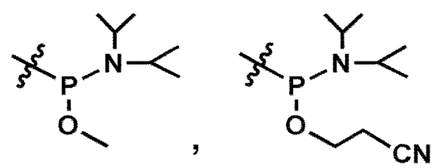

$R^3$, $R^4$, $R^5$, and $R^6$ are each preferably a hydrogen atom, an alkyl group, an alkenyl group, an alkynyl group, a cycloalkyl group, or an aryl group, more preferably a hydrogen atom, an alkyl group, or an aryl group, still more preferably a hydrogen atom or an alkyl group, even more preferably a hydrogen atom or a $C_{1-4}$ alkyl group, and particularly preferably a hydrogen atom, a methyl group, or an ethyl group. In one embodiment, $R^3$, $R^4$, $R^5$, and $R^6$ are each preferably a hydrogen atom.

In one embodiment, $Z^1$ is preferably O. In another embodiment, $Z^1$ is preferably $NZ^{11}$. In this embodiment, $Z^{11}$ is preferably a hydrogen atom, an alkyl group, an alkenyl group, an alkynyl group, a cycloalkyl group, an aryl group, an aralkyl group, or a protecting group for an amino group, more preferably a hydrogen atom or an alkyl group, still more preferably a hydrogen atom or a $C_{1-4}$ alkyl group, and particularly preferably a hydrogen atom, a methyl group, or an

ethyl group.

In one embodiment, $Z^2$ is preferably a single bond. In this embodiment, $Z^1$ is preferably O. In another embodiment, $Z^2$ is preferably $CZ^{21}Z^{22}$. In this embodiment, $Z^{21}$ and $Z^{22}$ are each preferably a hydrogen atom, an alkyl group, an alkenyl group, an alkynyl group, a cycloalkyl group, or an aryl group, more preferably a hydrogen atom or an alkyl group, still more preferably a hydrogen atom or a $C_{1-4}$ alkyl group, and particularly preferably a hydrogen atom, a methyl group, or an ethyl group. In this embodiment, $Z^{21}$ and $Z^{22}$ are each preferably a hydrogen atom ($Z^2$ is $CH_2$).

The compound (1) is preferably any of compounds (1A), (1B), and (1C):

[Formula 14]

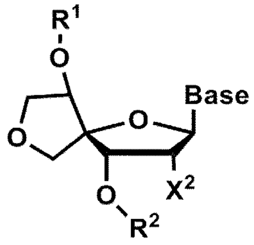

(1A)

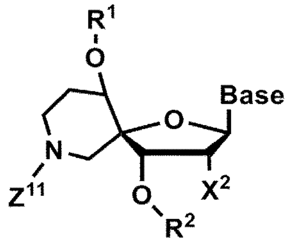

(1B)

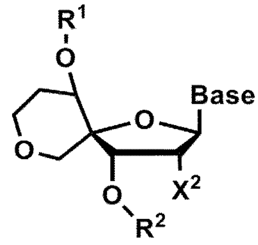

(1C)

(wherein "Base", $X^2$, $R^1$, $R^2$, and $Z^{11}$ are as defined above).

The compound (1) may be a stereoisomer (R-isomer, S-isomer, α-isomer, β-isomer, enantiomer, or diastereomer) or a racemate when an asymmetric carbon is present. For example, the compound (1) may be any of compounds (1A-1), (1A-2), (1B-1), and (1C-1):

[Formula 15]

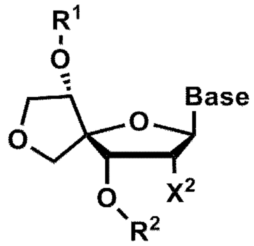

(1A-1)

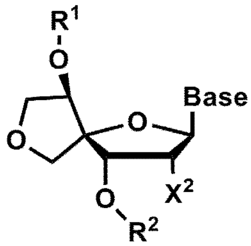

(1A-2)

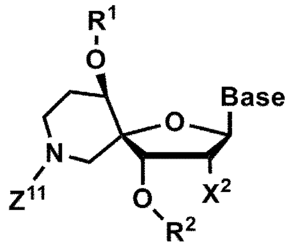

(1B-1)

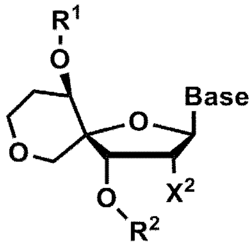

(1C-1)

(wherein "Base", $X^2$, $R^1$, $R^2$, and $Z^{11}$ are as defined above).

The salt may be a pharmaceutically acceptable salt or may not be a pharmaceutically acceptable salt. The salt may be an inorganic salt or an organic salt.

Examples of the salt include an alkali metal salt (e.g., a sodium salt, a potassium salt, a lithium salt), an alkaline earth metal salt (e.g., a calcium salt, a magnesium salt), another metal salt (e.g., an aluminium salt, az iron salt, a zinc salt, a copper salt, a nickel salt, a cobalt salt), an ammonium salt, a tetramethylammonium salt, an amire salt (e.g., a t-octylamine salt, a dibenzylamine salt, a morpholine salt, a glucosamine salt, a phenylglycine alkyl ester salt, an ethylenediamine salt, an N-methylglucamine salt, a guanidine salt, a diethylamine salt, a triethylamine salt, a dicyclohexylamine salt, an N,N'-dibenzylethylenediamine salt, a chloroprocaine salt, a procaine salt, a diethanolamine salt, an N-benzyl-phenethylamine salt, a piperazine salt, a tris(hydroxymethyl)aminomethane salt), an inorganic acid salt (e.g., a hydrofluoric acid salt, a hydrochloric acid salt, a hydrobromic acid salt, a hydroiodic acid salt, a nitric acid salt, a perchloric acid salt, a sulfuric acid salt, a phosphoric acid salt), an organic acid salt (e.g., a methanesulfonic acid salt, a trifluoromethanesulfonic acid salt, an ethanesulfonic acid salt, a benzenesulfonic acid salt, a p-toluenesulfonic acid salt, an acetic acid salt, a malic acid salt, a fumaric acid salt, a succinic acid salt, a citric acid salt, a tartaric acid salt, an oxalic acid salt, a maleic acid salt), and an amino acid salt (e.g., a glycine acid, a lysine acid, an arginine salt, an ornithine salt, a glutamic acid salt, an aspartic acid salt).

3. Method for producing compound (1)

The compound (1) can be produced by, for example, a method including cyclization (or ring closure) of a compound (2) in the presence or absence of an amine represented by the formula (2'): $Z^{11}$-$NH_2$ (wherein $Z^{11}$ is as defined above), as shown below, but not limited to this method, the compound (1) can be synthesized using known reactions:

[Formula 16]

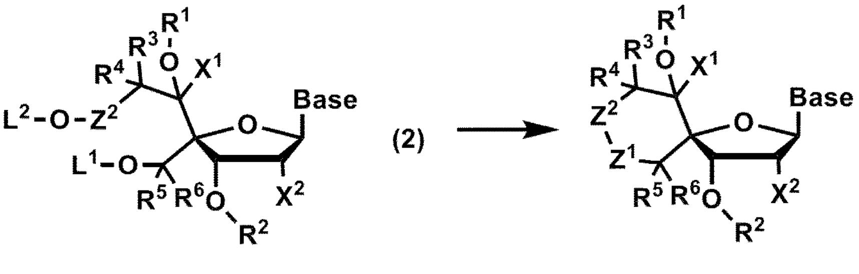

(2)

(1)

(wherein $L^1$ is a hydroxy activating group, $L^2$ is a hydrogen atom or a hydroxy activating group, and Base, $X^1$, $X^2$, $R^1$, $R^2$, $R^3$, $R^4$, $R^5$, $R^6$, $Z^1$, and $Z^2$ are as defined above).

In the compound (2), examples of the hydroxy activating group represented by $L^1$ include an alkylsulfonyl group (e.g., a $C_{1-4}$ alkylsulfonyl group such as a mesyl group), a haloalkylsulfonyl group (e.g., a fluoro $C_{1-4}$ alkanesulfonyl group such as a trifluoromethanesulfonyl group), an arylsulfonyl group (e.g., a $C_{6-10}$ arylsulfonyl group such as a benzenesulfonyl group, a nitrobenzenesulfonyl group, a tosyl group). When $L^2$ is a hydroxy activating group, examples of the hydroxy activating group include the same as the examples of $L^1$. $L^2$ may be the same as or different from $L^1$.

The compound (2) can be obtained by a method including a step of hydroxylation(e.g., hydroxylation with an oxidizing agent such as osmium tetroxide) a compound represented by the following formula (3):

[Formula 17]

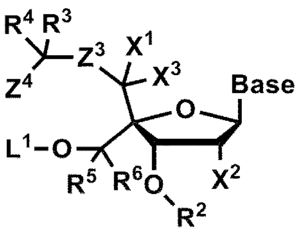

(3)

(wherein $Z^3$ is a single bond or a double bond. When $Z^3$ is a single bond, $X^3$ is a $OR^1$, $Z^4$ is $CH_2$=$CZ^5$-, and $Z^5$ is an atomic hydrogen, an alkyl group which may have a substituent, an alkenyl group which may have a substituent, an alkynyl group may have a substituent, a cycloalkyl group which may have a substituent, or an aryl group which may have a substituent. When $Z^3$ is a double bond, $X^3$ and $Z^4$ are absent, and "Base", $L^1$, $X^1$, $X^2$, $R^1$, $R^2$, $R^3$, $R^4$, $R^5$, and $R^6$ are as defined above), but is not limited to this method, and can be synthesized using known reactions.

A compound (3) can be synthesized by a method including a step of oxidization of a compound represented by the following formula (4):

[Formula 18]

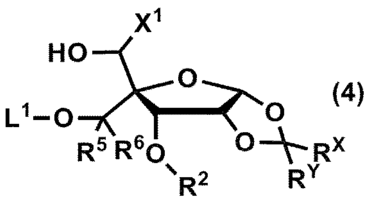

(4)

(wherein $L^1$, $X^1$, $R^2$, $R^5$, $R^6$, $R^X$, and $R^Y$ are as defined above), with an oxidizing agent (e.g., swern oxidation using

dimethyl sulfoxide as an oxidizing agent and using oxalylchloride or the like as an activating agent), followed by a step of olefination such as a Wittig reaction and a Grignard reaction, and a step of introduction of a nucleobase.

$R^X$ and $R^Y$ are each preferably a hydrogen atom, an alkyl group, an alkenyl group, an alkynyl group, a cycloalkyl group, or an aryl group, more preferably a hydrogen atom, an alkyl group, or an aryl group, still more preferably an alkyl group, and even more preferably a $C_{1-4}$ alkyl group such as a methyl group.

When the cyclization step of the compound (2) is carried out in the absence of an amine represented by the formula (2'): $Z^{11}$-$NH_2$ (wherein $Z^{11}$ is as defined above), the compound (1) in which $Z^1$ is O can be obtained. On the other hand, when the cyclization step of the compound (2) is performed in the presence of the amine, the compound (1) in which $Z^1$ is $NZ^{11}$ can be obtained. The amount of the amine to be used is, for example, 1 to 200 moles, preferably 50 to 100 moles, relative to 1 mole of the compound (2).

The cyclization step of the compound (2) may be carried out in the presence of a base. Examples of the base include an inorganic base [e.g., a carbonate salt of an alkali metal (e.g., sodium carbonate, potassium carbonate, cesium carbonate), a hydrogen carbonate salt of an alkali metal (e.g., sodium hydrogen carbonate), a carbonate salt of an alkaline earth metal (e.g., calcium carbonate), a hydroxide of an alkali metal (e.g., sodium hydroxide, potassium hydroxide), a hydroxide of an alkaline earth metal (e.g., calcium hydroxide), an alkali metal hydride (e.g., sodium hydride), a metal alkoxide (e.g., sodium methoxide, sodium ethoxide)], an organic base [e.g., a tertiary amine (e.g., trialkylamine, N-ethyldiisopropylamine), a cyclic amine (e.g., pyridine, e.g., 4-(dimethylamino)pyridine, diazabicycloundecene (DBU), diazabicyclononene (DBN))], and a combination of two or more of these bases.

The amount of the base to be used is, for example, 1 to 20 moles, preferably 1 to 10 moles, relative to 1 mole of the compound (2).

The cyclization step of compound (2) is preferably carried out in the presence of a solvent. Examples of the solvent include an ether-based solvent (e.g., tetrahydrofuran), an amide-based solvent (e.g., N,N-dimethylformamide, N,N-dimethylacetamide), a cyclic amine (e.g., pyridine, 2,4,6-trimethylpyridine).

In the cyclization step of the compound (2), the reaction temperature and the reaction time are not particularly limited as long as the reaction proceeds. The reaction temperature is, for example, 20 to 170°C, preferably 25 to 150°C or 60 to 120°C. The reaction time is, for example, 0.5 to 72 hours, 1 to 24 hours, or 6 to 12 hours.

"Base" of the compounds (1), (1A), (1B), (1C), (2), and (3) can be converted, for example, by the following scheme:

[Formula 19]

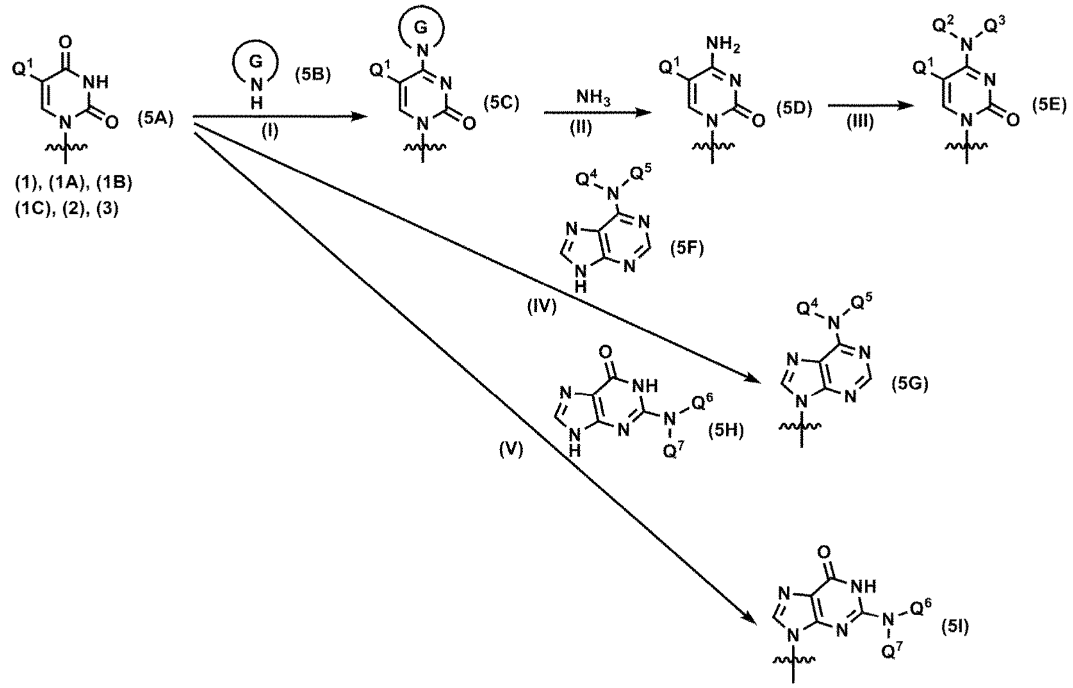


(wherein $Q^1$ represents a hydrogen atom or a substituent; $Q^2$ and $Q^3$ are the same as or different from each other and independently represent a hydrogen atom or a protecting group for an amino group (provided that $Q^2$ and $Q^3$ do not represent hydrogen atoms coincidentally); $Q^4$, $Q^5$, $Q^6$, and $Q^7$ are the same as or different from each other and independently represent a hydrogen atom or a protecting group for an amino group; and the ring G represents a 5- or 6-membered nitrogenated heterocyclic ring).

<Step (I)>

Step (I) is a step of reacting a compound (5A) whose "Base" is "a 2,4-dioxo-1,2,3,4-tetrahydropyrimidin-1-yl group which may have a substituent" with a compound (5B) and a phosphoric acid halide to obtain a compound (SC).

In the compound (5A), Q1 is preferably a hydrogen atom or an alkyl group, more preferably a hydrogen atom or a $C_{1-4}$ alkyl group.

The compound (5B) is preferably a 5-membered nitrogen-containing heterocyclic compound, more preferably triazole. The amount of the compound (5B) to be used is generally 5 to 20 moles, preferably 7 to 9 moles, relative to 1 mole of the compound (5A).

The phosphoric acid halide is preferably phosphoric acid trichloride. The amount of the phosphoric acid halide to be used is generally 1 to 5 moles, preferably 1 to 3 moles, relative to 1 mole of the compound (5A).

Step (I) is preferably carried out in the presence of a solvent. Examples of the solvent include a nitrile-type solvent (e.g., acetonitrile), an ether-type solvent (e.g., tetrahydrofuran), a halogen-based solvent (e.g., a haloalkane), and a mixed solvent composed of two or more of these solvents. Among these solvents, a nitrile-type solvent (e.g., acetonitrile) is preferred.

Step (I) is preferably carried out in the presence of a base. Examples of the base include an inorganic base [e.g., a carbonate salt of an alkali metal (e.g., sodium carbonate, cesium carbonate), a hydrogen carbonate salt of an alkali metal (e.g., sodium hydrogen carbonate), a carbonate salt of an alkaline earth metal (e.g., calcium carbonate), a hydroxide of an alkali metal (e.g., sodium hydroxide, potassium hydroxide), a hydroxide of an alkaline earth metal (e.g., calcium hydroxide), a metal alkoxide (e.g., sodium methoxide, sodium ethoxide)], an organic base [e.g., a tertiary amine (e.g., trialkylamine), a cyclic amine (e.g., 4-(dimethylamino)pyridine, diazabicycloundecene (DBU), diazabicyclononene (DBN))], and a combination of two or more of these bases. Among these bases, a tertiary amine is preferred, and a tri-$C_{1-4}$-alkylamine is more preferred. The amount of the base to be used is generally 5 to 20 moles, preferably 10 to 15 moles, relative to 1 mole of the compound (5A).

The reaction temperature for the reaction in step (I) is not particularly limited as long as the reaction can proceed, and is, for example, -5°C to 10°C.

Step (I) can also be carried out, for example, in the manner described in US Patent No. 5359067.

<Step (II)>

Step (II) is a step of reacting compound (SC) with ammonia to obtain compound (5D).

The amount of ammonia to be used is generally 5 to 100 moles, preferably 20 to 50 moles, relative to 1 mole of the compound (5C).

Step (II) is preferably carried out in the presence of a solvent. Examples of the solvent include an amide-type solvent (e.g., N,N-dimethylformamide, N,N-dimethylacetamide, N-methylpyrrolidone), an ether-type solvent (e.g., a cyclic ether such as tetrahydrofuran and dioxane), and a mixed solvent composed of two or more of these solvents. Among these solvents, an ether-type solvent is preferred, and a cyclic ether is more preferred, and at least one solvent selected from tetrahydrofuran and dioxane is still more preferred.

The reaction temperature for the reaction in step (II) is not particularly limited as long as the reaction can proceed, and is, for example, 15 to 30°C.

<Step (III)>

Step (III) is a step of protecting the amino group in the compound (5D) by a protecting group to obtain a compound (5E). As the method for protecting the amino group by the protecting group, a known method (for example, the method described in the description of US Patent Application Publication No. 2007/167387) or a conventional method may be employed.

<Step (IV)>

Step (IV) is a step of converting the "Base" from "a 2,4-dioxo-1,2,3,4-tetrahydropyrimidin-1-yl group which may have a substituent" to "a purin-9-yl group which may have a substituent" in the compound (5A).

More specifically, step (VI) is a step of reacting the compound (5A) wherein "Base" is "a 2,4-dioxo-1,2,3,4-tetrahydropyrimidin-1-yl group which may have a substituent" with a compound (5F) to obtain a compound (5G).

The amount of the compound (5F) to be used is generally 1 to 5 moles, preferably 1 to 3 moles, relative to 1

mole of the compound (5A).

Step (IV) is preferably carried out in the presence of a Lewis acid. Examples of Lewis acids include trimethylsilyl trifluoromethanesulfonate. The amount of the Lewis acid to be used is generally 0.5 to 5 moles, preferably 1 to 3 moles, relative to 1 mole of the compound (5A).

Step (IV) is preferably carried out in the presence of a silylating agent. Examples of the silylating agent include N,O-bis(trimethylsilyl)acetamide (BSA), N,O-bis-silyltrifluoroacetamide (BSTFA), hexamethyldisilazane (HMD), N,O-bis-tert-butyldimethylsilylacetamide, N-(trimethylsilyl)diethylamine, N-(trimethylsilyl)dimethylamine, N-methoxy-N,O-bis(trimethylsilyl)carbamate, N-methyl-N-trimethylsilylacetamide, N-methyl-N-trimethylsilylheptafluorobutyramide, N-methyl-N-trimethylsilyltrifluoroacetamide, N-trimethylsilylacetamide, and a combination of two or more of these compounds. Among these compounds, N,O-bis(trimethylsilyl)acetamide (BSA) is preferred. The amount of the silylating agent to be used is generally 1 to 20 moles, preferably 3 to 8 moles, relative to 1 mole of the compound (5A).

The reaction temperature for the reaction in step (IV) is not particularly limited as long as the reaction can proceed, and is for example, 30 to 150°C, preferably 50 to 120°C.

Step (IV) can also be carried out, for example, in the manner described in US Patent Application No. 2012/071646.

<Step (V)>

Step (V) is a step of converting "Base" from "a 2,4-dioxo-1,2,3,4-tetrahydropyrimidin-1-yl group which may have a substituent" to "a 6-oxo-1,6-dihydro-9H-purin-9-yl group which may have a substituent" in the compound (5A).

More specifically, step (V) is a step of reacting the compound (5A) wherein "Base" is "a 2,4-dioxo-1,2,3,4-tetrahydropyrimidin-1-yl group which may have a substituent" with a compound (5H) to obtain a compound (5I). The reaction can be carried out under the same conditions as those employed in step (IV).

If necessary, the method for producing the compound (1) may further include a step of purifying an intermediate product and a final product by a conventional method, such as concentration, recrystallization and silica gel column chromatography.

4. Composition containing compound (1)

The composition of the present invention contains the compound (1). The composition may contain one compound (1) or two or more compounds (1). For example, the composition may contain one, two, three or four compounds selected from the group consisting of:

a compound (1) wherein "Base" is a thymin-1-yl group;
a compound (1) wherein "Base" is a 5-methylcytosin-1-yl group, an N-acetyl-5-methylcytosin-1-yl group, an N-isobutyryl-5-methylcytosin-1-yl group, or an N-benzoyl-5-methylcytosin-1-yl group;
a compound (1) wherein "Base" is an adenin-9-yl group, an N-acetyl-adenin-9-yl group, an N-isobutyryl-adenin-9-yl group, or an N-benzoyl-adenin-9-yl group; and
a compound (1) wherein "Base" is a guanin-9-yl group, an N-acetyl-guanin-9-yl group, an N-isobutyrylguanin-9-yl group, an N-benzoyl-guanin-9-yl group, or an N-(N,N-dimethylformamidyl)-guanin-9-yl group.

An example of the form of the composition is a liquid form.

When the composition has a liquid form, the composition generally contains a solvent. As the solvent, a known solvent can be used, and preferred examples of the solvent include a halogenated hydrocarbon-type solvent (e.g., dichloromethane), a nitrile-type solvent (e.g., acetonitrile), an aromatic hydrocarbon-type solvent (e.g., toluene, xylene), water, and a TE buffer. Among these solvents, dichloromethane, toluene, and acetonitrile are more preferably used.

The composition is generally provided to a user in a form packed in a container.

The composition can be used for the below-mentioned synthesis of an oligonucleotide or a salt thereof. The composition can also be used as a pharmaceutical composition.

5. Oligonucleotides or salt thereof

The oligonucleotide or the salt thereof according to the present invention has a unit represented by formula (6):

[Formula 20]

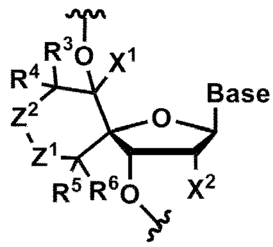

(6)

(wherein "Base", $X^1$, $X^2$, $R^3$, $R^4$, $R^5$, $R^6$, $Z^1$, and $Z^2$ are as defined above).

The unit is preferably a unit represented by formula (6a):

[Formula 21]

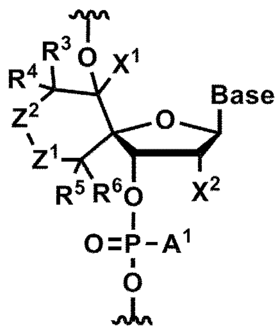

(6a)

(wherein $A^1$ represents OH, $O^-$, SH or $S^-$; and "Base", $X^1$, $X^2$, $R^3$, $R^4$, $R^5$, $R^6$, $Z^1$, and $Z^2$ are as defined above).

Hereinbelow, an oligonucleotide having a unit represented by formula (6) or (6a) or a salt thereof is referred to as an "oligonucleotide (6)".

When the oligonucleotide (6) has at least two units each represented by formula (6) or (6a), the structures of the units may be the same as or different from each other.

The oligonucleotide (6) may further contain another unit, in addition to the unit represented by formula (6) or (6a). An example of the other unit is at least one unit selected from units respectively represented by formulae (7) to (10):

[Formula 22]

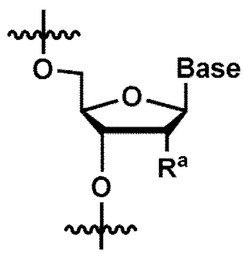

(7) ,

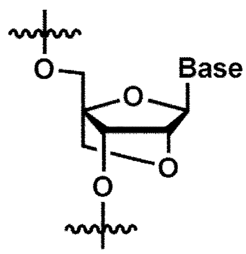

(8) ,

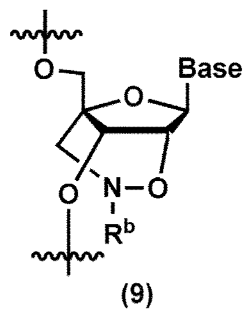

(9) ,

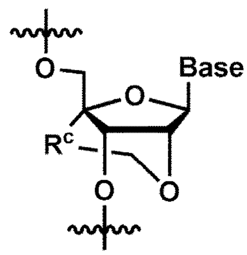

(10)

[wherein $R^a$ is a hydrogen atom or a hydroxy group,

$R^b$ is a hydrogen atom, an alkyl group which may have a substituent, an alkenyl group which may have a substituent, a cycloalkyl group which may have a substituent, an aryl group which may have a substituent, an acyl group which may have a substituent, a sulfonyl group which may have a substituent, a silyl group which has a substituent, a functional molecular unit substituent, or a group represented by a formula $R^{b1}$-X- (wherein $R^{b1}$ is an amino group which may have a substituent, and X is an alkylene group which may have a substituent or a group in which at least one methylene group in the alkylene group is replaced by -N ($R^{b2}$)- (wherein $R^{b2}$ is a hydrogen atom or an alkyl group), -O-, or $-S(=O)_k-$ (wherein k is 0, 1, or 2),

$R^c$ is the same and different and is a single bond or an alkylene group which may have a substituent, and "Base" is as defined above].

The other unit is preferably at least one unit selected from units respectively represented by formulae (7a) to (10a):

[Formula 23]

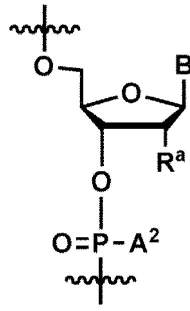
(7a) ,
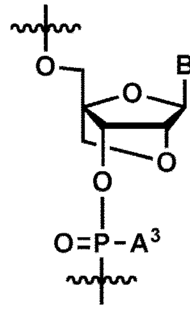
(8a) ,
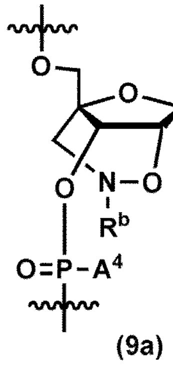
(9a) ,
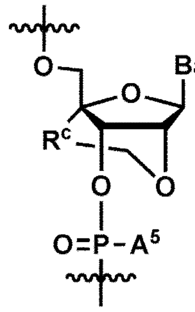
(10a)

(wherein $A^2$, $A^3$, $A^4$, and $A^5$ are the same as or different from each other and independently represent OH, $O^-$, SH, or $S^-$, and "Base", $R^a$, $R^b$, and $R^c$ are as defined above).

For example, the other unit may be a unit derived from any one of the nucleotides described in the descriptions of US Patent Application Publication No. 2003/105309, US Patent Application Publication No. 2017/044528, US Patent Application Publication No. 2006/166908, US Patent Application Publication No. 2012/208991, US Patent Application Publication No. 2015/266917, and US Patent Application Publication No. 2003/207841 (the entire contents thereof are incorporated herein by reference).

The nucleotide sequence for the oligonucleotide (6) is not particularly limited, as long as the nucleotide sequence is complementary to (the full length or a part of) the nucleotide sequence for target DNA or target RNA.

The length of the oligonucleotide (6) is not particularly limited, and can be selected depending on the length of the nucleotide sequence for a target. The lower limit of the length of the oligonucleotide (6) is, for example 5-mer, preferably 10-mer, more preferably 15-mer, and the upper limit of the length of the oligonucleotide (6) is, for example 200-mer, preferably 100-mer, more preferably 50-mer, still more preferably 30-mer. The length of the oligonucleotide (6) is, for example 5- to 200-mer, preferably 5- to 50-mer, more preferably 10- to 40-mer, more preferably 15- to 30-mer. The binding force of the oligonucleotide (6) to the nucleotide sequence for a target becomes stronger with the increase in the length of the oligonucleotide (6).

In the oligonucleotide (6), the ratio of the number of the units represented by formula (6) to the total number of the nucleotide units is not particularly limited, and can be designed appropriately depending on the intended use (e.g., as a primer, a probe, clamp nucleic acid, a medicine). By increasing the ratio of the number of units represented by the formula (6), the binding strength with the complementary strand can be reduced, and the oligonucleotide (6) can be used, for example, as a double-stranded nucleic acid such as siRNA promoted to be dissociated in vivo, or as a clamp nucleic acid having a site that is not bound to a template in a clamp PCR method.

The oligonucleotide (6) may be in the form of a salt. In other words, at least one nucleotide unit among the nucleotide units constituting the oligonucleotide (6) may be in the form of a salt. The salt may be a pharmaceutically acceptable salt or may not be a pharmaceutically acceptable salt. The salt may be an inorganic salt or an organic salt. As in the case of the salt exemplified for the compound (1), examples of the salt include an alkali metal salt, an alkaline earth metal salt, another metal salt, an ammonium salt, a tetramethylammonium salt, an amine salt, an inorganic acid salt, an organic acid salt, and an amino acid salt.

The oligonucleotide (6) may be modified with a labeling substance. The labeling substance is not particularly limited, and may be a substance known in the art as a label to be attached to a nucleic acid, such as a fluorescent substance, a hapten (e.g., biotin, digoxigenin, DNP), and a radioisotope.

The oligonucleotide (6) has sequence specificity. The oligonucleotide (6) can be utilized suitably as a probe for examining the nucleotide sequence for single-stranded RNA or single-stranded DNA or for detecting single-stranded RNA or single-stranded DNA in a sequence-selective manner.

The oligonucleotide (6) is less likely to be decomposed with a nuclease. Therefore, the oligonucleotide (6) can be present in a living body for a longer period after the administration to the living body. For example, the oligonucleotide

(6) can form a double strand with sense RNA to inhibit the transcription of mRNA for a biological component (a protein) that may cause a disease. The oligonucleotide (6) can also inhibit the proliferation of a virus with which the living body has been infected.

The oligonucleotide (6) is useful as a therapeutic medicine, such as an anti-tumor agent and anti-viral agent, which can inhibit the activity of a gene.

The oligonucleotide (6) also has a stable and excellent activity for use as an antisense or antigene or an aptamer, or has an excellent activity for use as a detecting drug for a specific gene or a primer for the initiation of the nucleic acid amplification of a specific gene.

The oligonucleotide (6) is useful as a physiological/bioactive substance, a material for a medicine, a functional material for a double-stranded oligonucleotide for use in an RNA interference method, a decoy method or the like, a functional material such as a DNA chip that targets a single-stranded nucleic acid (e.g., cDNA), a molecular beacon or the like, a functional material for genome editing use in various antisense methods (including a ribozyme or a DNA-zyme), antigene methods, crispr-cas9, or the like, a material which can be used in combination with a fluorescence or a light-emitting substance in a highly sensitive analysis of a biological trace component, or a material for use in the development of a reagent for research use (e.g., the elucidation of the function of a gene).

6. Method for producing oligonucleotide (6)

The oligonucleotide (6) can be synthesized in accordance with a conventional method such as a phosphoramidite protocol.

For example, the method for producing the oligonucleotide (6) includes:

(I) a step of reacting a compound represented by formula (6B):

[Formula 24]

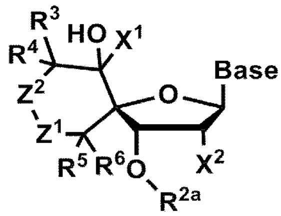

(6B)

(wherein $R^{2a}$ represents an alkyl group which may have a substituent, an alkenyl group which may have a substituent, a cycloalkyl group which may have a substituent, a cycloalkenyl group which may have a substituent, an aryl group which may have a substituent, a protecting group for a hydroxy group, a phosphino group which has a substituent, a dihydroxyphosphinyl group which may have a substituent, or a hydroxymercaptophosphinyl group which may have a substituent; and
"Base", $X^1$, $X^2$, $R^3$, $R^4$, $R^5$, $R^6$, $Z^1$, and $Z^2$ are as defined above)
or a salt thereof with at least one compound or salt selected from compounds respectively represented by formulae (6C) to (10C):

[Formula 25]

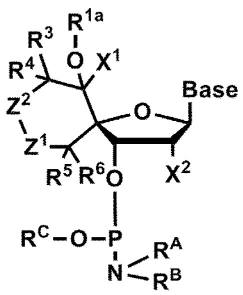

(6C) ,

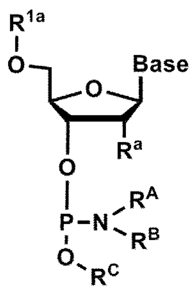

(7C) ,

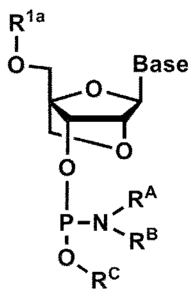

(8C) ,

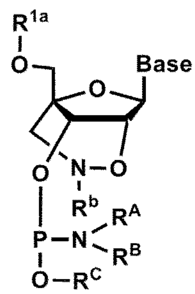

(9C) ,

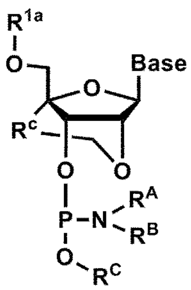

(10C)

(wherein $R^{1a}$ represents a hydrogen atom, an alkyl group which may have a substituent, an alkenyl group which may have a substituent, a cycloalkyl group which may have a substituent, a cycloalkenyl group which may have a substituent, an aryl group which may have a substituent, a protecting group for a hydroxy group, a phosphino group which has a substituent, a dihydroxyphosphinyl group which may have a substituent, or a hydroxymercaptophosphinyl group which may have a substituent;
$R^A$ and $R^B$ are the same as or different from each other and independently represent a hydrogen atom or an alkyl group;
$R^C$ represents a hydrogen atom, an alkyl group, a haloalkyl group, or a cyanoalkyl group; and
"Base", $X^1$, $X^2$, $R^3$, $R^4$, $R^5$, $R^6$, $R^a$, $R^b$, $R^c$, $Z^1$, and $Z^2$ are as defined above) or salts thereof; and/or

(II) a step of reacting a compound represented by formula (6C):

[Formula 26]

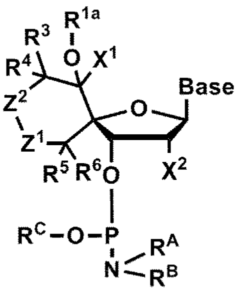

(6C)

(wherein "Base", $X^1$, $X^2$, $R^{1a}$, $R^3$, $R^4$, $R^5$, $R^6$, $R^A$, $R^B$, $R^C$, $Z^1$, and $Z^2$ are as defined above)
or a salt thereof with at least one compound or salt selected from compounds respectively represented by formulae (6B) to (10B):

[Formula 27]

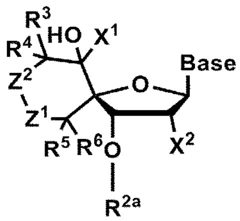

(6B) ,

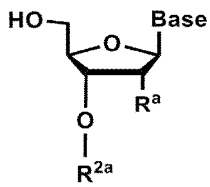

(7B) ,

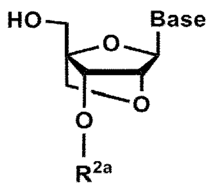

(8B) ,

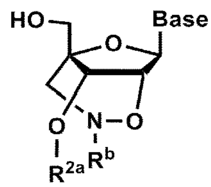

(9B) ,

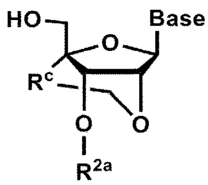

(10B)

(wherein "Base", $X^1$, $X^2$, $R^{2a}$, $R^3$, $R^4$, $R^5$, $R^6$, $R^a$, $R^b$, $R^c$, $Z^1$, and $Z^2$ are as defined above)
or salts thereof; and

(III) a step of oxidizing (particularly oxidizing a phosphorus atom in) the compound obtained in step (I) and/or step (II).

If necessary, the method for producing the oligonucleotide (6) optionally includes a step of purifying the intermediate product and the final product by a conventional method, e.g., concentration, recrystallization, silica gel column chromatography, gel filtration, ethanol precipitation, preparative HPLC.

7. Composition containing oligonucleotide (6)

The composition of the present invention contains the oligonucleotide (6). The composition may contain only one oligonucleotide (6) or may contain two or more oligonucleotides (6).
The composition may be in any form such as solid and liquid. As an example, the composition may be liquid containing the oligonucleotide (6) and a solvent. As the solvent, a known solvent can be used, and preferably a halogenated hydrocarbon-type solvent (e.g., dichloromethane), a nitrile-type solvent (e.g., acetonitrile), an aromatic hydrocarbon-type solvent (e.g., toluene, xylene) or water is used. Among these solvents, water is more preferred, water containing a buffer is more preferred. Examples of the buffer include tris(hydroxymethyl)aminomethane (Tris buffer), tris(hydroxymethyl)aminomethane-hydrochloric acid (Tris-HCl buffer), tris(hydroxymethyl)aminomethane-EDTA (TE buffer), sodium phosphate, 2-morpholinoethanesulfonic acid (MES), N-(2-acetamido)iminodiacetic acid (ADA), piperazine-1,4-bis(2-ethanesulfonic acid), N-(2-acetamido)-2-aminoethanesulfonic acid (ACES), cholamine chloride, N,N-bis(2-hydroxyethyl)-2-aminoethanesulfonic acid (BES), N-tris(hydroxymethyl)methyl-2-aminoethanesulfonic acid (TES), 2-[4-(2-hydroxyethyl)-1-piperazinyl]ethanesulfonic acid (HEPES), acetaminoglycine, tricine, glycinamide, and bicine.

The composition may further contain a salt. The salt includes a metal chloride, and examples of the metal chloride include NaCl, $MgCl_2$, and KCl.

The composition may further contain an additive and a co-solvent. For example, dimethyl sulfoxide (DMSO), glycerol, formamide, bovine serum albumin, ammonium sulfate, polyethylene glycol (PEG), gelatin, and a nonionic surfactant may be contained. Examples of the nonionic surfactant include Tween20 (registered trademark) and Triton X-100 (registered trademark).

As another example, the composition may have, for example, such a form that the oligonucleotide (6) is supported on a solid support. Examples of the solid support include an inorganic material and an organic material which can support a biopolymer. Preferred examples include a glass (e.g., a porous glass (a CPG)), silica gel, and a resin (e.g., a crosslinked non-swellable polystyrene resin (a HPS)). Among these materials, a HPS and a CPG is more preferred.

The composition is generally provided to a user in a form packed in a container.

The composition can be used for the amplification or detection of a target nucleic acid, strand invasion, RNAi and the like as mentioned below. The composition can also be used as a pharmaceutical composition.

## 8. Method for detecting target nucleic acid

The present invention includes a method for detecting a target nucleic acid. Preferably, each step of the method is carried out in vitro. The method includes:

(I) a step of amplifying the target nucleic acid selectively by a nucleic acid amplification method; and
(II) a step of detecting the target nucleic acid that has been amplified in the step (I).

It is preferred that the oligonucleotide to be used for the amplification or the detection comprises the oligonucleotide (6).

In the nucleic acid amplification in step (I), a plurality of primer molecules is generally used. At least a portion of the primer molecules may be the oligonucleotide (6). For example, a forward primer that contains the oligonucleotide (6) and a reverse primer that does not contain the oligonucleotide (6) are used.

In the nucleic acid amplification in step (I), primer molecules and probe molecules may be used. In this case, at least a portion of the primer molecules and the probe molecules may be the oligonucleotide (6). For example, a forward primer that does not contain the oligonucleotide (6), a reverse primer that does not contain the oligonucleotide (6), and a probe that contains the oligonucleotide (6) are used.

The type of the nucleic acid amplification method is not particularly limited, as long as the target nucleic acid can be amplified selectively. Examples of the nucleic acid amplification method include a PCR method (including, e.g., a hot-start PCR method, a multiplex PCR method, a nested PCR method, a RT-PCR method, a real-time PCR method, a digital PCR method, TaqMan (registered trademark) PCR method, a clamp PCR method), a NASBA method (see the description of US Patent No. 5130238), a TMA method (see the description of US Patent No. 5399491), a TRC method (see the description of US Patent Application Publication No. 2001/0053518), a LAMP method (see the description of US Patent No. 6410278), an ICAN method (see the description of US Patent Application Publication No. 2003/073081), a LCR method (see the description of EP Patent Application No. 320328), and a SDA method (see the description of US Patent No. 5455166).

In one embodiment, the nucleic acid amplification method to be employed in step (I) is preferably a clamp PCR method. In the clamp PCR method, at least a primer and a clamp nucleic acid are used. Either one or both of the primer and the clamp nucleic acid may contain the oligonucleotide (6).

In general, a clamp nucleic acid can clamp a non-target nucleic acid (e.g., a wild-type gene) in a test specimen stronger compared with a target nucleic acid (e.g., a mutant gene) in a test specimen. A target nucleic acid can be amplified selectively by binding a clamp nucleic acid to a non-target nucleic acid stronger to inhibit the amplification of the non-target nucleic acid. For example, for the detection of a mutation in a specific gene, the amplification of a nucleic acid is carried out in the presence of a clamp nucleic acid having a nucleotide sequence absolutely complementary to that of a wild-type gene. As a result, the amplification of the wild-type nucleic acid is inhibited, while the mutant nucleic acid is amplified selectively.

More specifically, for example, when a target site in a target nucleic acid is defined as a mutating site in a mutant gene and a nucleotide sequence containing the mutating site (the target site in the target nucleic acid) is defined as "sequence A", a clamp nucleic acid is absolutely complementary to a nucleotide sequence corresponding to the sequence A in a wild-type gene that is a non-target nucleic acid.

The oligonucleotide (6) has sequence selectivity. Therefore, with respect to the oligonucleotide (6), the capability of binding to a nucleotide sequence that is different from the oligonucleotide (6) by at least one nucleotide is weak. The oligonucleotide (6) can bind specifically to an absolutely complementary nucleotide sequence.

The length of the clamp nucleic acid is not particularly limited, and is for example 5- to 30-mer.

The nucleic acid amplification to be employed in step (I) may be a reverse transcription reaction. In the reverse transcription reaction, cDNA is synthesized with a RNA-dependent DNA polymerase by using RNA as a template. The oligonucleotide (6) binds to RNA. Therefore, the reverse transcription reaction from specific RNA in a test specimen can be inhibited by binding the oligonucleotide (6) to the RNA. In this manner, cDNA can be synthesized more specifically from the target RNA. The synthesized cDNA may be further amplified by a PCR method or the like.

The target nucleic acid may be contained in a test specimen. The test specimen is typically a specimen collected from a living body (wherein the test specimen is also referred to as a "biological specimen", hereinafter). In general, a specimen produced by a pre-treatment such as the removal of contaminants from a specimen collected from a living body, the extraction/purification of nucleic acid and pre-amplification is used. More specifically, blood, plasma, serum, pleural effusion, a broncho-alveolar lavage fluid, bone marrow fluid, lymphatic fluid, a bowel lavage fluid, an excised tissue, a nasopharyngeal swab fluid, saliva, a nasal discharge snot, a sputum and the like can be used. A specimen prepared by subjecting the above-mentioned specimen to a pretreatment as mentioned above is also included in the "biological specimen". The method of the present invention can detect a nucleic acid with very high sensitivity. Therefore, for example, a gene mutation associated with an abnormal cell can be detected using an excised tissue in which both of normal cells and abnormal cells are present in a mixed state. The method of the present invention can be used suitably in a case where the amount of the test specimen is very small or a case where the amount of a nucleic acid in the test specimen is very small. The test specimen is preferably a solution containing a target nucleic acid. When it is intended to detect a target nucleic acid in a cell contained in blood or an excised tissue, the cell may be lysed by a known method. In addition to the biological specimen, a test specimen such as an excrement, sewage water, river water, sea water, soil, and a specimen that is produced by subjecting each of the aforementioned specimens to a pretreatment as mentioned above can be used. Examples of the excrement include urine and feces.

The target nucleic acid may be DNA or RNA. DNA may be cDNA synthesized from RNA by a reverse transcription reaction. The target nucleic acid may be a gene or a specific region on genomic DNA (e.g., a promoter region in a gene). The detection method of the present invention can be used for the detection of a mutation, a polymorphism or the like in a target site in a target nucleic acid. The detection method of the present invention can also be used for the determination of an allele. It also becomes possible to detect as to what type of an allele is contained in a test specimen. The detection method of the present invention can also be used for the detection of methylation. When the detection method of the present invention is applied after a bisulphite treatment of a target nucleic acid, the presence or absence of methylated cytosine in the target nucleic acid can be detected.

A gene containing a mutation (hereinafter, also referred to as a "mutant gene") has a difference in nucleotide sequence from a wild-type gene, i.e., a mutation. The difference is caused by at least one mutation selected from the group consisting of substitution, insertion, deletion, inversion, duplication and translocation or a combination thereof.

In general, the difference is often associated with the onset (development) and/or therapeutic sensitivity of a specific disease. The term "onset (development)" includes the actual onset (development) of a disease as well as the risk of onset (development) and the like. The term "therapeutic sensitivity" includes the efficacy of a treatment with a drug or the like as well as the level of an adverse side effect. Examples of the disease include, but are not limited to, cancer, a myelodysplastic syndrome, and an infectious disease. A preferred example of the disease is cancer.

A preferred example of the gene is ABLBCR fusion gene, HER2 gene, EGFR gene, c-KIT gene, KRAS gene, BRAF gene, PIK3CA gene, FLT3 gene, MYC gene, MYCN gene, MET gene, BCL2 gene, or EML4/ALK fusion gene.

In step (II), a nucleic acid amplified by a known method can be detected in accordance with the above-mentioned nucleic acid amplification method. For example, the amplification can be detected qualitatively or quantitatively by detecting a fluorescent generated from a reaction mixture or the turbidity of a reaction mixture.

An example of the method for detecting an amplified target nucleic acid is a nucleotide sequence analysis method. The target nucleic acid can be detected by analyzing the nucleotide sequence of the amplified target nucleic acid using a known sequence analysis device (sequencer).

For example, as the method for detecting the target nucleic acid in a test specimen, the method described in the description of US Patent Application Publication No. 2015/240299 may be employed.

The method of the present invention includes a method for detecting a target nucleic acid in a test specimen, the method comprising a step of reacting a probe containing the oligonucleotide (6) and a label with the test specimen containing the target nucleic acid. Examples of the detection method include in situ hybridization and a microarray.

When in situ hybridization is employed as the detection method, the target nucleic acid in a test specimen (e.g., a cell) can be detected by hybridizing the oligonucleotide (6) that is labeled with a fluorescent dye or the like with the target nucleic acid in the test specimen and then measuring the label.

When a microarray is employed as the detection method, the target nucleic acid can be detected by reacting a microarray onto which a probe containing the oligonucleotide (6) has been immobilized with the test specimen containing the target nucleic acid and then detecting the hybridization between the target nucleic acid and the probe.

As mentioned above, sequence selective detection of target nucleic acids in a test specimen can be achieved using the oligonucleotides (6).

9. Kit for detecting or selectively amplifying a target nucleic acid

A kit for detecting or selectively amplifying a target nucleic acid includes the oligonucleotide (6). The target nucleic acid may be contained in a test specimen.

In the kit, the oligonucleotide (6) can be used as a primer, a probe and/or a clamp nucleic acid for the amplification and/or detection of a target nucleic acid, as mentioned above. The primer, the probe and/or the clamp nucleic acid can be designed appropriately depending on the types of the nucleotide sequence of a target nucleic acid or a non-target nucleic acid.

A kit according to one embodiment includes a primer and/or a probe. In this case, at least a portion of the primer and/or the probe may be the oligonucleotide (6).

Fig. 1A shows a schematic diagram of one example of a kit that includes a container in which a composition containing a primer and a probe is included. The kit 11 includes an outer packaging box 12, a container support which is arranged in the outer packaging box 12 and has a depressed part formed on the surface thereof, a container 13 which is installed in the depressed part and in which a composition containing a primer and a probe is included, and a package insert 14. On the package insert 14, a method for handling the kit 11, the conditions for storage of the kit 11, the validity date of the kit 11 and the like can be written.

Fig. 1B shows a schematic diagram of one example of a kit that includes both of a container in which a composition containing a primer is included a container in which a composition containing a probe is included. The kit 21 includes an outer packaging box 22, a container support which is arranged in the outer packaging box 22 and has a first depressed part and a second depressed part formed apart from each other on the surface thereof along the length direction, a container 23a which is installed in the first depressed part and in which a composition containing a primer is included, a container 23b which is installed in the second depressed part and in which a composition containing a probe is included, and a package insert 24.

A kit according to another embodiment includes a forward primer, a reverse primer, and a probe. At least a portion of them may be the oligonucleotide (6). All three components, i.e., the forward primer, the reverse primer, and the probe, may be contained in a single container (e.g., a kit shown in Fig. 1A), or any two of them may be contained in a single container (e.g., a kit shown in Fig. 1B), or the three components are contained in different containers separately.

Fig. 1C shows a schematic diagram of one example of a kit that includes a container in which a composition containing a forward primer is included, a container in which a composition containing a reverse primer is included, and a container in which a composition containing a probe is included. The kit 31 includes an outer packaging box 32, a container support which is arranged in the outer packaging box 32 and has first to third depressed parts formed apart from each other on the surface thereof along the length direction, a container 33a which is installed in the first depressed part and in which a composition containing the forward primer is included, a container 33b which is installed in the second depressed part and in which a composition containing the reverse primer is included, a container 33c which is installed in the third depressed part and in which a composition containing the probe is included, and a package insert 34.

A kit according to another embodiment includes a clamp nucleic acid and a primer. At least a portion of them may be the oligonucleotide (6). The kit may be a kit for selectively amplifying a target nucleic acid. A composition containing the clamp nucleic acid and a composition containing the primer may be included in a single container (e.g., a kit shown in Fig. 1A), or may be included in different containers separately (e.g., a kit shown in Fig. 1B).

A kit according to still another embodiment includes a clamp nucleic acid, a primer, and a probe. At least a portion of them may be the oligonucleotide (6). All three components, i.e., a composition containing the clamp nucleic acid, a composition containing the primer and a composition containing the probe, may be included in a single container (e.g., a kit shown in Fig. 1A), or any two of them may be included in a single container (e.g., a kit shown in Fig. 1B), or the three components are included in different containers separately (e.g., a kit shown in Fig. 1C).

In the kit, a DNA polymerase, deoxynucleoside triphosphates (dNTPs), a reaction buffer, a salt, a restriction enzyme, and the like may also be contained.

The present invention includes a use of the oligonucleotide (6) for the detection of a target nucleic acid or the selective amplification of a target nucleic acid. The oligonucleotide (6) to be used in the use has the same characteristic properties as, for example, those of the oligonucleotide (6) included in the kit.

10. Pharmaceutical composition (or preparation)

The pharmaceutical composition (or preparation) of the present invention contains the compound (1) or the oligonucleotide (6). An example of the pharmaceutical composition (or preparation) containing the compound (1) is a low-molecular-weight medicine such as azidothymidine (AZT) that is a nucleoside analogue reverse transcriptase inhibitor: NRTI. An example of the pharmaceutical composition (or preparation) containing the oligonucleotide (6) is a nucleic acid medicine comprising a middle-molecular-weight molecule such as an antisense, siRNA (small interfering RNA), an aptamer, a decoy nucleic acid, and a CpG oligonucleotide or a high-molecular-weight molecule.
The pharmaceutical composition may have any dosage form selected from a liquid preparation (e.g., an injection, eye drops, nasal drops, a suspension), a solid preparation (e.g., tables, granules, a powder), a semi-solid preparation (e.g., an ointment, a suppository), and other dosage form known to persons skilled in the art.
Preferred examples of the pharmaceutical composition include preparations for parenteral administration (e.g., a preparation for subcutaneous administration, a preparation for intravenous administration, a preparation of transnasal administration, a preparation for intrathecal administration, a preparation for intraventricular administration, a preparation for intravitreous administration).
Another preferred example of the pharmaceutical composition is a topical preparation.
In general, the pharmaceutical composition further contains a pharmaceutically acceptable carrier or an additive.
The carrier includes a solid carrier and a liquid carrier. Examples of the solid carrier include starch, lactose, calcium sulfate dihydrate, sucrose, talc, gelatin, agar, pectin, gum arabic, magnesium stearate, stearic acid, and atelo collagen.
An example of the liquid carrier is water (including physiological saline).
The additive includes a stabilizing agent, and examples of the stabilizing agent include: a para-hydroxybenzoic acid ester such as methylparaben and propylparaben; an alcohol such as benzyl alcohol; and a phenol compound such as phenol and cresol.
The oligonucleotide (6) has sequence selectivity and is therefore less likely to be decomposed with a nuclease. Accordingly, the pharmaceutical composition (or preparation) containing the compound (1) or the oligonucleotide (6) can capture a target (e.g., a target gene) in vivo with sequence selectivity and can act on the target.

EXAMPLES

Hereinbelow, the present invention will be described in more detail with reference to examples. However, the present invention is not limited by these examples.

[Synthesis Examples of Compound (1)]

Symbols and abbreviated words used in synthesis examples are as follows.

$Ac_2O$: Acetic anhydride
AcOH: Acetic acid
$AgBF_4$: Silver tetrafluoroborate
AgOTf: Silver trifluoromethanesulfonate
AIBN: 2,2'-azobis(isobutyronitrile)
BnBr: Benzyl bromide
BSA: N,O-bis(trimethylsilyl)acetamide
BusSnH: Tributyltin hydride
BzCl: Benzoyl chloride
$CH_2Cl_2$: Dichloromethane
$CH_2$=$CHCH_2MgBr$: Allylmagnesium bromide
$CH_3CN$: Acetonitrile
$(COCl)_2$: Oxalyl chloride
DMAP: 4-dimethylaminopyridine
DMF: N,N-dimethylformamide
DMSO: Dimethyl sulfoxide
DMTrCl: 4,4'-dimethoxytrityl chloride
$Et_3N$: Triethylamine
$Et_2O$: Diethyl ether
EtOH: Ethanol
$HCOONH_4$: Ammonium formate

$H_2SO_4$: Sulfuric acid
$(i\text{-}Pr_2N)_2POC_2H_4CN$: 2-cyanoethyl N,N,N', N'-tetraisopropylphosphorodiamidite
LiOH: Lithium hydroxide
MeI: Methyl iodide
MsCl: Methanesulfonyl chloride
$NaBH_4$: Sodium borohydride
NaH: Sodium hydride
$NaIO_4$: Sodium periodate
NMO: 4-methylmorpholine N-oxide
$OsO_4$: Osmium(VIII) oxide
$Pd(OH)_2$-C: Palladium hydroxide-carbon powder
$Ph_3P^+CH_3Br^-$: Methyltriphenylphosphonium bromide
TBAF: Tetra-n-butylammonium fluoride
TBDPSCl: Tert-butyldiphenylchlorosilane
THF: Tetrahydrofuran
TMSOTf: Trimethylsilyl trifluoromethanesulfonate
TsCl: p-toluenesulfonyl chloride
rt: room temperature
h: hour
min: minute

[Formula 28-1]

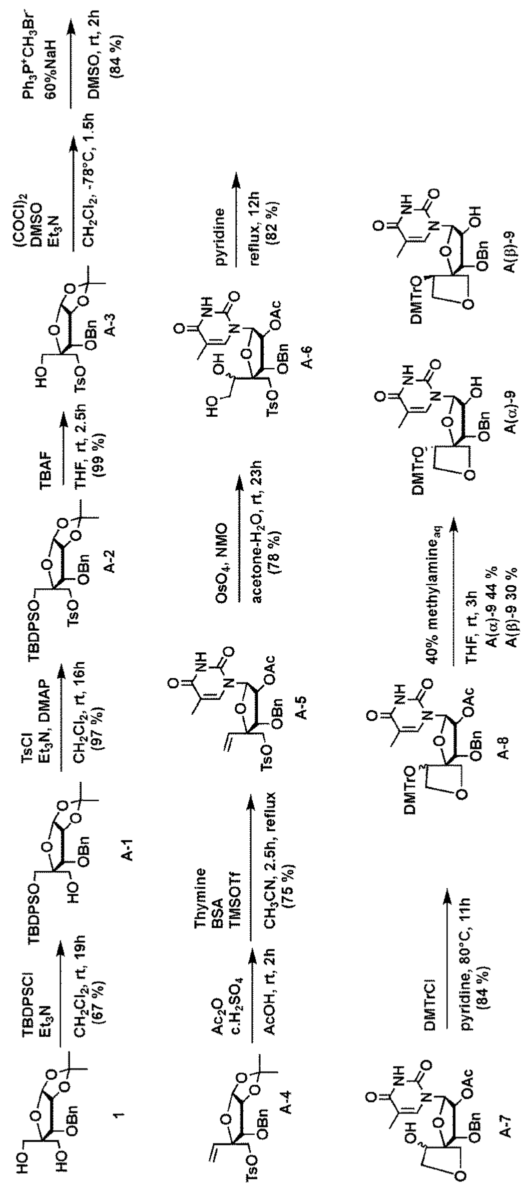


1 A-1 A-2 A-3 A-4 A-5 A-6 A-7 A-8 A(α)-9 A(β)-9

[Formula 28-2]

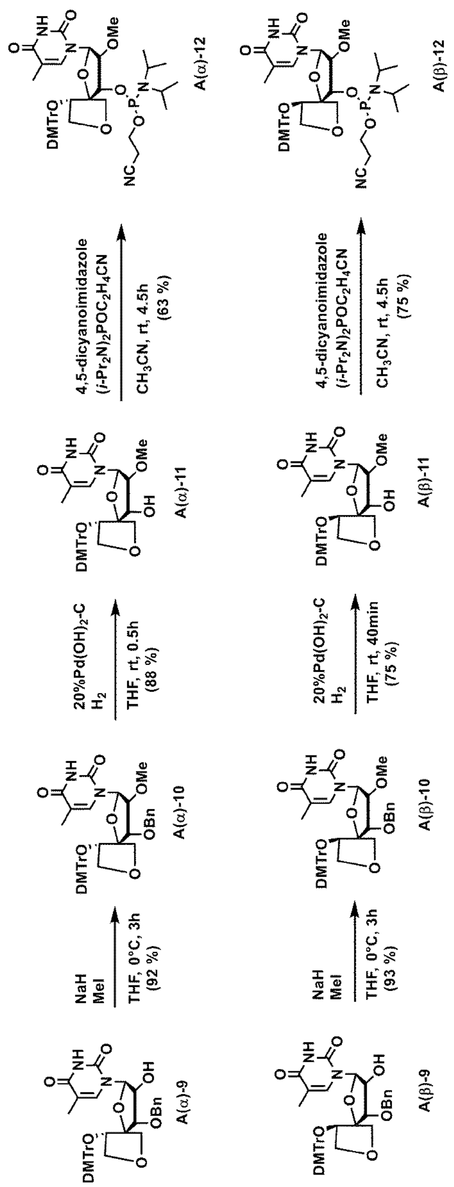


<Synthesis of compound A-1>

Under an argon atmosphere, compound 1 (30.0 g, 96.67 mmol) was dissolved in dichloromethane (300 mL), then triethylamine (4.2 mL, 299.66 mmol), and tert-butyldiphenylchlorosilane (40.0 mL, 154.66 mmol) were sequentially added to the solution in an ice bath, and the mixture was stirred at room temperature for 19 hours. The reaction mixture was cooled and the reaction was quenched with water, and the resultant solution was diluted with dichloromethane and fractionated into an organic layer and an aqueous layer. The aqueous layer was subjected to extraction with dichloromethane, then the organic layer obtained in the first fractionation was combined with an organic layer obtained in the extraction, then the resultant solution was washed with brine, then the washed solution was dried over anhydrous sodium sulfate, and the solvent was concentrated under reduced pressure. A crude product thus obtained was purified by silica gel column chromatography (hexane : ethyl acetate = 4 : 1 to 0 : 1) to obtain compound A-1 (35.4 g, yield: 67%) as a white solid.

$^1$H NMR($CDCl_3$)δ 0.99 (9H, s), 1.37 (3H, s), 1.65 (3H, s), 2.39-2.42 (1H, m), 3.68, 3.78 (2H, ABq, J=11.0 Hz), 3.77-3.88 (2H, m), 4.44 (1H, d, J=5.0 Hz), 4.52, 4.82 (2H, ABq, J=12.0 Hz), 4.70 (1H, dd, J=4.0 Hz, 5.0 Hz), 5.82 (1H, d, J=4.0 Hz), 7.29-7.45 (11H, m), 7.59-7.64 (4H, m).

<Synthesis of compound A-2>

Under an argon atmosphere, compound A-1 (35.4 g, 63.16 mmol) was dissolved in dichloromethane (420 mL),

triethylamine (27.0 mL, 194.25 mmol), p-toluenesulfonyl chloride (15.9 g, 83.88 mmol), and 4-dimethylaminopyridine (0.79 g, 6.45 mmol) were sequentially added to the solution in an ice bath, and the mixture was stirred at room temperature for 16 hours. The reaction mixture was cooled and the reaction was quenched with water, and then, the resultant solution was diluted with dichloromethane and fractionated into an organic layer and an aqueous layer. The obtained organic layer was washed with brine, and the washed solution was dried over anhydrous sodium sulfate, and the solvent was concentrated under reduced pressure. A crude product thus obtained was purified by silica gel column chromatography (hexane : ethyl acetate = 10 : 1) to obtain compound A-2 (44.1 g, 97%) as a white solid.
$^1$H NMR($CDCl_3$)$\delta$ 0.96 (9H, s), 1.29 (3H, s), 1.33 (3H, s), 2.38 (3H, s), 3.55, 3.71 (2H, ABq, J=10.5 Hz), 4.33, 4.49 (2H, ABq, J=10.5 Hz), 4.33 (1H, d, J=4.5 Hz), 4.51, 4.70 (2H, ABq, J=12.0 Hz), 4.57 (1H, dd, J=3.5 Hz, 4.5 Hz), 5.70 (1H, d, J=3.5 Hz), 7.20-7.22 (2H, m), 7.27-7.46 (11H, m), 7.55-7.59 (4H, m), 7.70-7.72 (2H, m).

<Synthesis of compound A-3>

Compound A-2 (77.54 g, 110.31 mmol) was dissolved in tetrahydrofuran (800 mL), butylammonium-n-fluoride (1M in tetrahydrofuran, 126.0 mL, 126.0 mmol) was sequentially added to the solution, and the mixture was stirred at room temperature for 2.5 hours. The solvent of the reaction mixture thus obtained was concentrated under reduced pressure and the residue was purified by silica gel column chromatography (hexane : ethyl acetate = 3 : 2 to 0 : 1) to obtain compound A-3 (50.91 g, 99%) as a white solid.
$^1$H NMR($CDCl_3$)$\delta$ 1.27 (3H, s), 1.33 (3H, s), 1.76-1.79 (1H, m), 2.43 (3H, s), 3.38-3.43 (1H, m), 3.78 (1H, dd, J=3.5 Hz, 12.0 Hz), 4.22 (1H, d, J=5.0 Hz), 4.28 (1H, d, J=11.0 Hz), 4.52-4.58 (3H, m), 4.72 (1H, d, J=12.0 Hz), 5.69 (1H, d, J=4.0), 7.30-7.39 (7H, m), 7.78-7.81 (2H, m).

<Synthesis of compound A-4>

Under an argon atmosphere, oxalyl chloride (9.7 mL, 113.0 mmol) was dissolved in dichloromethane (135 mL) and cooled with dry ice-acetone. Dimethyl sulfoxide (12.8 mL, 180.8 mmol) and a dichloromethane solution (135 mL) were added dropwise slowly to the reaction mixture and the mixture was stirred for 20 minutes, and a solution of compound A-3 (20.4 g, 43.85 mmol) in a dichloromethane solution (350 mL) was added dropwise slowly to the reaction mixture and the mixture was stirred for 15 minutes. Subsequently, triethylamine (50.2 mL, 361.6 mmol) was added dropwise slowly to the reaction mixture, and the mixture was warmed to room temperature and stirred for 1 hour. The reaction mixture was cooled and the reaction was quenched with water, and the resultant solution was fractionated into an organic layer and an aqueous layer. The aqueous layer was subjected to extraction with dichloromethane, then the organic layer obtained in the first fractionation was combined with an organic layer obtained in the extraction, then the resultant solution was washed with brine, then the washed solution was dried over anhydrous sodium sulfate, and the solvent was concentrated under reduced pressure to obtain an intermediate. Subsequently, under an argon atmosphere, sodium hydride (60% in mineral oil, 4.5 g, 113.0 mmol) was dissolved in dimethyl sulfoxide (45 mL), then the mixture was stirred at 80°C for 50 minutes and cooled to room temperature, methyltriphenylphosphonium bromide (40.3 g, 113.0 mmol) in a dimethyl sulfoxide solution (150 mL) was added to the solution, and the mixture was stirred for 20 minutes. Subsequently, a solution of the intermediate obtained above in the dimethyl sulfoxide solution (105 mL) was added to the reaction mixture, and the mixture was stirred for 1.5 hours. The reaction mixture was cooled and the reaction was quenched with water, and then, diluted with ethyl acetate to be fractionated into an organic layer and an aqueous layer. The aqueous layer was subjected to extraction with ethyl acetate, then the organic layer obtained in the first fractionation was combined with an organic layer obtained in the extraction, then the resultant solution was washed with brine, then the washed solution was dried over anhydrous sodium sulfate, and the solvent was concentrated under reduced pressure. A crude product thus obtained was purified by silica gel column chromatography (hexane : ethyl acetate = 6: 1 to 1 : 1) to obtain compound A-4 (17.6 g, 84%) as a light-yellow oily substance.
$^1$H NMR($CDCl_3$)$\delta$ 1.28 (3H, s), 1.32 (3H, s), 2.42 (3H, s), 3.80 (1H, d, J=5.0 Hz), 4.36 (1H, d, J=10.5 Hz), 4.51-4.57 (3H, m), 4.72 (1H, d, J=11.5 Hz), 5.13 (1H, dd, J=1.0 Hz, 11.0 Hz), 5.30 (1H, dd, J=1.0 Hz, 17.0 Hz), 5.76 (1H, d, J=4.0 Hz), 5.82 (1H, dd, J=11.0 Hz, 17.0 Hz), 7.29-7.39 (7H, m), 7.77-7.81 (2H, m).

<Synthesis of compound A-5>

Under an argon atmosphere, compound A-4 (2.45 g, 5.31 mmol) was dissolved in acetic acid (37 mL), then acetic anhydride (27.0 mL, 194.25 mmol) and sulfuric acid (25 mg, 0.25 mmol) were sequentially added to the solution at room temperature, and the mixture was stirred for 2 hours. The reaction mixture was cooled, neutralized with saturated aqueous sodium bicarbonate, diluted with water and ethyl acetate, and fractionated into an organic layer and an aqueous layer. The aqueous layer was subjected to extraction with ethyl acetate, then the organic layer obtained in the first fractionation was combined with an organic layer obtained in the extraction, then the resultant solution was washed

sequentially with saturated aqueous sodium bicarbonate solution and brine, then the washed solution was dried over anhydrous sodium sulfate, and the solvent was concentrated under reduced pressure to obtain an intermediate. The intermediate thus obtained was azeotropically dried with acetonitrile and the resultant product was dissolved in acetonitrile (53 mL) under a nitrogen atmosphere. Thymine (1.07 g, 8.50 mmol) and N,O-bis(trimethylsilyl)acetamide (5.3 mL, 21.24 mmol) were sequentially added to the solution and the mixture was stirred at 50°C for 15 minutes. Subsequently, trimethylsilyl trifluoromethanesulfonate (1.2 mL, 6.90 mmol) was added to the solution and the mixture was heated under reflux for 2 hours. The reaction mixture was cooled in an ice bath and the reaction was quenched with saturated aqueous sodium bicarbonate, then the resultant solution was diluted with ethyl acetate, and the diluted solution was fractionated to obtain an organic layer. The obtained organic layer was washed with brine, then the washed solution was dried over anhydrous sodium, and the solvent was concentrated under reduced pressure. A crude product thus obtained was purified by silica gel column chromatography (hexane : ethyl acetate = 3 : 2 to 1 : 3) to obtain compound A-5 (2.28 g, 75%) as a white foam-like solid.
$^1$H NMR($CDCl_3$)$\delta$ 1.91 (3H, d, J=1.0 Hz), 2.07 (3H, s), 2.43 (3H, s), 4.20 (2H, ABq, J=10.0 Hz), 4.40 (1H, d, J=6.0 Hz), 4.54 (2H, ABq, J=11.0 Hz), 5.26-5.35 (2H, m), 5.41 (1H, dd, J=4.5 Hz, 6.0 Hz), 5.72 (1H, d, J=4.5 Hz), 5.87 (1H, dd, J=11.0 Hz, 17.5 Hz), 6.97 (1H, d, J=1.0 Hz), 7.24-7.38 (7H, m), 7.74-7.78 (2H, n), 8.52 (1H, s).

<Synthesis of compound A-6>

Compound A-5 (8.19 g, 14.43 mmol) was dissolved in acetone (140 mL), water (20 mL), 4-methylmorpholine N-oxide (5.25 g, 44.82 mmol), and osmium(VIII) oxide (76 mg, 0.30 mmol) were sequentially added to the solution at room temperature, and the mixture was stirred for 22 hours. Subsequently, 4-methylmorpholine N-oxide (0.50 g, 4.27 mmol) was added to the solution and the mixture was stirred for another hour. The reaction was quenched with a saturated potassium hydrogen sulfate solution and water, and then, the precipitate was filtered off and the obtained filtrate was collected. The collected filtrate was diluted with ethyl acetate and saturated aqueous sodium bicarbonate and resultant solution was fractionated into an organic layer and an aqueous layer. The obtained organic layer was washed with brine, then the washed solution was dried over anhydrous sodium sulfate, and the solvent was concentrated under reduced pressure. A crude product thus obtained was purified by silica gel column chromatography (hexane : ethyl acetate = 1 : 2 to 0 : 1) to obtain compound A-6 (6.80 g, 78%) as a white foam-like solid.
HRMS(MALDI): calcd for C28H32N2NaO11S [M+Na$^+$] 627.1619, found 627.1611.

<Synthesis of compound A-7>

Under a nitrogen atmosphere, compound A-6 (0.32 g, 5.31 mmol) was dissolved in pyridine (10 mL) and heated under reflux for 12 hours. The reaction mixture was cooled and the reaction was quenched with water, then the resultant solution was diluted with ethyl acetate and brine, and the diluted solution was fractionated to obtain an organic layer. The obtained organic layer was washed with brine, then the washed solution was dried over anhydrous sodium, and the solvent was concentrated under reduced pressure. A crude product thus obtained was purified by silica gel column chromatography (hexane : ethyl acetate = 1 : 1) to obtain compound A-7 (0.18 g, 82%) as a white foam-like solid.
HRMS(MALDI): calcd for C21H24N2NaO8 [M+Na$^+$] 455.1425, found 455.1420.

<Synthesis of compound A-8>

Under an argon atmosphere, compound A-7 (1.94 g, 4.49 mmol) was dissolved in pyridine (20 mL), then 4,4'-dimethoxytrityl chloride (3.05 g, 8.99 mmol) was added to the solution at room temperature, and then the mixture was stirred at 80°C for 11 hours. Methanol was added to the solution and the reaction was quenched, and the resultant solution was diluted with ethyl acetate and water and fractionated into an organic layer and an aqueous layer. The aqueous layer was subjected to extraction with ethyl acetate. The organic layer obtained in the first fractionation was combined with an organic layer obtained in the extraction, then the resultant solution was washed with brine, then the washed solution was dried over anhydrous sodium sulfate, and the solvent was concentrated under reduced pressure. A crude product thus obtained was purified by silica gel column chromatography (hexane : ethyl acetate = 3 : 2 to 0 : 1) to obtain compound A-8 (2.76 g, 84%) as a yellow foam-like solid.
HRMS(MALDI): calcd for C42H42N2NaO10 [M+Na$^+$] 757.2732, found 757.2733.

<Synthesis of compound A($\alpha$)-9 and A($\beta$)-9>

Compound A-8 (2.74 g, 3.73 mmol) was dissolved in tetrahydrofuran (42 mL), then a 40% aqueous methylamine solution (4.8 mL, 57.15 mmol) was added to the solution in an ice bath, then the mixture was stirred for 30 minutes, and the resultant solution was stirred at room temperature for 3 hours. The resultant reaction mixture was concentrated

under reduced pressure, and then the residue was diluted with ethyl acetate and water and fractionated into an organic layer and an aqueous layer. The aqueous layer was subjected to extraction with ethyl acetate. The organic layer obtained in the first fractionation was combined with an organic layer obtained in the extraction, then the resultant solution was washed with brine, then the washed solution was dried over anhydrous sodium sulfate, and the solvent was concentrated under reduced pressure. A crude product thus obtained was purified by silica gel column chromatography (chloroform : methanol = 1 : 0 to 70 : 1) to obtain compound A(α)-9 (1.14 g, 44 %) and compound A(β)-9 (0.79 g, 30 %) as white foam-like solids, respectively.

Compound A(α)-9

$^1$H NMR($CDCl_3$)δ 1.80 (3H, d, J=1.0 Hz), 3.13 (1H, d, J=6.5 Hz), 3.13-3.17 (1H, m), 3.29-3.32 (1H, m), 3.78 (3H, s), 3.78 (3H, s), 3.84, 4.21 (2H, ABq, J=10.0 Hz), 3.94 (1H, t, J=5.5 Hz), 4.05-4.09 (1H, m), 4.79, 4.88 (2H, ABq, J=11.5 Hz), 4.78 (1H, d, J=6.0 Hz), 5.62 (1H, d, J=6.0 Hz), 6.80-6.83 (4H, m), 6.85 (1H, d, J=1.0 Hz), 7.21-7.45 (14H, m), 8.43 (1H, s).

Compound A(β)-9

$^1$H NMR($CDCl_3$)δ 1.45 (3H, s), 3.07-3.10 (1H, m), 3.50 (1H, t, J=9.0 Hz), 3.76 (1H, d, J=5.5 Hz), 3.77 (6H, s), 3.83, 3.92 (2H, ABq, J=11.0 Hz), 4.00-4.04 (1H, m), 4.22-4.26 (1H, m), 4.30, 4.50 (2H, ABq, J=11.5 Hz), 5.87 (1H, d, J=3.5 Hz), 6.82-6.85 (4H, m), 7.15-7.52 (14H, m), 7.81 (1H, s), 8.97 (1H, brs).

<Synthesis of compound A(α)-10>

Under a nitrogen atmosphere, compound A(α)-9 (0.38 g, 0.54 mmol) was dissolved in tetrahydrofuran (6 mL), sodium hydride (60% in mineral oil, 0.066 g, 1.64 mmol) and methyl iodide (0.27 mL, 4.38 mmol) were sequentially added to the solution in an ice bath, and the mixture was stirred for 3 hours. The reaction mixture was cooled and the reaction was quenched with water, and the resultant solution was diluted with ethyl acetate and fractionated into an organic layer and an aqueous layer. The aqueous layer was subjected to extraction with ethyl acetate. The organic layer obtained in the first fractionation was combined with an organic layer obtained in the extraction, then the resultant solution was washed with brine, then the washed solution was dried over anhydrous sodium sulfate, and the solvent was concentrated under reduced pressure. A crude product thus obtained was purified by silica gel column chromatography (hexane : ethyl acetate = 1 : 1 to 2 : 3) to obtain compound A(α)-10 (0.35 g, 92%) as a white foam-like solid.
$^1$H NMR($CDCl_3$)δ 1.68 (3H, s), 3.07-3.11 (1H, m), 3.27-3.31 (1H, m), 3.42 (3H, 1H, d, J=1.0 Hz), 3.78 (6H, s), 3.79, 4.30 (2H, ABq, J=10.5 Hz), 3.80-3.82 (1H, m), 4.02 (1H, t, J=6.0 Hz), 4.70 (1H, d, J=5.0 Hz), 4.76, 4.86 (2H, ABq, J=11.5 Hz), 5.82 (1H, d, J=3.5 Hz), 6.77-6.80 (4H, m), 6.90 (1H, s), 7.20-7.45 (14H, m), 8.28 (1H, s).

<Synthesis of compound A(β)-10>

Under a nitrogen atmosphere, compound A(β)-9 (0.79 g, 1.14 mmol) was dissolved in tetrahydrofuran (12 mL), sodium hydride (60% in mineral oil, 0.137 g, 3.42 mmol) and methyl iodide (0.57 mL, 9.12 mmol) were sequentially added to the solution in an ice bath, and the mixture was stirred for 3 hours. The reaction mixture was cooled and the reaction was quenched with water, then the resultant solution was diluted with ethyl acetate, and the diluted solution was fractionated to obtain an organic layer. The obtained organic layer was washed with brine, then the washed solution was dried over anhydrous sodium sulfate, and the solvent was concentrated under reduced pressure. A crude product thus obtained was purified by silica gel column chromatography (hexane : ethyl acetate = 1 : 1 to 1 : 2) to obtain compound A(β)-10 (0.75 g, 93%) as a white foam-like solid.
$^1$H NMR($CDCl_3$)δ 1.21 (3H, d, J=1.0 Hz), 2.96-3.00 (1H, m), 3.47 (1H, dd, J=8.5 Hz, 9.5 Hz), 3.51 (3H, s), 3.69 (1H, dd, J=1.0 Hz, 5.5 Hz), 3.76 (3H, s), 3.77 (3H, s), 3.80, 4.01 (2H, ABq, J=11.0 Hz), 3.85 (1H, d, J=5.5 Hz), 4.15-4.17 (1H, m), 4.23, 4.43 (2H, ABq, J=11.5 Hz), 5.97 (1H, d, J=1.0 Hz), 6.80-6.84 (4H, m), 7.15-7.18 (2H, m), 7.24-7.33 (4H, m), 7.40-7.43 (2H, m), 7.99 (1H, d, J=1.0 Hz), 8.50 (1H, brs).

<Synthesis of compound A(α)-11>

Compound A(α)-10 (0.34 g, 0.48 mmol) was dissolved in tetrahydrofuran (14 mL), 20% palladium hydroxide-carbon powder (34 mg) was added to the solution at room temperature, and under a hydrogen atmosphere at atmospheric pressure, the mixture was stirred for 0.5 hours. The reaction mixture was filtered and the solvent was concentrated under reduced pressure, and a crude product thus obtained was purified by silica gel column chromatography (hexane : ethyl acetate = 2 : 3 to 1 : 3) to obtain compound A(α)-11 (0.26 g, 88%) as a white foam-like solid.

$^1H$ NMR($CDCl_3$)δ 1.74 (3H, d, J=1.5 Hz), 2.81 (1H, d, J=5.5 Hz), 3.16 (1H, dd, J=5.0 Hz, 10.0 Hz), 3.33 (1H, dd, J=5.0 Hz, 10.0 Hz), 3.51 (3H, s), 3.74-3.76 (1H, m), 3.78, 4.22 (2H, ABq, J=11.0 Hz), 3.79 (6H, s), 4.05 (1H, t, J=5.0 Hz), 4.85 (1H, t, J=6.0 Hz), 5.84 (1H, d, J=4.0 Hz), 6.82-6.85 (5H, m), 7.22-7.30 (3H, m), 7.31-7.39 (4H, m), 7.46-7.49 (2H, m), 8.31 (1H, s).

<Synthesis of compound A(β)-11>

Compound A(β)-10 (0.72 g, 1.02 mmol) was dissolved in tetrahydrofuran (30 mL), 20% palladium hydroxide-carbon powder (72 mg) was added to the solution at room temperature, and under a hydrogen atmosphere at atmospheric pressure, the mixture was stirred for 40 minutes. The reaction mixture was filtered and the solvent was concentrated under reduced pressure, and a crude product thus obtained was purified by silica gel column chromatography (hexane : ethyl acetate = 2 : 3 to 1 : 4) to obtain compound A(β)-11 (0.47 g, 75%) as a white foam-like solid.
$^1H$ NMR($CDCl_3$)δ 1.29 (3H, d, J=1.0 Hz), 2.46 (1H, d, J=8.0 Hz), 3.08 (1H, dd, J=7.0 Hz, 8.5 Hz), 3.48-3.52 (1H, m), 3.56 (3H, s), 3.69 (1H, dd, J=2.5 Hz, 5.5 Hz), 3.74, 3.86 (2H, ABq, J=10.5 Hz), 3.80 (3H, s), 3.81 (3H, s), 3.87-3.90 (1H, m), 4.19 (1H, dd, J=7.5 Hz, 9.5 Hz), 6.00 (1H, d, J=2.5 Hz), 6.82-6.87 (4H, m), 7.25-7.35 (7H, m), 7.42-7.45 (2H, m), 7.90 (1H, d, J=1.5 Hz), 8.32(1H, brs).

<Synthesis of compound A(α)-12>

Under a nitrogen atmosphere, compound A(α)-11 (0.30 g, 0.48 mmol) was dissolved in acetonitrile (3.6 mL), then 4,5-dicyanoimidazole (63 mg, 0.53 mmol) and 2-cyanoethyl N,N,N',N'-tetraisopropylphosphorodiamidite (0.19 mL, 0.58 mmol) were sequentially added to the solution in an ice bath, and the mixture was stirred at room temperature for 3 hours. Subsequently, 4,5-dicyanoimidazole (30 mg, 0.25 mmol), 2-cyanoethyl N,N,N',N'-tetraisopropylphosphorodiamidite (95 μL, 0.29 mmol) were added to the solution at room temperature and the mixture was stirred for 1.5 hours. Water was added to the solution and the reaction was quenched, then the resultant solution was diluted with ethyl acetate, and the diluted solution was fractionated to obtain an organic layer. The organic layer was washed sequentially with water and brine, then the washed solution was dried over anhydrous sodium sulfate, and the solvent was concentrated under reduced pressure. A crude product thus obtained was purified by silica gel column chromatography (hexane : ethyl acetate = 2 : 1) to obtain compound A(α)-12 (0.25 g, 63%) as a white foam-like solid.

$^{31}P$ NMR($CDCl_3$)δ 150.1, 150.9.
HRMS(MALDI): calcd for C43H53N4NaO10P [M+$Na^+$] 839.3392, found 839.3379.

<Synthesis of compound A(β)-12>

Under a nitrogen atmosphere, compound A(β)-11 (0.33 g, 0.53 mmol) was dissolved in acetonitrile (4.0 mL), then 4,5-dicyanoimidazole (88 mg, 0.75 mmol) and 2-cyanoethyl N,N,N',N'-tetraisopropylphosphorodiamidite (0.26 mL, 0.80 mmol) were sequentially added to the solution in an ice bath, and the mixture was stirred at room temperature for 4.5 hours. Water was added to the solution and the reaction was quenched, then the resultant solution was diluted with ethyl acetate, and the diluted solution was fractionated to obtain an organic layer. The organic layer was washed sequentially with water and brine, then the washed solution was dried over anhydrous sodium sulfate, and the solvent was concentrated under reduced pressure. A crude product thus obtained was purified by silica gel column chromatography (hexane : ethyl acetate = 2 : 1) to obtain compound A(β)-12 (0.32 g, 75%) as a white foam-like solid. $^{31}P$ NMR($CDCl_3$)δ 150.1, 151.1.
HRMS(MALDI): calcd for C43H53N4NaO10P [M+$Na^+$] 839.3392, found 839.3396.

[Formula 29]

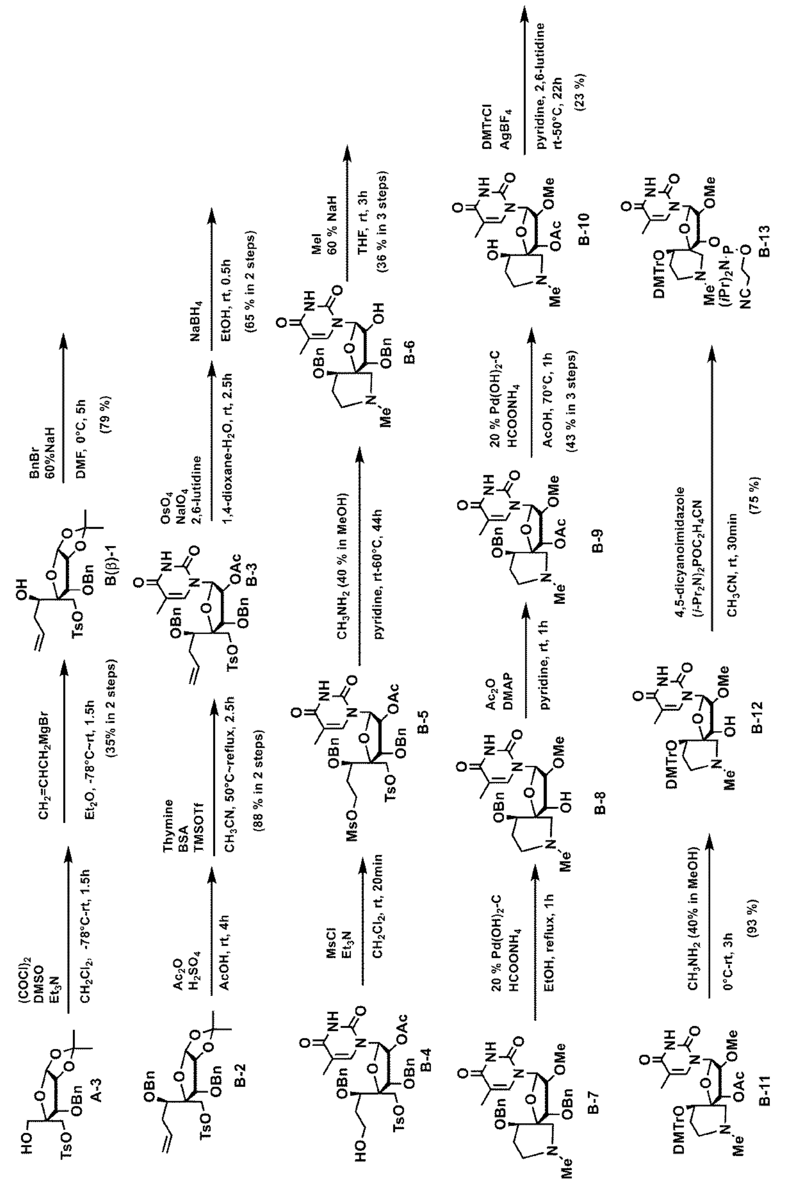


<Synthesis of compound B(β)-1>

Under an argon atmosphere, oxalyl chloride (14.2 mL, 166.0 mmol) was dissolved in dichloromethane (230 mL) and cooled with dry ice-acetone. Dimethyl sulfoxide (20.0 mL, 276.6 mmol) and a dichloromethane solution (230 mL) were added dropwise slowly to the reaction mixture and the mixture was stirred for 30 minutes, and a solution of compound A-3 (51.4 g, 110.7 mmol) in a dichloromethane solution (430 mL) was added dropwise slowly to the reaction mixture and the mixture was stirred for 25 minutes. Subsequently, triethylamine (68.0 mL, 486.9 mmol) was added dropwise slowly to the reaction mixture, and the resultant solution was warmed to room temperature and stirred for 40 minutes. The reaction mixture was cooled and the reaction was quenched with water, and the resultant solution was fractionated into an organic layer and an aqueous layer. The aqueous layer was subjected to extraction with dichloromethane, then the organic layer obtained in the first fractionation was combined with an organic layer obtained in the extraction, then the resultant solution was washed sequentially with 1N hydrochloric acid, a saturated aqueous sodium bicarbonate solution, and brine, then the washed solution was dried over anhydrous sodium sulfate, and the solvent was concentrated under reduced pressure to obtain an intermediate. Subsequently, under an argon atmosphere, the intermediate thus obtained was dissolved in diethyl ether (540 mL) and cooled with dry ice-acetone. Allylmagnesium bromide (0.7M in diethyl ether, 200.0 mL, 143.9 mmol) was added dropwise slowly to the reaction mixture, and the mixture was stirred at room temperature for 1.5 hours. The reaction mixture was cooled and the reaction was quenched

with 1N hydrochloric acid, and the resultant solution was diluted with ethyl acetate and fractionated into an organic layer and an aqueous layer. The obtained organic layer was washed with brine, then the washed solution was dried over anhydrous sodium sulfate, and the solvent was concentrated under reduced pressure. A crude product thus obtained was purified by silica gel column chromatography (hexane : ethyl acetate = 3: 1 to 2 : 1) to obtain compound B(α)-1 (21.5 g, 38%) as a white solid and compound B(β)-1 (19.6 g, 35 %) as a light-yellow oily substance.

Compound B(α)-1

$^1$H NMR($CDCl_3$)δ 1.27 (3H, s), 1.32 (3H, s), 1.88-1.99, 2.13-2.22 (2H, m), 2.43 (3H, s), 3.97 (1H, dd, J=3.0, 10.5), 4.25 (1H, d, J=4.5 Hz), 4.34, 4.60 (2H, ABq, J=11.0), 4.48, 4.71 (2H, ABq, J=11.5), 4.56-4.59 (1H, dd, m), 4.98-5.06 (2H, m), 5.70-5.84 (1H, m), 5.73 (1H, d, J=3.5 Hz), 7.31-7.40 (7H, m), 7.78-7.83 (2H, m).

Compound B(β)-1

$^1$H NMR($CDCl_3$)δ 1.28 (3H, s), 1.36 (3H, s), 1.90 (1H, d, J=6.5), 1.98-2.09, 2.29-2.35 (2H, m), 2.43 (3H, s), 3.77-3.85 (1H, m), 4.24 (1H, d, J=5.0 Hz), 4.30, 4.59 (2H, ABq, J=10.0), 4.52 (1H, dd, J=3.5, 5.0), 4.60, 4.72 (2H, ABq, J=12.0), 5.06-5.14 (2H, m), 5.64 (1H, d, J=3.5 Hz), 5.75-5.89 (1H, m), 7.26-7.39 (7H, m), 7.77 (2H, d, J=8.5).

<Synthesis of compound B-2>

Under an argon atmosphere, compound B(β)-1 (300 mg, 0.61 mmol) was dissolved in N,N-dimethylformamide (6 mL), then sodium hydride (60% in mineral oil, 32 mg, 0.79 mmol) was added to the solution in an ice bath, and the mixture was stirred for 30 minutes. Subsequently, benzyl bromide (94 μL, 0.79 mmol) was added to the solution in an ice bath, and the mixture was stirred for 4.5 hours. The reaction mixture was cooled and the reaction was quenched with water, then the resultant solution was diluted with ethyl acetate, and the diluted solution was fractionated to obtain an organic layer. The aqueous layer was subjected to extraction with ethyl acetate. The organic layer obtained in the first fractionation was combined with an organic layer obtained in the extraction, then the resultant solution was washed with brine, then the washed solution was dried over anhydrous sodium sulfate, and the solvent was concentrated under reduced pressure. A crude product thus obtained was purified by silica gel column chromatography (hexane : ethyl acetate = 4: 1 to 1 : 1) to obtain compound B-2 (288 mg, 79%) as a light-yellow oily substance.
$^1$H NMR($CDCl_3$)δ 1.27 (3H, s), 1.30 (3H, s), 2.15-2.25, 2.28-2.36 (2H, m), 2.39 (3H, s), 3.85 (1H, dd, J=4.0, 7.5), 4.10 (1H, d, J=5.0), 4.35, 4.63 (2H, ABq, J=10.5), 4.38, 4.53 (2H, ABq, J=10.0), 4.42 (1H, dd, J=3.5, 5.0), 4.46, 4.60 (2H, ABq, J=12.5), 5.00-5.10 (2H, m), 5.60 (1H, d, J=3.5), 5.82-5.96 (1H, m), 7.19-7.32 (12H, m), 7.79-7.82 (2H, m).

<Synthesis of compound B-3>

Under an argon atmosphere, compound B-2 (15.21 g, 25.57 mmol) was dissolved in acetic acid (122 mL), then acetic anhydride (19.3 mL, 204.6 mmol) and sulfuric acid (74 mg, 0.75 mmol) were sequentially added to the solution at room temperature, and the mixture was stirred for 4 hours. The reaction mixture was cooled, neutralized with saturated aqueous sodium bicarbonate, diluted with water and ethyl acetate, and fractionated into an organic layer and an aqueous layer. The aqueous layer was subjected to extraction with ethyl acetate, then the organic layer obtained in the first fractionation was combined with an organic layer obtained in the extraction, then the resultant solution was washed sequentially with saturated aqueous sodium bicarbonate solution and brine, then the washed solution was dried over anhydrous sodium sulfate, and the solvent was concentrated under reduced pressure to obtain an intermediate. The intermediate thus obtained was azeotropically dried with acetonitrile and the resultant product was dissolved in acetonitrile (250 mL) under a nitrogen atmosphere. Thymine (5.48 g, 43.47 mmol) and N,O-bis(trimethylsilyl)acetamide (31.6 mL, 127.9 mmol) were sequentially added to the solution and the mixture was stirred at 50°C for 30 minutes. Subsequently, trimethylsilyl trifluoromethanesulfonate (6.2 mL, 35.80 mmol) was added to the solution and the mixture was heated under reflux for 2 hours. The reaction mixture was cooled in an ice bath and the reaction was quenched with saturated aqueous sodium bicarbonate, then the resultant solution was diluted with ethyl acetate, and the diluted solution was fractionated to obtain an organic layer. The obtained organic layer was washed with brine, then the washed solution was dried over anhydrous sodium, and the solvent was concentrated under reduced pressure. A crude product thus obtained was purified by silica gel column chromatography (hexane : ethyl acetate = 2: 1 to 1 : 2) to obtain compound B-3 (16.35 g, 88%) as a white foam-like solid.
$^1$H NMR($CDCl_3$)δ 1.56 (3H, d, J=1.0), 1.94 (3H, s), 2.29-2.50 (2H, m),2.37 (3H, s), 3.87, 3.89 (1H, dd, J=3.5, 3.5), 4.00, 4.28 (2H, ABq, J=9.5), 4.35 (1H, d, J=5.5), 4.42, 4.58 (2H, ABq, J=11.0), 4.53, 4.71 (2H, ABq, J=10.0), 5.06-5.15 (2H, m), 5.29, 5.31 (1H, dd, J=6.0, 6.0), 5.82-5.96 (1H, m), 6.06 (1H, d, J=7.0), 7.19-7.41 (13H, m), 7.68 (2H, d, J=8.5), 8.14 (1H, s).

<Synthesis of compound B-4>

Compound B-3 (0.99 g, 1.40 mmol) was dissolved in 1,4-dioxane (18 mL) and water (6 mL), then 2,6-lutidine (0.25 mL, 2.10 mmol), osmium(VIII) oxide (14 mg, 0.056 mmol), and sodium periodate (0.90 g, 4.20 mmol) were sequentially added to the solution at room temperature, and the mixture was stirred for 2.5 hours. The reaction mixture was diluted with water and dichloromethane and fractionated into an organic layer and aqueous layer. The obtained organic layer was washed with brine, then the washed solution was dried over anhydrous sodium sulfate, and the solvent was concentrated under reduced pressure to obtain an intermediate. Subsequently, the intermediate thus obtained was dissolved in ethanol (17 mL), then sodium borohydride (74 mg, 1.96 mmol) was added to the solution in an ice bath, and the mixture was stirred at room temperature for 30 minutes. The reaction was quenched with water, then the resultant solution was diluted with ethyl acetate and brine, and the diluted solution was fractionated to obtain an organic layer. The aqueous layer was subjected to extraction with ethyl acetate, then the organic layer obtained in the first fractionation was combined with an organic layer obtained in the extraction, then the resultant solution was washed sequentially with 1N hydrochloric acid and brine, then the washed solution was dried over anhydrous sodium, and the solvent was concentrated under reduced pressure. A crude product thus obtained was purified by silica gel column chromatography (hexane : ethyl acetate = 3 : 2 to 1 : 4) to obtain compound B-4 (0.64 g, 65%) as a white foam-like solid.
$^1$H NMR($CDCl_3$)$\delta$ 1.64 (3H, s), 1.69-1.75 (1H, m), 1.86-1.96 (1H, m), 2.01 (3H, s), 2.40 (3H, s), 3.71-3.78 (2H, m), 4.07-4.13 (1H, m), 4.11, 4.31 (2H, ABq, J=9.5), 4.41 (1H, d, J=6.0), 4.45, 4.55 (2H, ABq, J=11.0), 4.65, 4.70 (2H, ABq, J=10.5), 5.38 (1H, t, J=6.0), 6.06 (1H, d, J=6.0), 7.17-7.38 (13H, m), 7.69 (2H, d, J=8.0), 8.79 (1H, s).

<Synthesis of compound B-7>

Under an argon atmosphere, compound B-4 (7.22 g, 10.19 mmol) was dissolved in dichloromethane (68 mL) and triethylamine (2.15 mL, 15.28 mmol) was added to the solution. Methanesulfonyl chloride (0.87 mL, 11.21 mmol) was added to the reaction mixture in an ice bath, and the mixture was stirred at room temperature for 20 minutes. The reaction was quenched with water, then the resultant solution was diluted with dichloromethane and brine, and the diluted solution was fractionated to obtain an organic layer. The obtained organic layer was washed with brine, then the washed solution was dried over anhydrous sodium sulfate, and the solvent was concentrated under reduced pressure to obtain an intermediate B-5.

Subsequently, the intermediate B-5 thus obtained was dissolved in pyridine (157 mL), and a 40% methylamine methanol solution (97 mL, 947 mmol) was added to the solution, and the mixture was stirred at room temperature for 24 hours, at 60°C for 4 hours, at room temperature for 13 hours, and at 60°C for 3 hours. The solvent was concentrated under reduced pressure until the volume of the reaction mixture became half, then the resultant solution was diluted with brine and ethyl acetate, and the diluted solution was fractionated to obtain an organic layer. The obtained organic layer was washed with brine, then the washed solution was dried over anhydrous sodium sulfate, and the solvent was concentrated under reduced pressure. From a crude product thus obtained, an intermediate B-6 was obtained by silica gel column chromatography (ethyl acetate : methanol : triethylamine = 20 : 1 : 1 to 20 : 2 : 1).

Under an argon atmosphere, the intermediate B-6 thus obtained was dissolved in tetrahydrofuran (200 mL), then sodium hydride (60% in mineral oil, 998 mg, 24.94 mmol) was added to the solution, and the mixture was stirred for 10 minutes. Subsequently, methyl iodide (518 μL, 8.31 mmol) was added to the solution and the mixture was stirred for 3 hours. The reaction mixture was cooled and the reaction was quenched with water, then the resultant solution was diluted with ethyl acetate, and the diluted solution was fractionated to obtain an organic layer. The obtained organic layer was washed with brine, then the washed solution was dried over anhydrous sodium sulfate, and the solvent was concentrated under reduced pressure. A crude product thus obtained was purified by silica gel column chromatography (chloroform : methanol = 30 : 1 to 20 : 1) to obtain compound B-7 (2.04 g, 36%) as a white foam-like solid.
$^1$H NMR($CDCl_3$)$\delta$ 1.34 (3H, d, J=1.0), 1.92-2.00 (2H, m), 2.04-2.11 (1H, m), 2.09 (1H, d, J=12.5), 2.31 (3H, s), 2.96-2.99 (1H, m), 3.28 (1H, d, J=12.0), 3.34-3.49 (1H, m), 3.53 (3H, s), 3.82 (1H, dd, J=3.0, 5.5), 4.15 (1H, d, J=6.0), 4.21, 4.59 (2H, ABq, J=10.5), 4.38, 4.73 (2H, ABq, J=11.5), 6.14 (1H, d, J=3.5), 7.14-7.17 (2H, m), 7.31-7.40 (8H, m), 7.76 (1H, d, J=1.0), 8.56 (1H, s).

<Synthesis of compound B-10>

Compound B-7 (1.92 g, 3.69 mmol) was dissolved in ethanol (77 mL), then 20% palladium hydroxide-carbon powder (1.35 g) and ammonium formate (2.33 g, 36.8 mmol) were sequentially added to the solution, and the mixture was heated under reflux for 1 hour. The reaction mixture was filtered and the solvent was concentrated under reduced pressure, and an intermediate B-8 was obtained from a crude product thus obtained by silica gel chromatography (chloroform : methanol = 50 : 1 to 30 : 1).

The intermediate B-8 thus obtained was dissolved in pyridine (23 mL), then acetic anhydride (764 μL, 8.08

mmol) and 4-dimethylaminopyridine (33 mg, 0.27 mmol) were sequentially added to the solution, and the mixture was stirred at room temperature for 1 hour. The solvent of the reaction mixture thus obtained was concentrated under reduced pressure, and an intermediate B-9 was obtained from the residue by silica gel column chromatography (chloroform : methanol = 30 : 1).

The intermediate B-9 thus obtained was dissolved in acetic acid (42 mL), then 20% palladium hydroxide-carbon powder (735 mg) and ammonium formate (1.40 g, 22.17 mmol) were sequentially added to the solution, and the mixture was stirred at 70°C for 1 hour. The reaction mixture was filtered and the solvent was concentrated under reduced pressure, and a crude product thus obtained was purified by silica gel chromatography (chloroform : methanol = 50 : 1) to obtain compound B-10 (770 mg, 43%) as a white foam-like solid.
HRMS(MALDI): calcd for C17H26N3O7 [M+H$^+$] 384.1765, found 384.1771.

<Synthesis of compound B-11>

Under an argon atmosphere, compound B-10 (50 mg, 0.13 mmol) was dissolved in a mixed solvent of pyridine (0.5 mL) and 2,6-lutidine (0.5 mL), then 4,4'-dimethoxytrityl chloride (88 mg, 0.26 mmol) and silver tetrafluoroborate (51 mg, 0.26 mmol) were sequentially added to the solution, and the mixture was stirred at room temperature for 1.5 hours, at 50°C for 4.5 hours, and at room temperature for 16 hours. Water was added to the solution and the reaction was quenched, then the resultant solution was diluted with ethyl acetate, and the diluted solution was fractionated to obtain an organic layer. The organic layer thus obtained was washed with brine and dried over anhydrous sodium sulfate, and the solvent was concentrated under reduced pressure. A crude product thus obtained was purified by silica gel chromatography (ethyl acetate : methanol = 50 : 1 to 0 : 1) to obtain compound B-11 (26 mg, 23%) as a light-yellow foam-like solid.
HRMS(MALDI): calcd for C38H44N3O9 [M+H$^+$] 686.3072, found 686.3075.

<Synthesis of compound B-12>

Compound B-11 (86 mg, 0.12 mmol) was dissolved in a 40% methylamine methanol solution (3.0 mL, 38 mmol) and the resultant solution was stirred in an ice bath for 1 hour and at room temperature for 2 hours. The solvent of the reaction mixture thus obtained was concentrated under reduced pressure and the residue was purified by silica gel chromatography to obtain compound B-12 (75 mg, 93%) as a white solid. HRMS(MALDI): calcd for C36H42N3O8 [M+H$^+$] 644.2966, found 644.2970.

<Synthesis of compound B-13>

Under an argon atmosphere, compound B-12 (31 mg, 0.04 mmol) was dissolved in acetonitrile, then 4,5-dicyanoimidazole (6 mg, 0.05 mmol) and 2-cyanoethyl N,N,N',N'-tetraisopropylphosphorodiamidite (18 $\mu$L, 0.06 mmol) were sequentially added to the solution in an ice bath, and the mixture was stirred at room temperature for 30 minutes. The reaction mixture was cooled and the reaction was quenched with saturated aqueous sodium bicarbonate, then the resultant solution was diluted with ethyl acetate, and the diluted solution was fractionated to obtain an organic layer. The obtained organic layer was washed with brine, then the washed solution was dried over anhydrous sodium sulfate, and the solvent was concentrated under reduced pressure. A crude product thus obtained was purified by silica gel chromatography (ethyl acetate : methanol = 10 : 1 to 5 : 1) to obtain compound B-13 (34 mg, 75%) as a white foam-like solid.

$^{31}$P NMR($CDCl_3$)$\delta$ 150.0, 151.0
HRMS(MALDI): calcd for C45H58N5NaO9P [M+Na$^+$] 866.3864, found 866.3826.

[Formula 30]

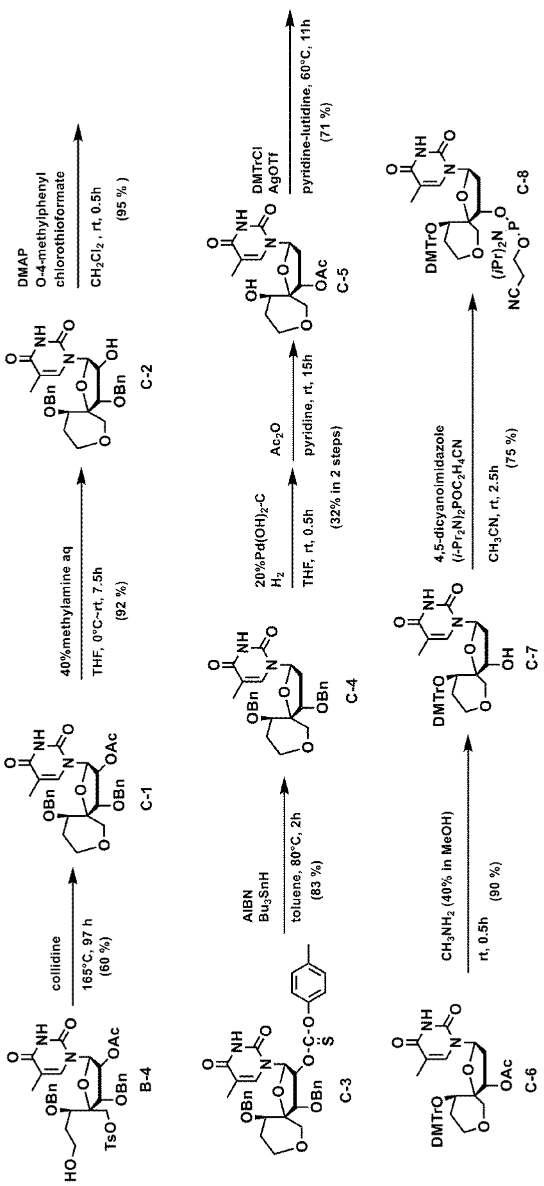


<Synthesis of compound C-1>

Under an argon atmosphere, compound B-4 (0.55 g, 0.77 mmol) was dissolved in collidine (10 mL) and the resultant solution was stirred at 165°C for 97 hours. The reaction mixture was diluted with water, ethyl acetate, and brine, and fractionated into an organic layer and an aqueous layer. The obtained organic layer was washed sequentially with 1N hydrochloric acid, saturated aqueous sodium bicarbonate solution, and brine, then the washed solution was dried over anhydrous sodium, and the solvent was concentrated under reduced pressure. A crude product thus obtained was purified by silica gel column chromatography (ethyl acetate : methanol = 1 : 0 to 10 : 1) to obtain compound C-1 (0.24 g, 60%) as a white foam-like solid.

$^1$H NMR($CDCl_3$)$\delta$ 1.45 (3H, d, J=1.0), 1.87-2.07 (2H, m), 2.11 (3H, s), 3.23, 4.17 (2H, ABq, J=12.5), 3.33 (1H, dt, J=2.0, 12.0), 3.42-3.47, 4.03-4.08 (2H, m), 4.27 (1H, d, J=5.5), 4.28, 4.59 (2H, ABq, J=11.5), 4.32, 4.63 (2H, ABq, J=10.5), 5.36 (1H, t, J=5.5), 6.33 (1H, d, J=6.0), 7.24-7.42 (10H, m), 7.64 (1H, d, J=1.0), 8.14 (1H, s).

<Synthesis of compound C-2>

Compound C-1 (0.27 g, 0.51 mmol) was dissolved in tetrahydrofuran (6 mL), then a 40% aqueous methylamine solution (0.56 mL, 6.71 mmol) was added to the solution in an ice bath, and the mixture was stirred in an ice bath for 4 hours and at room temperature for 2 hours. A 40% aqueous methylamine solution (0.14 mL, 1.67 mmol) was added to the solution, and the mixture was stirred at room temperature for 1.5 hours. The solvent of the obtained reaction mixture

was concentrated under reduced pressure, the residue was diluted with ethyl acetate and washed with brine, then the washed solution was dried over anhydrous sodium, and the solvent was concentrated under reduced pressure. A crude product thus obtained was purified by silica gel column chromatography (hexane : ethyl acetate = 1 : 4 to 0 : 1) to obtain compound C-2 (0.21 g, 92%) as a white foam-like solid.
$^1$H NMR($CDCl_3$)δ 1.50 (3H, d, J=1.0), 1.88-2.05 (2H, m), 3.24, 4.23 (2H, ABq, J=12.5), 3.33 (1H, dt, J=2.5, 11.5), 3.43-3.48, 4.00-4.08 (2H, m),3.52 (1H, d, J=9.0), 4.01 (1H, d, J=6.0), 4.32-4.39 (1H, m), 4.35, 4.65 (2H, ABq, J=11.0), 4.47, 4.75 (2H, ABq, J=11.5), 6.07 (1H, d, J=6.0), 7.25-7.40 (10H, m), 7.65 (1H, d, J=1.0), 8.92 (1H, s).

<Synthesis of compound C-3>

Under an argon atmosphere, compound C-2 (50 mg, 0.10 mmol) was dissolved in dichloromethane (3 mL), then 4-dimethylaminopyridine (49 mg, 0.40 mmol), and p-tolyl chlorothioformate (31 μL, 0.20 mmol) were sequentially added to the solution at room temperature, and the mixture was stirred for 30 minutes. The reaction mixture was diluted with water and fractionated into an organic layer and an aqueous layer. The obtained organic layer was washed sequentially with 1N hydrochloric acid, water, and brine, then the washed solution was dried over anhydrous sodium, and the solvent was concentrated under reduced pressure. A crude product thus obtained was purified by silica gel column chromatography (hexane : ethyl acetate = 3 : 2 to 2 : 3) to obtain compound C-3 (62 mg, 95%) as a white foam-like solid.
$^1$H NMR($CDCl_3$)δ 1.51 (3H, s), 1.91-1.99 (1H, m), 2.05-2.09(1H, m), 2.36 (3H,s), 3.21, 4.22 (2H, ABq, J=13.0), 3.33 (1H, dt, J=2.0, 12.5), 3.24, 4.23 (2H, ABq, J=12.5), 3.33 (1H, dt, J=2.5, 11.5), 3.46-3.49, 4.05-4.08 (2H, m), 4.41 (1H, d, J=11.5), 4.45 (1H, d, J=10.5), 4.48 (1H, d, J=5.5), 4.68 (1H, d, J=11.0), 4.76 (1H, d, J=11.0), 5.85 (1H, dd, J=5.5, 6.5), 6.56 (1H, d, J=7.0), 6.91 (2H, d, J=8.0), 7.19 (2H, d, J=8.5), 7.30-7.43 (10H, m), 7.63 (1H, s), 8.09 (1H, s).

<Synthesis of compound C-4>

Under an argon atmosphere, compound C-3 (25 mg, 0.038 mmol) was dissolved in toluene (0.8 mL), then 2,2'-azobis(isobutyronitrile) (1.3 mg, 7.74 μmol), and tributyltin hydride (0.10 mL, 0.39 mmol) were sequentially added to the solution at room temperature, and the mixture was stirred at 80°C for 2 hours. The solvent of the reaction mixture thus obtained was concentrated under reduced pressure and the residue was purified by silica gel column chromatography (hexane : ethyl acetate = 3 : 2 to 1 : 2) to obtain compound C-4 (15 mg, 83%) as a white foam-like solid.
$^1$H NMR($CDCl_3$)δ 1.53 (3H, d, J=1.0), 1.91-2.10 (2H, m), 2.14-2.23 (1H, m), 2.42-2.49 (1H, m), 3.36-3.44 (1H,m), 3.39, 4.11 (2H, ABq, J=12.5), 3.54-3.60 (1H, m), 4.06-4.11 (1H, m), 4.17 (1H, dd, J=2.5, 6.0), 4.30, 4.61 (2H, ABq, J=12.0), 4.38, 4.72 (2H, ABq, J=10.5), 6.43 (1H, dd, J=5.5, 8.0), 7.24-7.40 (10H, m), 7.78 (1H, d, J=1.0), 8.23 (1H, s).

<Synthesis of compound C-5>

Compound C-4 (0.90 g, 1.84 mmol) was dissolved in tetrahydrofuran (45 mL), then a 20% palladium hydroxide-carbon powder (0.54 g) was added to the solution at room temperature, and under a hydrogen atmosphere at atmospheric pressure, the mixture was stirred for 0.5 hours. The reaction mixture was filtered and the solvent was concentrated under reduced pressure to obtain an intermediate. Subsequently, the intermediate thus obtained was dissolved in pyridine (17 mL), then acetic anhydride (0.42 mL, 3.67 mmol) was added to the solution, and the mixture was stirred at room temperature for 15 hours. The solvent of the reaction mixture thus obtained was concentrated under reduced pressure and the residue was purified by silica gel column chromatography (ethyl acetate : methanol = 100 : 0 to 5 : 1) to obtain compound C-5 (0.20 g, 32%) as a white solid.
$^1$H NMR(DMSO-d6)δ 1.56-1.72 (2H, m), 1.77 (3H, d, J=1.0), 2.07 (3H, s), 2.12-2.23 (1H, m), 2.33-2.42 (1H, m), 3.19, 3.78 (2H, ABq, J=12.0), 3.27-3.36 (1H, m), 3.70-3.80 (2H, m), 5.42 (1H, dd, J=1.5, 6.5), 5.64 (1H, d, J=4.5), 6.24 (1H, dd, J=5.5, 9.0), 8.02 (1H, s), 11.4 (1H, s).

<Synthesis of compound C-6>

Under an argon atmosphere, compound C-5 (0.13 g, 0.39 mmol) was dissolved in a mixed solvent of pyridine (5 mL) and 2,6-lutidine (5 mL), then 4,4'-dimethoxytrityl chloride (1.06 g, 3.12 mmol) and silver trifluoromethanesulfonate (0.80 g, 3.12 mmol) were sequentially added to the solution, and the mixture was stirred at 60° C for 11 hours. Water was added to the solution and the reaction was quenched, then the resultant solution was diluted with ethyl acetate, and the diluted solution was fractionated to obtain an organic layer. The organic layer thus obtained was washed with brine and dried over anhydrous sodium sulfate, and the solvent was concentrated under reduced pressure. A crude product thus obtained was purified by silica gel chromatography (ethyl acetate : methanol = 100 : 0 to 50 : 1) to obtain compound C-6 (0.19 g, 71%) as a light-yellow foam-like solid.
$^1$H NMR(DMSO-d6)δ 1.27-1.30 (1H, m), 1.67 (3H, s), 1.77-1.81 (1H, m), 1.95 (3H, s), 2.30-2.38 (1H, m), 2.47-2.57 (1H,

m), 2.72-2.79 (1H, m), 2.92, 3.82 (2H, ABq, J=12.5), 3.17-3.22 (1H, m), 3.60-3.64 (1H, m), 3.74 (3H,s),3.74 (3H, s), 5.26 (1H, dd, J=3.5, 7.5), 6.22 (1H, 7, J=7.0), 6.82-6.93 (4H, m), 7.17-7.42 (9H, m), 8.02 (1H, s), 9.31 (1H, s).

<Synthesis of compound C-7>

Compound C-6 (0.18 g, 0.26 mmol) was dissolved in a 40% methylamine methanol solution (7.3 mL, 69.9 mmol), and the resultant solution was stirred at room temperature for 0.5 hours. The solvent of the reaction mixture thus obtained was concentrated under reduced pressure and the residue was purified by silica gel chromatography (hexane : ethyl acetate = 1 : 4 to 0 : 1) to obtain compound C-7 (0.15 g, 90%) as a white foam-like solid.
$^{1}H$ NMR(DMSO-d6)6 1.21-1.30 (1H, m), 1.60 (3H, s), 1.74-1.89 (1H, m), 2.17-2.24 (1H, m), 2.35-2.44 (1H, m), 2.72 (1H, t, J=11.5), 2.98, 3.75 (2H, ABq, J=12.0), 3.15 (1H, dd, J=3.5, 11.5), 3.58-3.61 (1H, m), 3.74 (3H, s), 3.75 (3H, s), 4.14-4.22 (1H, m), 5.14 (1H, d, J=5.0), 6.21 (1H, dd, J=6.0, 7.5), 6.88-6.93 (4H, m), 7.20-7.41 (9H, m), 8.05 (1H, s), 11.4 (1H, s).

<Synthesis of compound C-8>

Under an argon atmosphere, compound C-7 (0.14 g, 0.24 mmol) was dissolved in acetonitrile (2.2 mL), then 4,5-dicyanoimidazole (40 mg, 0.34 mmol) and 2-cyanoethyl N,N,N',N'-tetraisopropylphosphorodiamidite (0.11 mL, 0.36 mmol) were sequentially added to the solution in an ice bath, and the mixture was stirred at room temperature for 2.5 hours. The reaction mixture was cooled and the reaction was quenched with water, then the resultant solution was diluted with ethyl acetate, and the diluted solution was fractionated to obtain an organic layer. The obtained organic layer was washed with brine, then the washed solution was dried over anhydrous sodium sulfate, and the solvent was concentrated under reduced pressure. A crude product thus obtained was purified by silica gel chromatography (hexane : ethyl acetate = 1 : 1 to 2 : 3) to obtain compound C-8 (0.14 g, 75%) as a white foam-like solid.

$^{31}P$ NMR(Acetone-d6)δ 149.3, 149.9.
HRMS(MALDI): calcd for C43H53N4NaO9P [M+Na$^+$] 823.3442, found 823.3458.

[Formula 31]

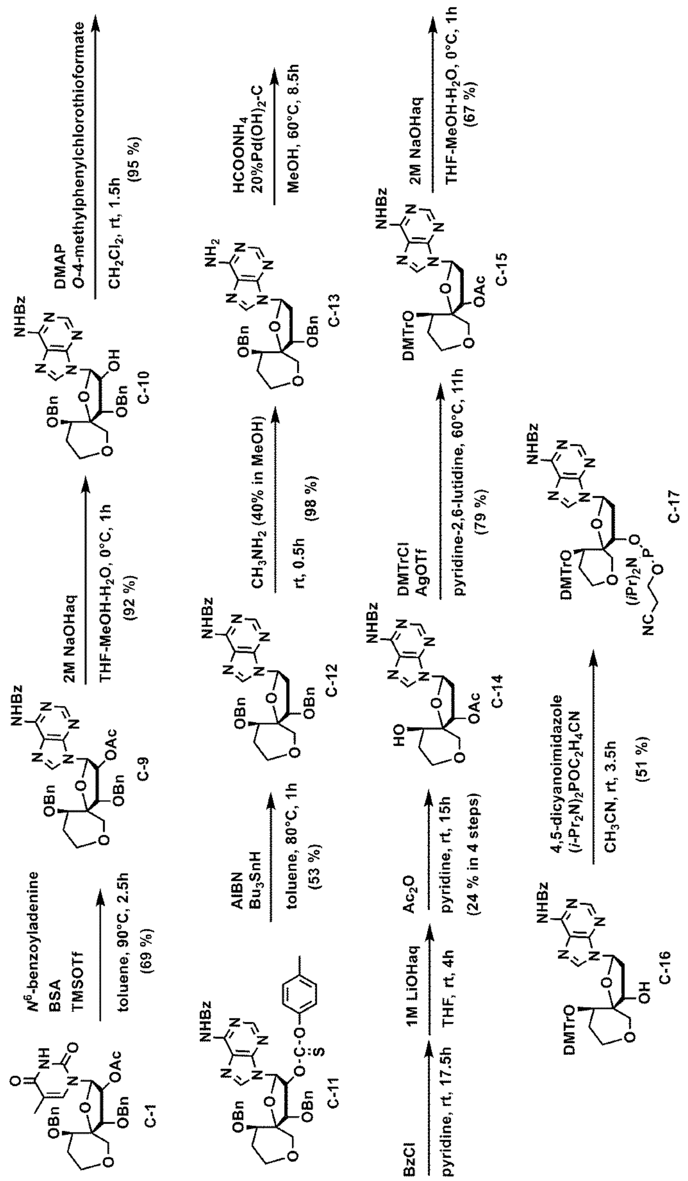


<Synthesis of compound C-9>

Under an argon atmosphere, compound C-1 (5.64 g, 10.5 mmol) was dissolved in toluene (90 mL), then $N^6$-benzoyladenine (4.28g, 17.9 mmol) and N,O-bis(trimethylsilyl)acetamide (16.6 mL, 67.1 mmol) were sequentially added to the solution, and the mixture was stirred at 90°C for 30 minutes. Subsequently, trimethylsilyl trifluoromethanesulfonate (3.2 mL, 17.9 mmol) was added to the solution, and the mixture was stirred at 90°C for 2 hours. The reaction mixture was cooled in an ice bath and the reaction was quenched with saturated aqueous sodium bicarbonate, then the resultant solution was diluted with ethyl acetate and water, then the diluted solution was subjected to filtration through celite to collect a filtrate, and the filtrate was fractionated to obtain an organic layer. The obtained organic layer was washed with brine, then the washed solution was dried over anhydrous sodium sulfate, and the solvent was concentrated under reduced pressure. A crude product thus obtained was purified by silica gel column chromatography (hexane : ethyl acetate = 1 : 4) to obtain compound C-9 (4.73 g, 69%) as a white foam-like solid.
$^1$H NMR($CDCl_3$)$\delta$ 1.86-1.98 (2H, m), 2.10 (3H, s), 3.29-3.34 (1H, m), 3.29, 4.23 (2H, ABq, J=12.5), 3.43-3.78, 4.01-4.05 (2H, m), 4.21, 4.56 (2H, ABq, J=11.5), 4.26, 4.57 (2H, ABq, J=11.5), 4.46 (1H, d, J=5.0), 6.01 (1H, dd, J=5.0, 5.0), 6.48 (1H, d, J=5.0), 7.19-7.39 (10H, m), 7.51-7.65 (3H, m), 8.00-8.03 (2H, m), 8.47 (1H, s), 8.79 (1H, s), 8.90 (1H, s).

<Synthesis of compound C-10>

Compound C-9 (4.73 g, 7.28 mmol) was dissolved in tetrahydrofuran (80 mL) and methanol (64 mL) and water

(16 mL) were sequentially added to the solution. Subsequently, a 2M sodium hydroxide solution (4.0 mL, 8.00 mmol) was added to the solution in an ice bath and the mixture was stirred for 1 hour. The reaction mixture was neutralized by adding acetic acid, then the solvent of the reaction mixture was concentrated under reduced pressure, then the residue thus obtained was diluted with ethyl acetate and water, and the diluted solution was fractionated to obtain an organic layer. The obtained organic layer was washed with brine, then the washed solution was dried over anhydrous sodium sulfate, and the solvent was concentrated under reduced pressure. A crude product thus obtained was purified by silica gel column chromatography (hexane : ethyl acetate = 1 : 4 to 0 : 1) to obtain compound C-10 (4.10 g, 92%) as a white foam-like solid.

$^1$H NMR($CDCl_3$)$\delta$ 1.92-2.00 (2H, m), 3.29-3.39 (2H, m), 3.31 (1H, d, J=12.5), 3.42-3.51 (2H, m), 4.02-4.06 (1H, m), 4.15 (1H, d, J=5.5), 4.26 (1H, d, J=11.5), 4.30 (1H, d, J=11.5), 4.48 (1H, d, J=11.5), 4.62 (1H, d, J=11.5), 4.67 (1H, d, J=11.5), 4.83-4.89 (1H, m), 6.23 (1H, d, J=5.0), 7.18-7.41 (10H, m), 7.50-7.64 (3H, m), 8.02 (2H, d, J=7.5), 8.45 (1H, s), 8.74 (1H, s), 8.97 (1H, s).

<Synthesis of compound C-11>

Under an argon atmosphere, compound C-10 (4.07 g, 6.70 mmol) was dissolved in dichloromethane (98 mL), then 4-dimethylaminopyridine (1.17 g, 9.58 mmol) and p-tolyl chlorothioformate (1.2 mL, 8.20 mmol) were sequentially added to the solution at room temperature, and the mixture was stirred for 1.5 hours. The reaction mixture was diluted with water and fractionated into an organic layer and an aqueous layer. The obtained organic layer was washed sequentially with a 0.5N hydrochloric acid and brine, then the washed solution was dried over anhydrous sodium sulfate, and the solvent was concentrated under reduced pressure. A crude product thus obtained was purified by silica gel column chromatography (hexane : ethyl acetate = 1 : 2 to 1 : 4) to obtain compound C-11 (4.82 g, 95%) as a white foam-like solid.

$^1$H NMR($CDCl_3$)$\delta$ 1.95-2.02 (2H, m), 2.63-2.71 (1H, m), 2.74-2.82 (1H, m), 3.33-3.44 (1H, m), 3.43, 4.15 (2H, ABq, J=13.0), 3.52-3.57 (1H, m), 4.01-4.10 (1H, m), 4.24 (1H, dd, J=3.5, 6.0), 4.29 (1H, d, J=11.5), 4.34 (1H, d, J=11.5), 4.58 (1H, d, J=12.0), 4.67 (1H, d, J=11.5), 6.66 (1H, t, J=6.5), 7.19-7.40 (10H, m), 7.50-7.64 (3H, m), 8.01-8.04 (2H, m), 8.59 (1H, s), 8.76 (1H, s), 8.97 (1H, s).

<Synthesis of compound C-12>

Under an argon atmosphere, compound C-11 (4.81 g, 6.34 mmol) was dissolved in toluene (126 mL), then 2,2'-azobis(isobutyronitrile) (0.21 g, 1.29 mmol), and tributyltin hydride (17.4 mL, 64.7 mmol) were sequentially added to the solution at room temperature, and the mixture was stirred at 80°C for 1 hours. The solvent of the reaction mixture thus obtained was concentrated under reduced pressure and the residue was purified by silica gel column chromatography (hexane : ethyl acetate = 1 : 1) to obtain compound C-12 (1.99 g, 53%) as a white foam-like solid.

$^1$H NMR($CDCl_3$)$\delta$ 1.95-2.02 (2H, m), 2.63-2.71 (1H, m), 2.74-2.82 (1H, m), 3.33-3.44 (1H, m), 3.43, 4.15 (2H, ABq, J=13.0), 3.52-3.57 (1H, m), 4.01-4.10 (1H, m), 4.24 (1H, dd, J=3.5, 6.0), 4.29 (1H, d, J=11.5), 4.34 (1H, d, J=11.5), 4.58 (1H, d, J=12.0), 4.67 (1H, d, J=11.5), 6.66 (1H, t, J=6.5), 7.19-7.40 (10H, m), 7.50-7.64 (3H, m), 8.01-8.04 (2H, m), 8.59 (1H, s), 8.76 (1H, s), 8.97 (1H, s).

<Synthesis of compound C-13>

Under an argon atmosphere, compound C-12 (0.40 g, 0.676 mmol) was dissolved in a 40% methylamine methanol solution (4.0 mL, 38.2 mmol) and the resultant solution was stirred at room temperature for 0.5 hours. The solvent of the reaction mixture thus obtained was concentrated under reduced pressure and the residue was purified by silica gel chromatography (ethyl acetate : methanol = 1 : 0 to 10 : 1) to obtain compound C-13 (0.32 g, 98%) as a white foam-like solid.

$^1$H NMR($CDCl_3$)$\delta$ 1.96-2.03 (2H, m), 2.58-2.71 (2H, m), 3.30-3.48 (1H, m), 3.43, 4.14 (2H, ABq, J=12.5), 3.53-3.59 (1H, m), 4.02-4.07 (2H, m), 4.22 (1H, t, J=4.5), 4.27, 4.57 (2H, ABq, J=12.0), 4.37, 4.70 (2H, ABq, J=11.5), 5.63 (2H, brs), 6.60 (1H, t, J=6.5), 7.22-7.39 (10H, m), 8.32 (1H, s), 8.36 (1H, s).

<Synthesis of compound C-14>

Under a hydrogen atmosphere, compound C-13 (320 mg, 0.664 mmol) was dissolved in methanol (6.4 mL), then a 20% palladium hydroxide-carbon powder (0.31 g) and ammonium formate (2.51 g, 39.8 mmol) were sequentially added to the solution and the mixture was heated to 60°C. The mixture was stirred for 6 hours while adding ammonium formate (1.26 g, 19.9 mmol) four times in the middle, and the reaction mixture was filtered and the solvent was concentrated under reduced pressure. The residue thus obtained was dissolved in methanol (6.4 mL), then a 20% palladium hydroxide-carbon powder (0.31 g) was added to the solution, and the mixture was stirred at 60°C for 2.5 hours. The reaction mixture was filtered and the solvent was concentrated under reduced pressure to obtain an intermediate. Subsequently, the intermediate thus obtained was dissolved in pyridine (4 mL), then benzoyl chloride (0.27 mL, 2.31 mmol) was added to the solution at room temperature, and the mixture was stirred for 17.5 hours. Water was added to the reaction mixture and the reaction was quenched, then the resultant solution was diluted with ethyl acetate, and the diluted solution was fractionated to obtain an organic layer. The obtained organic layer was washed with brine, then the washed solution was dried over anhydrous sodium sulfate, and the solvent was concentrated under reduced pressure to obtain an intermediate. Subsequently, the intermediate thus obtained was dissolved in tetrahydrofuran (5 mL), and a 1M lithium-hydroxide aqueous solution (2.8 mL, 2.80 mmol) was added to the solution at room temperature, and the mixture was stirred for 4 hours. The reaction mixture was neutralized by adding acetic acid, and the solvent of the reaction mixture was concentrated under reduced pressure to obtain an intermediate. Subsequently, the intermediate thus obtained was dissolved in pyridine (6 mL), then acetic anhydride (0.11 mL, 0.924 mmol) was added to the solution at room temperature, and the mixture was stirred for 15 hours. The solvent of the reaction mixture was concentrated under reduced pressure, and a crude product thus obtained was purified by silica gel chromatography (ethyl acetate : methanol = 20 : 1 to 10 : 1) to obtain compound C-14 (73 mg, 24%) as a light-yellow solid.
$^1$H NMR($CDCl_3$)δ 1.76-1.91 (3H, m), 2.15 (3H, s), 2.40 (1H, dd, J=5.0, 14.0), 3.35 (1H, d, J=12.0), 3.39-3.48 (1H, m), 3.81-3.90 (1H, m), 3.93-4.00 (1H, m), 4.04 (1H, d, J=12.5), 5.78 (1H, d, J=5.5), 6.31 (1H, d, J=11.5), 6.42 (1H, dd, J=5.0, 10.0), 7.47-7.65 (3H, m), 8.01-8.04 (2H, m), 8.09 (1H, s), 8.79 (1H, s), 9.14 (1H, s).

<Synthesis of compound C-15>

Under an argon atmosphere, compound C-14 (73 mg, 0.163 mmol) was dissolved in a mixed solvent of pyridine (1.6 mL) and 2,6-lutidine (1.6 mL), then 4,4'-dimethoxytrityl chloride (0.43 g, 1.28 mmol) and silver trifluoromethanesulfonate (0.32 g, 1.28 mmol) were sequentially added to the solution, and the mixture was stirred at 60°C for 11 hours. Methanol was added to the solution and the reaction was quenched, then the resultant solution was diluted with ethyl acetate and water, and the diluted solution was fractionated to obtain an organic layer. The organic layer thus obtained was washed with brine and dried over anhydrous sodium sulfate, and the solvent was concentrated under reduced pressure. A crude product thus obtained was purified by silica gel chromatography (ethyl acetate : hexane = 4 : 1) to obtain compound C-15 (95 mg, 79%) as a light-yellow foam-like solid.
$^1$H NMR($CD_3OD$)δ1.45-1.49 (1H, m), 1.74-1.94 (1H, m), 2.00 (3H, s), 2.59-2.67 (1H, m), 3.03-3.09 (1H, m), 3.26-3.43 (2H, m), 3.50-3.58 (1H, m), 3.71-3.80 (1H, m), 3.73 (3H, s), 3.76 (3H, s), 4.00 (1H, d, J=12.5), 5.39 (1H, dd, J=3.5, 7.0), 6.56 (1H, t, J=6.5), 6.68 (2H, d, J=8.5), 6.78 (2H, d, J=8.5), 7.13-7.38 (9H, m), 7.55-7.69 (3H, m), 7.90 (1H, s), 8.09-8.12 (2H, m), 8.72 (1H, s), 8.79 (1H, s).

<Synthesis of compound C-16>

Compound C-15 (95 mg, 0.125 mmol) was dissolved in tetrahydrofuran (1.5 mL) and methanol (1.2 mL) and water (0.3 mL) were sequentially added to the solution. Subsequently, a 2M sodium hydroxide solution (76 μL, 0.152 mmol) was added to the solution in an ice bath and the mixture was stirred for 1 hour. The reaction mixture was neutralized by adding acetic acid, then the solvent of the reaction mixture was concentrated under reduced pressure, then the residue thus obtained was diluted with ethyl acetate and water, and the diluted solution was fractionated to obtain an organic layer. The obtained organic layer was washed with brine, then the washed solution was dried over anhydrous sodium sulfate, and the solvent was concentrated under reduced pressure. A crude product thus obtained was purified by silica gel column chromatography (hexane : ethyl acetate = 1 : 4 to 0 : 1) to obtain compound C-16 (60 mg, 67%) as a light-yellow foam-like solid.
$^1$H NMR($CD_3OD$)δ 1.46-1.56 (1H, m), 1.81-1.94 (1H, m), 2.54-2.62 (1H, m), 3.12 (1H, quint, J=6.5), 3.20-3.60 (1H, m), 3.56-3.67 (1H, m), 3.70-3.90 (9H, m), 4.10 (1H, d, J=11.5), 4.51-4.60 (1H, br), 6.48 (1H, t, J=6.5), 6.61 (2H, d, J=9.0), 6.72 (2H, d, J=9.0), 7.13-7.19 (3H, m), 7.25-7.38 (6H, m), 7.52-7.65 (3H, m), 8.06 (2H, d, J=7.0), 8.35 (1H, brs), 8.75 (1H, s), 9.06 (1H, brs).

<Synthesis of compound C-17>

Under an argon atmosphere, compound C-16 (50 mg, 0.0700 mmol) was dissolved in acetonitrile (1.5 mL), then 4,5-dicyanoimidazole (15 mg, 0.126 mmol) and 2-cyanoethyl N,N,N',N'-tetraisopropylphosphorodiamidite (46 μL, 0.140 mmol) were sequentially added to the solution in an ice bath, and the mixture was stirred at room temperature for 3.5 hours. The reaction mixture was cooled and the reaction was quenched with water, then the resultant solution was diluted with ethyl acetate, and the diluted solution was fractionated to obtain an organic layer. The obtained organic layer was washed with brine, then the washed solution was dried over anhydrous sodium sulfate, and the solvent was concentrated under reduced pressure. A crude product thus obtained was purified by silica gel chromatography (hexane : ethyl acetate = 1 : 2 to 1 : 4) to obtain compound C-17 (32 mg, 51%) as a white foam-like solid. $^{31}$P NMR($CDCl_3$)δ 148.7, 150.2.
HRMS(MALDI): calcd for C50H56N7NaO8P [M+Na+] 936.3820, found 936.3802.

[Synthesis Example of Oligonucleotide]

Oligonucleotides were synthesized in accordance with a standard phosphoramidite protocol using the compounds (A(α)-12, A(β)-12, B-13, C-8, C-17) obtained in the synthesis examples by using a nucleic acid automatic synthesizer (Expedite (registered trademark) 8909, manufactured by ABI). In this manner, the following oligonucleotides were produced.
An oligonucleotide having a unit as an α-isomer represented by formula (6) wherein "Base" was a thymin-1-yl group, $X^1$ was a hydrogen atom, $X^2$ was a methoxy group, $R^3$, $R^4$, $R^5$, and $R^6$ were hydrogen atoms, $Z^1$ was O, and $Z^2$ was a single bond (hereinafter referred to as (A)α-T).

[Formula 32]

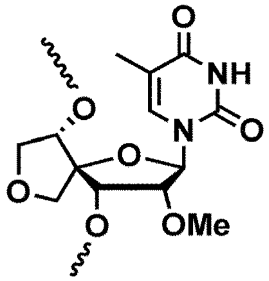

Hereinafter, an oligonucleotide having at least one (A)α-T is referred to as a "(A)α-T modified oligonucleotide".
An oligonucleotide having a unit as a β-isomer represented by formula (6) wherein "Base" was a thymin-1-yl group, $X^1$ was a hydrogen atom, $X^2$ was a methoxy group, $R^3$, $R^4$, $R^5$, and $R^6$ were hydrogen atoms, $Z^1$ was O, and $Z^2$ was a single bond (hereinafter referred to as (A) β-T).

[Formula 33]

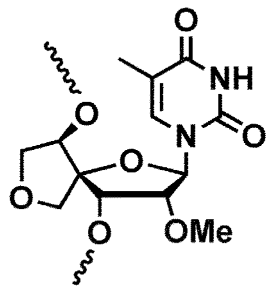

Hereinafter, an oligonucleotide having at least one (A)P-T is referred to as a "(A)β-T modified oligonucleotide".
An oligonucleotide having a unit as a β-isomer represented by formula (6) wherein "Base" was a thymin-1-yl group, $X^1$ was a hydrogen atom, $X^2$ was a methoxy group, $R^3$, $R^4$, $R^5$, and $R^6$ were hydrogen atoms, $Z^1$ was $NZ^{11}$, $Z^{11}$ was a methyl group, $Z^2$ was $CZ^{21}Z^{22}$, and $Z^{21}$ and $Z^{22}$ were hydrogen atoms (hereinafter referred to as (B)β-T).

[Formula 34]

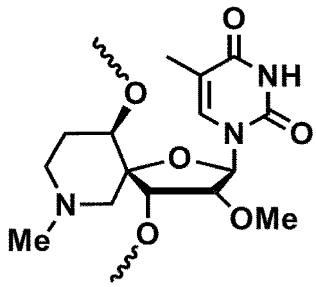

Hereinafter, an oligonucleotide having at least one (B)β-T is referred to as a "(B)P-T modified oligonucleotide".

An oligonucleotide having a unit as a β-isomer represented by formula (6) wherein "Base" was a thymin-1-yl group, $X^1$ was a hydrogen atom, $X^2$ was a hydrogen atom, $R^3$, $R^4$, $R^5$, and $R^6$ were hydrogen atoms, $Z^1$ was O, $Z^2$ was $CZ^{21}Z^{22}$, and $Z^{21}$ and $Z^{22}$ were hydrogen atoms (hereinafter referred to as (C)Hβ-T).

[Formula 35]

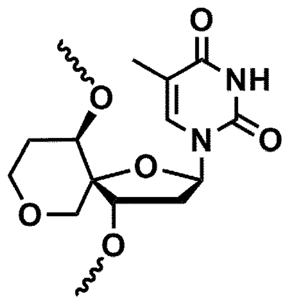

Hereinafter, an oligonucleotide having at least one (C)Hβ-T is referred to as a "(C)Hβ-T modified oligonucleotide".

An oligonucleotide having a unit as a β-isomer represented by formula (6) wherein "Base" was an adenin-9-yl group, $X^1$ was a hydrogen atom, $X^2$ was a hydrogen atom, $R^3$, $R^4$, $R^5$, and $R^6$ were hydrogen atoms, $Z^1$ was O, $Z^2$ was $CZ^{21}Z^{22}$, and $Z^{21}$ and $Z^{22}$ were hydrogen atoms (hereinafter referred to as (C)Hβ-A).

[Formula 36]

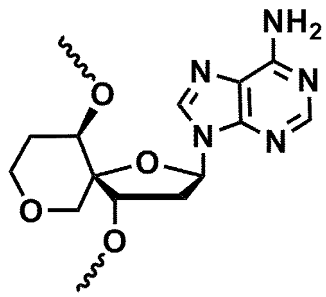

Hereinafter, an oligonucleotide having at least one (C)Hβ-A is referred to as a "(C)Hβ-A modified oligonucleotide".

Each of the oligonucleotides of which the 5'-terminal was protected by a dimethoxytrityl group and which was supported on a solid phase was subjected to the cleavage from a column with a 28% aqueous ammonia (for 1.5 hours), and then a cleaved oligonucleotide was reacted in a 28% aqueous ammonia for 16 hours at 60°C to deprotect all of the protecting groups.

The oligonucleotide was subjected to a simple purification using an NAP-10 column, and was then purified by reversed-phase HPLC [WakoPak (registered trademark) WS-DNA column, 10.0 mm × 250 mm)] [conditions: a gradient

of 8 to 16% of acetonitrile in a 0.1M triethylammonium acetate buffer (pH 7.0) at 3 ml/min for 30 minutes, column temperature of 50°C].

The purities of the synthesized oligonucleotides were confirmed by reversed-phase HPLC [WakoPak (registered trademark) WS-DNA column, 4.6 mm × 250 mm)] [conditions: a gradient of 8 to 16% of acetonitrile in a 0.1M triethylammonium acetate buffer (pH 7.0) at 1 ml/min for 30 minutes, column temperature of 50°C, detection wavelength of 254 nm]. Each of the synthesized oligonucleotides had purity of 93.2% or more.

The molecular weights of the synthesized oligonucleotides were determined by a MALDI-TOF-MASS measurement. The calculated values and the found values (measurement results) of the molecular weights are shown in table 1A and table 1B. The unit represented by formula (6) was incorporated at a position indicated by x in each of the antisense strands (SEQ ID NOs: 1 to 5) shown in table 1A or a position indicated by x in the sense strand (SEQ ID NO:6) shown in table 1B. Each of the nucleotide sequences excluding x was composed of DNA (formula (7) wherein $R^a$ = H).

[Table 1A]

MALDI-TOF-MASS

| | Calculated value $[M-H]^-$/found value $[M-H]^-$ | | | |
|---|---|---|---|---|
| | **x** | | | |
| Antisense strands | (A)α-T | (A)β-T | (B)β-T | (C)Hβ-T |
| 5'-d(gcgtt**x**tttgct)-3' | 3704.48/3705.80 | 3704.48/3704.89 | 3732.56/3732.07 | 3688.48/3689.81 |
| 5'-d(gcg**x**t**x**t**x**tgct)-3' | 3848.60/3849.72 | 3848.60/3849.89 | 3932.84/3930.60 | 3800.60/3802.79 |
| 5'-d(gcgt**xxx**ttgct)-3' | 3848.60/3849.52 | 3848.60/3849.86 | 3932.84/3930.18 | 3800.60/3800.43 |
| 5'-d(gcg**xxxxxx**gct)-3' | 4064.78/4065.35 | 4064.78/4065.47 | 4227.20/4229.00 | 3968.78/3968.90 |
| 5'-d(tttttttt**x**t)-3' | 3051.09/3053.07 | 3051.09/3053.10 | 3079.17/3078.28 | 3035.09/3035.96 |

[Table 1B]

MALDI-TOF-MASS

| | Calculated value $[M-H]^-$/found value $[M-H]^-$ |
|---|---|
| | **x** |
| Sense strand | (C)Hβ-A |
| 5'-d(agcaaa**x**aacgc)-3' | 3712.53/3713.00 |

[Test Examples]

[Test Example 1] Measurement of melting temperatures (Tm) of oligonucleotides

(Evaluation of double strand forming capability)

The double strand forming capability of antisense strands was examined by measuring a melting temperature (Tm) between: each of oligonucleotides ((A)α-T modified oligonucleotide, (A)β-T modified oligonucleotide, (B)β-T modified oligonucleotide, (C)Hβ-T modified oligonucleotide) (antisense strands) according to the present invention which were respectively synthesized by incorporating the compounds (A(α)-12, A(β)-12, β-13, C-8) produced in the synthesis examples at a position of the antisense strands indicated by x in the sequence represented by SEQ ID NOs: 1 to 3 in Table 2 and Table 3; and single-stranded DNA (5'-d(agcaaaaaacgc)-3'; SEQ ID NO: 7) (a sense strand) or single-stranded RNA (5'-r(agcaaaaaacgc)-3'; SEQ ID NO: 8) (a sense strand). As references, an oligonucleotide (hereinafter, called DNA-T oligonucleotide) which was synthesized in such a manner that a unit (T) (hereinafter, called DNA-T) represented by formula (7) wherein "Base" was a thymin-1-yl group and $R^a$ was H was positioned at a position of the antisense strands in Table 2 and Table 3 indicated by x and an oligonucleotide (hereinafter, called NMe-T modified oligonucleotide) which was synthesized in such a manner that a unit (hereinafter, called NMe-T) represented by formula (9) wherein "Base" was a thymin-1-yl group and $R^b$ was Me was positioned at a position indicated by x were prepared as antisense strands.

A sample solution (120 μl) in which the final concentrations of sodium chloride, a sodium phosphate buffer solution (pH 7.2), each of the antisense strands, and each of the sense strands were adjusted to 100 mM, 10 mM, 4 μM, and 4 μM, respectively, was prepared, then the sample solution was warmed from 0°C or 15°C to 110°C at a rate

of 0.5°C/min, and an absorbance at 260 nm was measured at 0.5°C intervals using a spectrophotometer (UV-1800 or UV-1900, manufactured by Shimadzu Corporation).

A Tm value was calculated from the obtained measurement value by a differentiation method. The results are shown in Table 2 and Table 3.

Capability of forming double strand with single-stranded DNA (Tm value)

[Table 2]

| Antisense strands | | Tm(°C) | | | | | |
|---|---|---|---|---|---|---|---|
| | x = | DNA-T | NMe-T | (A)α-T | (A)β-T | (B)β-T | (C)Hβ-T |
| 5'-d(gcgttxtttgct)-3' | | 52 | 50 | 38 | 45 | 49 | 48 |
| 5'-d(gcgxtxtxtgct)-3' | | 52 | 52 | 5 | 34 | 38 | 42 |
| 5'-d(gcgtxxxttgct)-3' | | 52 | 51 | 16 | 39 | 40 | 41 |

Sense strand = 5'-d(agcaaaaaacgc)-3'

Capability of forming double strand with single-stranded RNA (Tm value)

[Table 3]

| Antisense strands | | Tm(°C) | | | | | |
|---|---|---|---|---|---|---|---|
| | x = | DNA-T | NMe-T | (A)α-T | (A)β-T | (B)β-T | (C)Hβ-T |
| 5'-d(gcgttxtttgct)-3' | | 48 | 51 | 40 | 45 | 47 | 46 |
| 5'-d(gcgxtxtxtgct)-3' | | 48 | 64 | 17 | 45 | 37 | 45 |
| 5'-d(gcgtxxxttgct)-3' | | 48 | 60 | 24 | 44 | 33 | 44 |

Sense strand = 5'-r(agcaaaaaacgc)-3'

It was demonstrated that each of the oligonucleotides ((B)β-T modified oligonucleotides, (C)Hβ-T modified oligonucleotides) (antisense strand as shown in SEQ ID NO: 1) modified with one base using the compound (B-13, C-8) according to the present invention had superior capability of forming a double strand together with single-stranded DNA over single-stranded RNA. Accordingly, the oligonucleotides are each suitable as a material for DNA selective nucleic acid therapeutics (antigene) and genetic diagnosis. Further, it was confirmed that the larger the number of the monomeric units ((A)α-T, (A)β-T, (B)β-T, and (C)Hβ-T) of the present invention in the oligonucleotide, the lower the binding strength with the complementary strand. For example, the oligonucleotides can be expected to be used for double-stranded nucleic acid therapeutics such as siRNA that needs to be dissociated in vivo or genetic examination in the clamp PCR method using a clamp oligonucleotide having (A)α-T, (A)β-T, (B)β-T, or (C)Hβ-T at the 3'-terminal as a site that is not bound to a template.

[Test Example 2] Measurement of enzyme resistance capability of oligonucleotides 1) Preparation of oligonucleotides for use in measurement of enzyme resistance capability

Oligonucleotides shown in the table below were prepared in the same manner as in the synthesis example of the oligonucleotides, each of which had a sequence represented by SEQ ID NO: 10 having such a structure that the sequence represented by SEQ ID NO: 9 (TTTTTTTTTT) or a portion thereof was modified.

[Table 4]

| No. | Name of oligonucleotide | Sequence (5'→3') | Length (mer) |
|---|---|---|---|
| 1 | DNA oligonucleotide | TTTTTTTTTT | 10 |
| 2 | LNA-T oligonucleotide | TTTTTTTTtT | 10 |
| 3 | (A)α-T oligonucleotide | TTTTTTTT$\underline{t}^{a}$T | 10 |

(continued)

| No. | Name of oligonucleotide | Sequence (5'→3') | Length (mer) |
|---|---|---|---|
| 4 | (A)β-T oligonucleotide | TTTT·TTTT$\underline{t}^{b}$T | 10 |
| 5 | (B)β-T oligonucleotide | TTTTTTTTT$\underline{t}^{c}$T | 10 |
| 6 | (C)Hβ-T oligonucleotide | TTTTTTTT$\underline{t}^{d}$T | 10 |

T = a unit represented by formula (7) wherein "Base" is a thymin-1-yl group and $R^a$ is H
t = a unit represented by formula (8) wherein "Base" is a thymin-1-yl group
$\underline{t}^{a}$ = a unit represented by formula (6) wherein "Base" is a thymin-1-yl group, $Z^1$ is O, $Z^2$ is a single bond, $R^{3\sim6}$ are each H, $X^1$ is H in (S) configuration, $X^2$ is a methoxy group
$\underline{t}^{b}$ = a unit represented by formula (6) wherein "Base" is a thymin-1-yl group, $Z^1$ is O, $Z^2$ is a single bond, $R^{3\sim6}$ are each H, $X^1$ is H in (R) configuration, $X^2$ is a methoxy group
$\underline{t}^{c}$ = a unit represented by formula (6) wherein "Base" is a thymin-1-yl group, $Z^1$ is $NZ^{11}$, $Z^{11}$ is a methyl group, $Z^2$ is $CZ^{21}Z^{22}$, $Z^{21}$ and $Z^{22}$ and $R^{3\sim6}$ are each H, $X^1$ is H in (R) configuration, $X^2$ is a methoxy group
$\underline{t}^{d}$ = a unit represented by formula (6) wherein "Base" is a thymin-1-yl group, $Z^1$ is O, $Z^2$ is $CZ^{21}Z^{22}$, $Z^{21}$ and $Z^{22}$ and $R^{3\sim6}$ are each H, $X^1$ is H in (R) configuration, $X^2$ is H

2) Preparation of sample solutions

Sample solutions shown in the table below were prepared.

[Table 5]

| Reagent | Final concentration |
|---|---|
| **Tris HCl pH8.0** | 50 **mM** |
| **Mg**$Cl_2$ | 10 **mM** |
| Oligonucleotide | 7.5 μ**M** |

3) Enzymatic reactions

The oligonucleotides No. 1 to 9 were subjected to procedure ((1) to (3) mentioned below) at a temperature of 37°C using a device (T100, manufactured by BIO RAD).

(1) An enzyme CAVP (Crotalus adamanteus Venom phosphodiesterase I) was added to the solution at a final concentration of 2.0 μg/mL to initiate a reaction.
(2) EDTA was added to the solution in such a manner that the concentration of EDTA became 5.0 mM in a reaction mixture at the time point of the completion of the reaction to terminate the reaction.
(3) The reaction time was 0 minute, 10 minutes, 20 minutes, 40 minutes, and 80 minutes.

4) Evaluation of enzyme resistance capability

Each of the sample solutions in each of which the enzymatic reaction in accordance with the procedure 3) had been completed was subjected to a HPLC analysis under the following conditions.

(Conditions)

Device: Alliance e2695 Separations Module/2 489 UV/VIS Detector

(manufactured by Waters)

Column: XBridge Oligonucleoties BEH C18 column 130 Å, 2.5 μm, 4.6 mm × 50 mm mobile phase

Solution A: a 0.1M triethylammonium acetate buffer (pH 7.0)
Solution B: a 0.1M triethylammonium acetate buffer (pH 7.0) : acetonitrile = 1 : 1 (v/v) gradient: 5 to 30% ((v/v) solution B), 15 minutes)
Flow rate: 0.8 mL/min
Column temperature: 50°C
Detection wavelength: 268 nm
Injection amount: 15 μL

The amount of each of the oligonucleotides which was undigested with the enzyme was measured from a HPLC analysis result, and a residual ratio (%) of the undigested oligonucleotide at each of the reaction times was calculated in accordance with the following formula.

[Mathematical formula 1]

$$\text{Residual ratio (\%) of undigested oligonucleotide at each rection time} = \frac{\text{Area of undigested oligonucleotide at each rection time}}{\text{Area of undigested oligonucleotide at rection time of 0 min}} \times 100$$

5) Results

The results are shown in Table 6 and Fig. 2.

[Table 6]

| CAVP 2.0 μg/mL (final concentration ) | | | | | | |
|---|---|---|---|---|---|---|
| Residual ratio (%) of undigested oligonucleotide at each reaction time | | | | | | |
| No. | Name of oligonucleotide | Reaction time | | | | |
| | | 0 min | 10 min | 20 min | 40 min | 80 min |
| 1 | DNA oligonucleotide | 100 | 0 | 1 | 1 | 0 |
| 2 | LNA-T oligonucleotide | 100 | 47 | 40 | 11 | 3 |
| 3 | (A)α-T oligonucleotide | 100 | 93 | 91 | 92 | 87 |
| 4 | (A)β-T oligonucleotide | 100 | 69 | 58 | 44 | 29 |
| 5 | (B)β-T oligonucleotide | 100 | 93 | 93 | 92 | 90 |
| 6 | (C)Hβ-T oligonucleotide | 100 | 73 | 65 | 50 | 33 |

As apparent from the results, the oligonucleotide (6) of the present invention had superior enzyme resistance compared with the naturally occurring types of oligonucleotides and conventional non-naturally-occurring oligonucleotides.

INDUSTRIAL APPLICABILITY

An oligonucleotide modified with the compound of the present invention or a salt (monomer unit) thereof has superior enzyme resistance capability to naturally occurring types of oligonucleotides and conventional non-naturally-occurring oligonucleotides, and have sufficient functionality as a material for nucleic acid therapeutics and genetic examination. Further, the compound of the present invention or a salt a thereof may be used as material for DNA selective nucleic acid therapeutics (antigenes) and the like. Further, using the oligonucleotide modified in plural with the compound or the salt thereof (monomer unit) of the present invention to lower the binding strength with a complementary strand is expected for usage for double-stranded nucleic acid therapeutics such as siRNA promoted to be dissociated in vivo or highly sensitive genetic examination such as the clamp PCR method.

[Sequence Listing]

## Claims

1. A compound represented by formula (1) or a salt thereof:

[Formula 1]

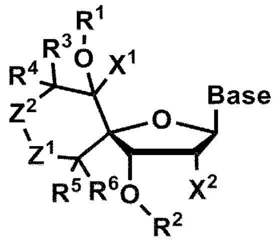

(1)

(wherein "Base" represents an aromatic heterocyclic group which may have a substituent, or an aromatic hydrocarbon ring group which may have a substituent;
$X^1$ represents a hydrogen atom, an alkyl group which may have a substituent, an alkenyl group which may have a substituent, an alkynyl group which may have a substituent, a cycloalkyl group which may have a substituent, or an aryl group which may have a substituent;
$X^2$ represents a hydrogen atom or $-OR^7$;
$R^1$, $R^2$, and $R^7$ are the same as or different from each other and independently represent a hydrogen atom, an alkyl group which may have a substituent, an alkenyl group which may have a substituent, an alkynyl group which may have a substituent, a cycloalkyl group which may have a substituent, a cycloalkenyl group which may have a substituent, an aryl group which may have a substituent, a protecting group for a hydroxy group, a phosphino group which has a substituent, a dihydroxyphosphinyl group which may have a substituent, or a hydroxymercaptophosphinyl group which may have a substituent;
$R^3$, $R^4$, $R^5$, and $R^6$ are the same as or different from each other and independently represent a hydrogen atom, an alkyl group which may have a substituent, an alkenyl group which may have a substituent, an alkynyl group which may have a substituent, a cycloalkyl group which may have a substituent, or an aryl group which may have a substituent;
$Z^1$ represents O or $NZ^{11}$;
$Z^{11}$ represents a hydrogen atom, an alkyl group, an alkenyl group, an alkynyl group, a cycloalkyl group, an aryl group, an aralkyl group, or a protecting group for an amino group;
$Z^2$ represents a single bond or $CZ^{21}Z^{22}$; and
$Z^{21}$ and $Z^{22}$ are the same as or different from each other and independently represent a hydrogen atom, an alkyl group which may have a substituent, an alkenyl group which may have a substituent, an alkynyl group which may have a substituent, a cycloalkyl group which may have a substituent, or an aryl group which may have a substituent).

2. The compound or the salt thereof according to claim 1, wherein $Z^1$ represents O.

3. The compound or the salt thereof according to claim 1, wherein $Z^1$ represents $NZ^{11}$ and $Z^2$ represents $CZ^{21}Z^{22}$.

4. The compound or the salt thereof according to claim 3, wherein $Z^{11}$ represents a hydrogen atom or an alkyl group.

5. The compound or the salt thereof according to any one of claims 1 to 4, wherein $Z^{21}$ and $Z^{22}$ are the same as or different from each other and independently represent a hydrogen atom or an alkyl group.

6. The compound or the salt thereof according to any one of claims 1 to 5, wherein $X^1$ represents a hydrogen atom or an alkyl group.

7. The compound or the salt thereof according to any one of claims 1 to 6, wherein $X^1$ represents a hydrogen atom.

8. The compound or the salt thereof according to any one of claims 1 to 7, wherein $R^3$, $R^4$, $R^5$, and $R^6$ are the same

as or different from each other and independently represent a hydrogen atom or an alkyl group.

**9.** The compound or the salt thereof according to any one of claims 1 to 8, wherein $R^3$, $R^4$, $R^5$, and $R^6$ independently represent a hydrogen atom.

**10.** The compound or the salt thereof according to any one of claims 1 to 9, wherein $R^7$ represents a hydrogen atom or an alkyl group.

**11.** The compound or the salt thereof according to any one of claims 1 to 10, wherein
$R^1$ and $R^2$ are the same as or different from each other and independently represent a hydrogen atom, an alkyl group which may have a substituent, an aryl group which may have a substituent, an alkylcarbonyl group, an arylcarbonyl group, an alkylsulfonyl group, an arylsulfonyl group, a group represented by a formula: $-Si(R^8)_3$ (wherein $R^8$'s are the same as or different from each other and independently represent an alkyl group or an aryl group), a group represented by a formula: $-P(R^{9a})(R^{9b})$ (wherein $R^{9a}$ and $R^{9b}$ are the same as or different from each other and independently represents a hydroxy group, a mercapto group, an amino group, an alkoxy group, a haloalkoxy group, a cyanoalkoxy group, an alkylthio group, a haloalkylthio group, a cyanoalkylthio group, or an alkylamino group), a dihydroxyphosphinyl group, or a hydroxymercaptophosphinyl group.

**12.** The compound or the salt thereof according to any one of claims 1 to 11, wherein
"Base" represents a 2,4-dioxo-1,2,3,4-tetrahydropyrimidin-1-yl group which may have a substituent, a 2-oxo-1,2-dihydropyrimidin-1-yl group which may have a substituent, a purin-9-yl group which may have a substituent, or a 6-oxo-1,6-dihydro-9H-purin-9-yl group which may have a substituent.

**13.** A method for producing the compound or the salt thereof according to any one of claims 1 to 12, the method comprising:

a step of cyclization of a compound represented by formula (2) or a salt thereof in the presence or absence of an amine represented by formula (2'): $Z^{11}-NH_2$ ($Z^{11}$ is as defined above),

[Formula 2]

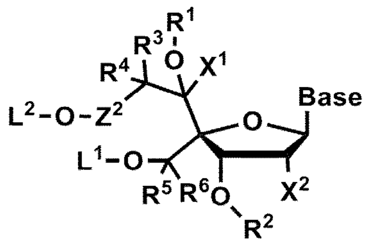

(2)

(wherein $L^1$ represents a hydroxy activating group, $L^2$ represents a hydrogen atom or a hydroxy activating group, and "Base", $X^1$, $X^2$, $R^1$, $R^2$, $R^3$, $R^4$, $R^5$, $R^6$, and $Z^2$ are as defined above).

**14.** An oligonucleotide comprising a unit represented by formula (6) or a salt thereof:

[Formula 3]

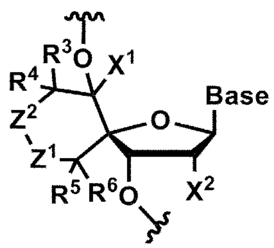

(6)

(wherein "Base" represents an aromatic heterocyclic group which may have a substituent, or an aromatic

hydrocarbon ring group which may have a substituent;

$X^1$ represents a hydrogen atom, an alkyl group which may have a substituent, an alkenyl group which may have a substituent, an alkynyl group which may have a substituent, a cycloalkyl group which may have a substituent, or an aryl group which may have a substituent;

$X^2$ represents a hydrogen atom or $-OR^7$;

$R^7$ represents a hydrogen atom, an alkyl group which may have a substituent, an alkenyl group which may have a substituent, an alkynyl group which may have a substituent, a cycloalkyl group which may have a substituent, a cycloalkenyl group which may have a substituent, an aryl group which may have a substituent, a protecting group for a hydroxy group, a phosphino group which has a substituent, a dihydroxyphosphinyl group which may have a substituent, or a hydroxymercaptophosphinyl group which may have a substituent;

$R^3$, $R^4$, $R^5$, and $R^6$ are the same as or different from each other and independently represent a hydrogen atom, an alkyl group which may have a substituent, an alkenyl group which may have a substituent, an alkynyl group which may have a substituent, a cycloalkyl group which may have a substituent, or an aryl group which may have a substituent;

$Z^1$ represents O or $NZ^{11}$;

$Z^{11}$ represents a hydrogen atom, an alkyl group, an alkenyl group, an alkynyl group, a cycloalkyl group, an aryl group, an aralkyl group, or a protecting group for an amino group;

$Z^2$ represents a single bond or $CZ^{21}Z^{22}$; and

$Z^{21}$ and $Z^{22}$ are the same as or different from each other and independently represent a hydrogen atom, an alkyl group which may have a substituent, an alkenyl group which may have a substituent, an alkynyl group which may have a substituent, a cycloalkyl group which may have a substituent, or an aryl group which may have a substituent).

**15.** A method of detecting a target nucleic acid, comprising steps of:

(I) amplifying the target nucleic acid selectively by a nucleic acid amplification method; and
(II) detecting the target nucleic acid that has been amplified in the step (I),

in which an oligonucleotide that is used for the amplification or the detection comprises the oligonucleotide or the salt thereof according to claim 14.

**16.** A kit for detecting or amplifying a target nucleic acid, comprising:

(a) a primer and a probe, in which at least one of the primer and the probe comprises the oligonucleotide or the salt thereof according to claim 14; or
(b) a clamp nucleic acid and a primer, in which at least one of the clamp nucleic acid and the primer comprises the oligonucleotide or the salt thereof according to claim 14.

**17.** A composition comprising the compound or the salt thereof according to any one of claims 1 to 12, or the oligonucleotide or the salt thereof according to claim 14.

**18.** A compound represented by formula (2) or a salt thereof:

[Formula 4]

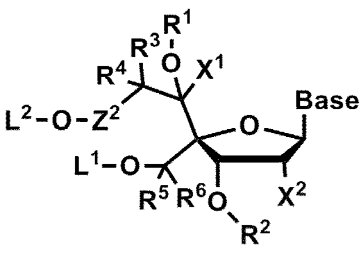

(2)

(wherein "Base" represents an aromatic heterocyclic group which may have a substituent, or an aromatic hydrocarbon ring group which may have a substituent;

$X^1$ represents a hydrogen atom, an alkyl group which may have a substituent, an alkenyl group which may have a substituent, an alkynyl group which may have

a substituent, an alkynyl group which may have a substituent, a cycloalkyl group which may have a substituent, or an aryl group which may have a substituent;

$X^2$ represents a hydrogen atom or $-OR^7$;

$R^1$, $R^2$, and $R^7$ are the same as or different from each other and independently represent a hydrogen atom, an alkyl group which may have a substituent, an alkenyl group which may have a substituent, an alkynyl group which may have a substituent, a cycloalkyl group which may have a substituent, a cycloalkenyl group which may have a substituent, an aryl group which may have a substituent, a protecting group for a hydroxy group, a phosphino group which has a substituent, a dihydroxyphosphinyl group which may have a substituent, or a hydroxymercaptophosphinyl group which may have a substituent;

$R^3$, $R^4$, $R^5$, and $R^6$ are the same as or different from each other and independently represent a hydrogen atom, an alkyl group which may have a substituent, an alkenyl group which may have a substituent, an alkynyl group which may have a substituent, a cycloalkyl group which may have a substituent, or an aryl group which may have a substituent;

$L^1$ represents a hydroxy activating group;

$L^2$ represents a hydrogen atom or a hydroxy activating group;

$Z^2$ represents a single bond or $CZ^{21}Z^{22}$; and

$Z^{21}$ and $Z^{22}$ are the same as or different from each other and independently represent a hydrogen atom, an alkyl group which may have a substituent, an alkenyl group which may have a substituent, an alkynyl group which may have a substituent, a cycloalkyl group which may have a substituent, or an aryl group which may have a substituent).

**19.** A compound represented by formula (4) or a salt thereof:

[Formula 5]

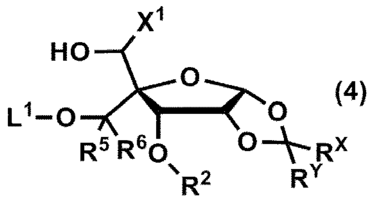

(4)

(wherein $R^2$ represents a hydrogen atom, an alkyl group which may have a substituent, an alkenyl group which may have a substituent, an alkynyl group which may have a substituent, a cycloalkyl group which may have a substituent, a cycloalkenyl group which may have a substituent, an aryl group which may have a substituent, a protecting group for a hydroxy group, a phosphino group which has a substituent, a dihydroxyphosphinyl group which may have a substituent, or a hydroxymercaptophosphinyl group which may have a substituent;

$R^5$, $R^6$, $R^X$, $R^Y$, and $X^1$ are the same as or different from each other and independently represent a hydrogen atom, an alkyl group which may have a substituent, an alkenyl group which may have a substituent, an alkynyl group which may have a substituent, a cycloalkyl group which may have a substituent, or an aryl group which may have a substituent; and

$L^1$ represents a hydroxy activating group).

Fig. 1A

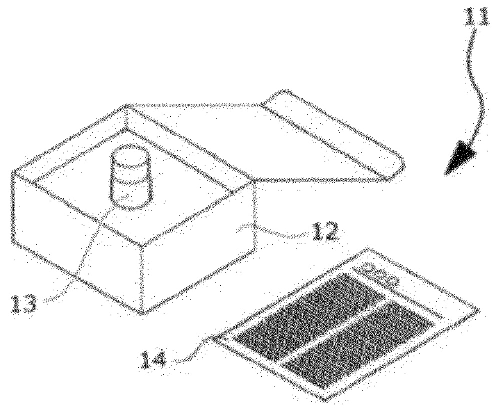


Fig. 1B

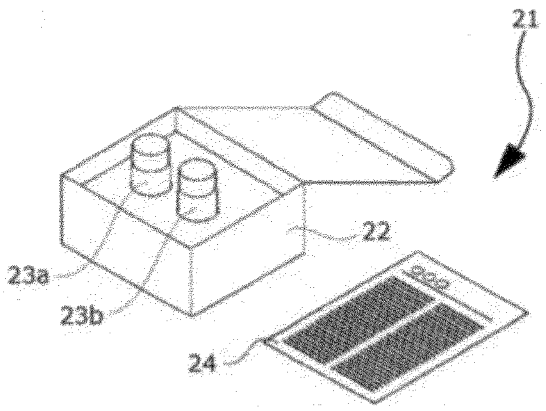


Fig. 1C

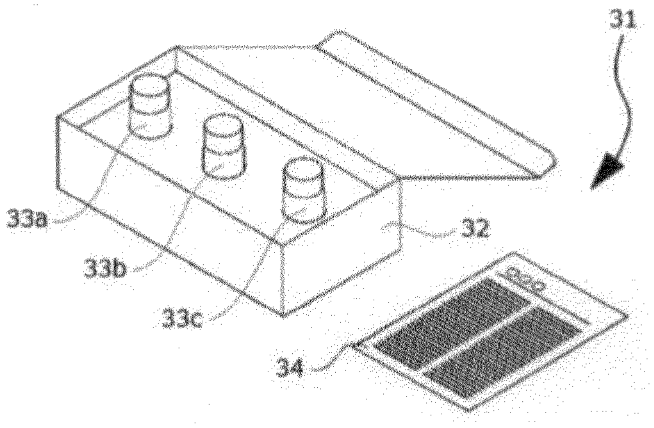

Fig. 2

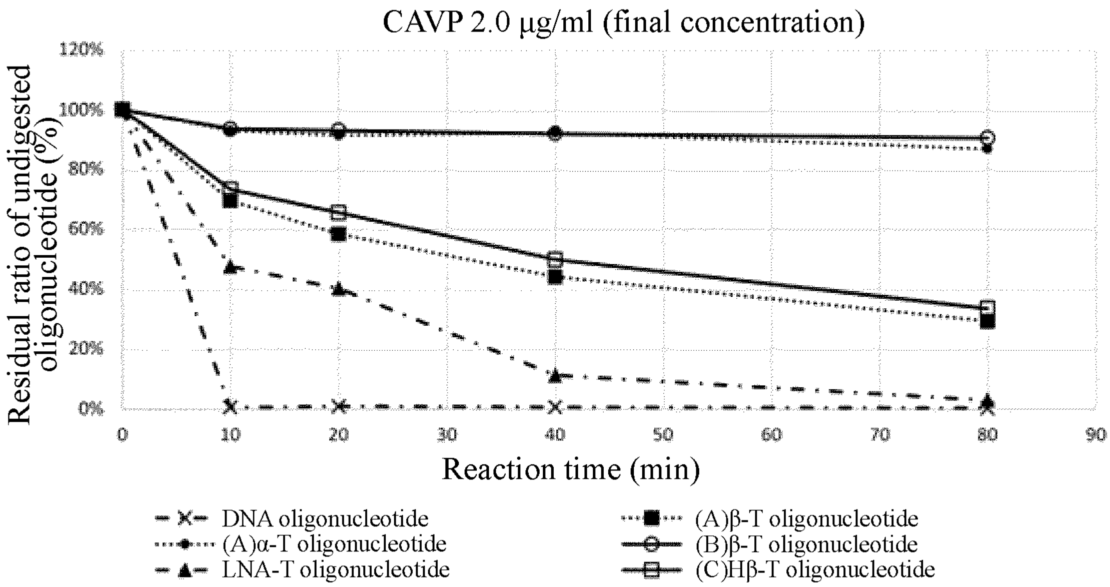
CAVP 2.0 μg/ml (final concentration)
Residual ratio of undigested oligonucleotide (%)
120%
100%
80%
60%
40%
20%
0%
0
10
20
30
40
50
60
70
80
90
Reaction time (min)
DNA oligonucleotide
(A)α-T oligonucleotide
LNA-T oligonucleotide
(A)β-T oligonucleotide
(B)β-T oligonucleotide
(C)Hβ-T oligonucleotide

**INTERNATIONAL SEARCH REPORT**

International application No. **PCT/JP2022/048121**

**A. CLASSIFICATION OF SUBJECT MATTER**

***C07H 19/10***(2006.01)i; ***A61K 31/7088***(2006.01)i; ***A61K 48/00***(2006.01)i; ***A61P 31/12***(2006.01)i; ***A61P 35/00***(2006.01)i; ***C07H 19/067***(2006.01)i; ***C07H 21/04***(2006.01)i; ***C12M 1/00***(2006.01)i; ***C12M 1/34***(2006.01)i; ***C12N 15/11***(2006.01)i; ***C12Q 1/6844***(2018.01)i

FI: C07H19/10 ZNA; A61K48/00; C07H19/067; C07H21/04 B; A61P35/00; A61P31/12; C12N15/11 Z; C12Q1/6844 Z; C12M1/00 A; C12M1/34 Z; A61K31/7088 ZNA

According to International Patent Classification (IPC) or to both national classification and IPC

**B. FIELDS SEARCHED**

Minimum documentation searched (classification system followed by classification symbols)

C07H19/10; A61K31/7088; A61K48/00; A61P31/12; A61P35/00; C07H19/067; C07H21/04; C12M1/00; C12M1/34; C12N15/11; C12Q1/6844

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | WAGA, T. et al. Bioscience, Biotechnology, and Biochemistry. 1993, vol. 57, pp. 1433<br>entire text, all drawings | 1-18 |
| X<br>A | WO 2009/006478 A2 (ISIS PHARMACEUTICALS, INC.) 08 January 2009 (2009-01-08)<br>example 2, scheme 2, compound 16 | 18<br>1-17 |
| X<br>A | JP 2009-524695 A (ISIS PHARMACEUTICALS, INC.) 02 July 2009 (2009-07-02)<br>examples 9, 13, 15, 17-18 | 18<br>1-17 |
| A | OSAWA, T. et al. Bioorganic & Medicinal Chemistry. 23 December 2020, vol. 31, 115966<br>entire text, all drawings | 1-18 |
| A | WO 2020/198323 A1 (CALIFORNIA INSTITUTE OF TECHNOLOGY) 01 October 2020 (2020-10-01) | 1-18 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 March 2023** | **20 March 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/048121**

**C. DOCUMENTS CONSIDERED TO BE RELEVANT**

| Category* | Citation of document, with indication, where appropriate, of the relevant passages | Relevant to claim No. |
|---|---|---|
| P, A | WO 2022/065413 A1 (RIKEN GENESIS CO LTD) 31 March 2022 (2022-03-31) | 1-18 |

Form PCT/ISA/210 (second sheet) (January 2015)

**INTERNATIONAL SEARCH REPORT**

International application No.
**PCT/JP2022/048121**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

"The form of Annex C/ST.25 text file" above shall read as "the form of ST.26.".

Form PCT/ISA/210 (continuation of first sheet) (January 2015)

**INTERNATIONAL SEARCH REPORT**

International application No.
**PCT/JP2022/048121**

**Box No. III Observations where unity of invention is lacking (Continuation of item 3 of first sheet)**

This International Searching Authority found multiple inventions in this international application, as follows:

(Invention 1) Invention in claims 1-18

The invention in claims 1-13 has the special technical feature of a "compound represented by formula (1)," and is thus classified as invention 1.

Also, the invention in claims 14-17 is the invention that relates to an oligonucleotide containing a compound represented by formula (1), and the invention in claim 18 is the invention that relates to a precursor of the compound represented by formula (1). It would be efficient to examine the inventions together with the invention subjected to examination on the basis of the special technical feature, and thus claims 14-18 are added as invention 1.

(Invention 2) Invention in claim 19

The invention in claim 19 shares, with the invention in claim 1, the common technical feature of a substituted tetrahydrofuran structure. However, said technical feature does not make a contribution over the prior art in light of the disclosure of Document 1 (in particular, compound 3, etc.), and thus cannot be considered a special technical feature. In addition, there are no other same or corresponding special technical features between the invention in claim 19 and the invention in claim 1.

Also, claim 19 is not dependent on claim 1 and is not substantially identical to or similarly closely related to any of the claims classified as invention 1.

Therefore, the invention in claim 19 is classified as invention 2.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-18**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/JP2022/048121**

| Patent document cited in search report | Publication date (day/month/year) | Patent family member(s) | Publication date (day/month/year) |
|---|---|---|---|
| WO 2009/006478 A2 | 08 January 2009 | US 2011/0053881 A1<br>EP 2176280 A2<br>AU 2008272918 A<br>CN 101796062 A<br>CA 2692579 A<br>AT 538127 T<br>DK 2176280 T<br>ES 2376507 T | |
| JP 2009-524695 A | 02 July 2009 | US 2007/0249049 A1<br>examples 9, 13, 15, 17-18<br>WO 2007/090071 A2<br>EP 1984381 A2<br>CA 2640171 A<br>CN 101410406 A<br>AT 482965 T<br>DK 1984381 T<br>ES 2351674 T<br>AU 2007211080 A<br>KR 10-2008-0088662 A<br>DK 2314594 T<br>PL 1984381 T<br>NO 20084738 L<br>MX 2008014100 A<br>AT 513912 T<br>DK 2021472 T<br>ES 2386578 T<br>HK 1128418 A<br>BR PI0711429 A | |
| WO 2020/198323 A1 | 01 October 2020 | JP 2022-521818 A<br>US 2022/0194949 A1<br>EP 3947387 A1<br>CN 114127072 A<br>KR 10-2022-0023330 A<br>CA 3134779 A<br>AU 2020245480 A<br>SG 11202110272S A<br>IL 286622 A | |
| WO 2022/065413 A1 | 31 March 2022 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5359067 A
- US 2007167387
- US 2012071646 A
- US 2003105309
- US 2017044528
- US 2006166908
- US 2012208991
- US 2015266917
- US 2003207841
- US 5130238 A
- US 5399491 A
- US 20010053518
- US 6410278 B
- US 2003073081
- EP 320328 A
- US 5455166 A
- US 2015240299


### Non-patent literature cited in the description


- *Bioorg. Med. Chem.,* 2021, vol. 31, 115966
- **SAMBROOK, J.** Molecular Cloning: A Laboratory Manual. Cold Spring Harbor Laboratory Press, 1998